# EUROPÄISCHE PATENTSCHRIFT

(11) **EP 0 423 482 B1**
(45) Veröffentlichungstag und Bekanntmachung des Hinweises auf die Patenterteilung: **11.01.1995**
(21) Anmeldenummer: 90117449.0
(22) Anmeldetag: 11.09.1990
(51) Int. Cl.: C07D 307/66, C07D 307/60, A01N 43/08

(54) **5H-Furan-2-on-Derivate**
5H-furan-2-one derivatives
Dérivés de 5H-furan-2-one

(30) Priorität: 23.09.1989 DE 3931773; 05.05.1990 DE 4014420
(43) Veröffentlichungstag der Anmeldung: 24.04.1991
(73) Patentinhaber: BAYER AG, 51368 Leverkusen (DE)
(72) Erfinder: Krämer, Wolfgang, Dr., D-5093 Burscheid 2 (DE); Kleefeld, Gerd, Dr., D-4000 Düsseldorf 13 (DE); Bachmann, Jürgen, Dr., D-5090 Leverkusen 1 (DE); Babczinski, Peter, Dr., D-5600 Wuppertal 1 (DE); Santel, Hans-Joachim, Dr., D-5090 Leverkusen 1 (DE); Lürssen, Klaus, Dr., D-5060 Bergisch Gladbach 2 (DE); Schmidt, Robert R., Dr., D-5060 Bergisch Gladbach 2 (DE)

(56) Entgegenhaltungen:
- EP-A- 0 299 694
- US-E- 27 894
- PATENT ABSTRACTS OF JAPAN, Band 10, Nr. 280 (C-374)[2336], 24. September 1986;& JP-A-61 100 577
- TETRAHEDRON LETTERS, Band 26, Nr. 20, Mai 1985, Seiten 2459-2462, Oxford, GB; T. HIYAMA et al.: "New synthetic methods for 4-amino-2(5H)-furanones"
- JOURNAL OF THE CHEMICAL SOCIETY, PERKIN TRANSACTIONS I, Nr. 8, August 1985,Seiten 1567-1576, Letchworth, GB; A.C. CAMPBELL et al.: "Synthesis of (E)- and(Z)-pulvinones"
- Knight W.D. et al.: JOURNAL OF THE CHEMICAL SOCIETY, PERKIN TRANSACTIONS, Band 1, 1979, Seiten 62-69
- Knight W.D. et al.: JOURNAL OF THE CHEMICAL SOCIETY, PERKIN TRANSACTIONS, Band 1, 1979, Seiten 84-88
- Knight W.D. et al.: JOURNAL OF THE CHEMICAL SOCIETY, CHEMICAL COMMUNICATIONS, 1976, Seiten 660-661
- Knight W.D. et al.: JOURNAL OF THE CHEMICAL SOCIETY, CHEMICAL COMMUNICATIONS, 1975, Seiten876-877

## Beschreibung

Die Erfindung betrifft neue 5H-Furan-2-on-Derivate, mehrere Verfahren zu ihrer Herstellung und ihre Verwendung als Herbizide.

Es ist bereits bekannt, daß bestimmte substituierte 2H-Furan-3-one, wie beispielsweise (±)-5-Methylamino-2-phenyl-4-[3-trifluormethyl)-phenyl]-2H-furan-3-on herbizide Eigenschaften besitzen (vgl. DE-OS 34 22 346).

Die Wirkung dieser Verbindungen ist jedoch, insbesondere bei niedrigen Aufwandmengen und -konzentrationen, nicht immer in allen Anwendungsbereichen völlig zufriedenstellend.

Weiterhin ist die Synthese zahlreicher 5H-Furan-2-on-Derivate und ihre Verwendung als Zwischenprodukte bei der Synthese von Naturstoffen bekannt Sie werden mittels Disclaimer von der Definition der Formel (I) ausgeschlossen (vgl. z.B. Tetrahedron Lett., 29, 2085-2088, 1988; Can. J. Chem., 64, 104-109, 1986; J. Chem. Soc., Perkin Trans. 1, 1567-76, 1985; J.Chem. Soc. Perkin Trans. 1, 1539-45, 1984; Tetrahedron, 35, 2181-2185, 1979; J.Chem. Soc. Perkin Trans. 1, 70-76, 1979; 3. Chem. Soc., Perkin Trans. 1, 62-69, 1979; J. Chem. Soc., Perkin Trans. 1, 84-88, 1979; J. Chem. Soc., Chem. Commun. 660-661, 1976; J.Chem. Soc., Chem. Commun. 635-637, 1976; Tetrahedron Lett. 4279 - 4282, 1975; J. Chem. Soc., Chem. Commun., 876 - 877, 1975; JP 6917901). Desweiteren sind bestimmte 5H-Furan-2-on-Derivate bekannt (Hiyama, T. et al: Tetrahedron Letters, 26, Nr. 20, 1985, 2459 - 2462; Campbell, A. C. et al: Journal of the Chemical Society, Perkin Transactions, 1, Nr. 8, 1985, 1567 - 1576). Eine herbizide Wirkung dieser Verbindungen ist aber nicht erwähnt.

Weiterhin ist die vorliegende Anmeldung von der europäischen Patentanmeldung EP-A 299 694 abgegrenzt, welche Furan-2-one mit fungizider Wirkung beschreibt.

Es wurden neue 5H-Furan-2-on-Derivate der Formel (I) 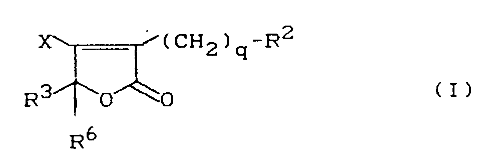
in welcher
- X: für den Rest -OR¹ oder den Rest 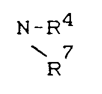 steht, wobei
- R⁴: für Wasserstoff, Alkyl, Alkenyl, Alkoxyalkyl oder Alkylcarbonyl steht und
- R⁷: für Wasserstoff, Hydroxy, Amino, Formyl, Alkyl, Alkoxyalkyl, Cyanoalkyl, Alkylcarbonyl, Halogenalkylcarbonyl, Alkoxycarbonylalkyl, Alkoxy, Alkylamino, Alkylcarbonyloxy, Aminocarbonylalkyl, Alkinyl, Cycloalkyl, jeweils unsubstituiertes oder substituiertes Arylcarbonyl, Aralkyl, Aralkyloxy oder für die Gruppe 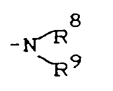 steht,
wobei R⁸ und R⁹ jeweils unabhängig voneinander für Alkyl oder Alkylcarbonyl stehen,
oder
- R⁴ und R⁷: gemeinsam mit dem Stickstoffatom, an welches sie gebunden sind, für einen gesättigten Heterocyclus stehen,
- q: für die Zahlen 0 oder 1 steht,
- R¹: für Alkyl, Alkoxyalkyl, Cyanoalkyl, Alkylcarbonyl, Halogenalkylcarbonyl oder Alkoxycarbonylalkyl steht,
- R²: für unsubstituiertes oder einfach oder mehrfach, gleich oder verschieden substituiertes Aryl steht, wobei als Substituenten ausgewählt sind:
Cyano, Nitro, Halogen, Alkyl, Alkoxy, Halogenalkyl, Halogenalkoxy, jeweils unsubstituiertes oder einfach oder mehrfach, gleich oder verschieden substituiertes Phenethenyl oder Phenethinyl steht, wobei als Phenylsubstituenten Cyano, Nitro, Halogen, Alkyl, Alkoxy, Halogenalkyl oder Halogenalkoxy genannt seien, als weiterer Phenylsubstituent sei außerdem der Rest -(CH₂)ₙ-Zₘ-(CH₂)ₚ-R⁵ genannt, wobei
- R⁵: für unsubstituiertes oder einfach oder mehrfach, gleich oder verschieden substituiertes Aryl steht, wobei als Substituenten genannt seien: Cyano, Nitro, Halogen, Alkyl, Alkoxy, Halogenalkyl oder Halogenalkoxy,
- Z: für Sauerstoff, Schwefel oder die Gruppe -CO-steht,
- n, m und p: unabhängig voneinander für die Zahlen 0 oder 1 stehen, und
- R³ und R⁶: unabhängig für Wasserstoff, Alkyl, jeweils unsubstituiertes oder einfach oder mehrfach, gleich oder verschieden substituiertes Aryl oder Aralkyl stehen, wobei als Substituenten im Arylteil ausgewählt sind: Cyano, Nitro, Halogen, Alkyl, Alkoxy, Halogenalkyl oder Halogenalkoxy,

mit der Maßgabe, daß, wenn X für den Rest OR¹ und gleichzeitig q für 0 steht, R² nur dann für ortho-substituiertes Phenyl steht, wenn die Substituenten Halogen, Halogenmethyl mit 1 bis 3 gleichen oder verschiedenen Halogenatomen oder jeweils unsubstituiertes oder einfach bis mehrfach gleich oder verschieden substituiertes Phenethenyl oder Phenethinyl sind, mit der Maßgabe, daß R² für substituiertes Phenyl steht, wenn gleichzeitig q für 1 und X für den Rest OR¹ steht und ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on und 4-Methoxy-3-(4-methoxyphenyl)-5H-furan-2-on, 3-(2-Chlorphenyl)-4-methoxy-5H-furan-2-on, 3-(2-Fluorphenyl)-4-methoxy-5H-furan-2-on, 4-Methoxy-3-(2-methoxyphenyl)-5H-furan-2-on, 5,5-Dimethyl-4-amino-3-phenyl-5H-furan-2-on, 4-Methoxy-3-phenyl-5H-furan-2-on, 5-Benzyl-4-methoxy-3-phenyl-5H-furan-2-on, 5,5-Diethyl-4-methoxy-3-phenyl-5H-furan-2-on, 5-(α-Methoxycarbonyl-α-hydroxy-benzyl)-4-methoxy-3-(4-methoxyphenyl)-5-H-furan-2-on, 5-(α-Methoxycarbonyl-α-hydroxy-4-methoxybenzyl)-4-methoxy-3-(3,4,5-trimethoxyphenyl)-5-H-furan-2-on, 5-(α-Hydroxy-4-methoxybenzyl)-4-methoxy-3-(4-methoxyphenyl)-5H-furan-2-on,
gefunden.

Weiterhin wurde gefunden, daß man die neuen 5H-Furan-2-on-Derivate der Formel (I) erhält, wenn man
a) für den Fall, daß in der Formel (I)
   X für den Rest 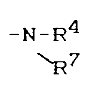 steht und
   R², R³, R⁴, R⁶ und q die oben angegebene Bedeutung haben und
   R⁷⁻⁰ für Wasserstoff, Alkyl, Alkoxyalkyl, Cyanoalkyl, Alkoxycarbonylalkyl, Alkoxy, Amino, Alkylamino, Hydroxy, Aralkyl, Aminocarbonylalkyl, Cycloalkyl, Arylalkyloxy, Alkinyl oder für die Gruppe 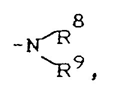 wobei R⁸ und R⁹ jeweils die oben angebene Bedeutung haben, stehen
   oder
   R⁷⁻⁰ und R⁴ gemeinsam mit dem Stickstoffatom, an welches sie gebunden sind für einen gesättigten Heterocyclus stehen,
a-α) 5H-Furan-2-on-Derivate der Formel (II) 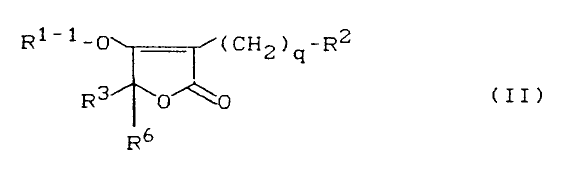 in welcher
   - R¹⁻¹: für Alkyl, insbesondere Methyl oder Ethyl steht und
   - R², R³, R⁶ und q: die oben angegebenen Bedeutungen haben,
   mit Aminen der Formel (III) 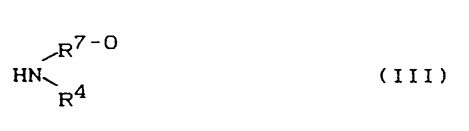 in welcher
   - R⁷⁻⁰ und R⁴: die oben angegebene Bedeutung haben,
   oder deren Hydrochloride,
   gegebenenfalls in Gegenwart eines Verdünnungsmittels und gegebenenfalls unter Druck umsetzt oder
a-β) die nach Verfahren (a-α) erhaltenen 5H-Furan-2-on-Derivate der Formel (Ia) 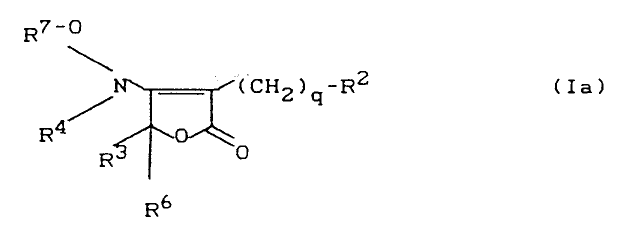 in welcher
   - R⁷⁻⁰, R², R³, R⁴, R⁶ und q: die oben angegebene Bedeutung haben,
   mit Acylierungsmitteln der Formel (VI)

   R⁷⁻¹-E² (VI)

   in welcher
   - R⁷⁻¹: für Alkylcarbonyl, Alkylcarbonyloxy, Halogenalkylcarbonyl oder Arylcarbonyl steht und
   - E²: für eine elektronenanziehende Abgangsgruppe steht,
   oder mit Orthoestern der Formel (VIa)

   (RO)₃CH (VIa)

   in welcher
   - R: für Methyl oder Ethyl steht,
   gegebenenfalls in Gegenwart eines Verdünnungsmittels und gegebenenfalls in Gegenwart eines Reaktionshilfsmittels umsetzt, oder wenn man
b) für den Fall, daß in der Formel (I)
   - X: für den Rest -OR¹ steht und
   - R¹, R², R³, R⁶ und q: die oben angegebenen Bedeutungen haben,
   substituierte Tetronsäure-Derivate der Formel (IV) 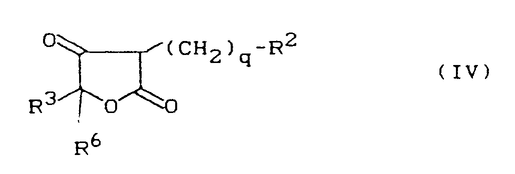 in welcher
   - R², R³, R⁶ und q: die oben angegebenen Bedeutungen haben,
   mit Alkylierungs- oder Acylierungsmitteln der Formel (V)

   R¹ - E¹ (V)

   in welcher
   - R¹: die oben angegebene Bedeutung hat und
   - E¹: für eine elektronenanziehende Abgangsgruppe steht,
   gegebenenfalls in Gegenwart eines Verdünnungsmittels und gegebenenfalls in Gegenwart eines Katalysators bzw. Reaktionshilfsmittels umsetzt.

Schließlich wurde gefunden, daß die neuen substituierten inerten 5H-Furan-2-on-Derivate der Formel (I) interessante herbizide Eigenschaften aufweisen.

Überraschenderweise zeigen die neuen substituierten 5H-Furan-2-on-Derivate der Formel (I) bessere herbizide Eigenschaften als das aus dem Stand der Technik bekannte (±)-5-Methylamino-2-phenyl-4-[3-(trifluormethyl)-phenyl]-2H-furan-3-on, welches ein konstitutionell ähnlicher Wirkstoff gleicher Wirkungsart ist.

Die Erfindung betrifft vorzugsweise Verbindungen der Formel (I), in welcher
- X: für den Rest OR¹ oder den Rest 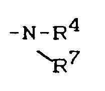 steht, wobei
- R⁴: für Wasserstoff, jeweils geradkettiges oder verzweigtes Alkyl mit 1 bis 4 Kohlenstoffatomen, Alkenyl mit 2 bis 4 Kohlenstoffatomen, Alkoxyalkyl mit jeweils 1 bis 4 Kohlenstoffatomen im Alkoxy- und Alkylteil oder Alkylcarbonyl mit 1 bis 4 Kohlenstoffatomen steht,
- R⁷: für Wasserstoff, Hydroxy, Amino, Formyl, für jeweils geradkettiges oder verzweigtes Alkyl, Alkoxy, Alkoxyalkyl, Cyanoalkyl, Alkylcarbonyl, Halogenalkylcarbonyl, Alkoxycarbonylalkyl, Alkylamino, Alkylcarbonyloxy oder Aminocarbonylalkyl mit 1 bis 6 Kohlenstoffatomen in den einzelnen Alkylteilen und gegebenenfalls 1 bis 9 gleichen oder verschiedenen Halogenatomen, für Alkinyl mit 2 bis 6 Kohlenstoffatomen, Cycloalkyl mit 3 bis 7 Kohlenstoffatomen, für jeweils unsubstituiertes oder einfach bis mehrfach, gleich oder verschieden substituiertes Arylcarbonyl, Aralkyl, Aralkyloxy mit 3 bis 10 Kohlenstoffatomen und 1 bis 4 Kohlenstoffatomen im geradkettigen oder verzweigten Alkylteil, wobei als Substituenten die bei R⁵ genannten Substituenten infrage kommen oder R⁷ für eine Gruppe 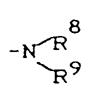 steht,
wobei R⁸ und R⁹ jeweils unabhängig voneinander für Alkyl oder Alkylcarbonyl mit jeweils 1 bis 4 Kohlenstoffatomen stehen, oder
- R⁴ und R⁷: gemeinsam mit dem Stickstoffatom, an welches sie gebunden sind, für eine gesättigte Heteroalkylenkette mit 4 bis 5 Kohlenstoffatomen stehen,
- q: für die Zahlen 0 oder 1 steht,
- R¹: für geradkettiges oder verzweigtes Alkyl mit 1 bis 4 Kohlenstoffatomen oder für jeweils geradkettiges oder verzweigtes Cyanoalkyl, Alkoxyalkyl, Alkylcarbonyl, Halogenalkylcarbonyl oder Alkoxycarbonylalkyl mit 1 bis 6 Kohlenstoffatomen in den einzelnen Alkylteilen und gegebenenfalls 1 bis 9 gleichen oder verschiedenen Halogenatomen steht,
- R²: für unsubstituiertes oder einfach oder mehrfach, gleich oder verschieden substituiertes Phenyl steht, wobei als Substituenten genannt seien: Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen, jeweils unsubstituiertes oder einfach oder mehrfach, gleich oder verschieden substituiertes Phenethenyl, Phenethinyl, wobei jeweils als Substituenten die schon oben genannten Phenylsubstituenten infrage kommen;
als weiterer Phenylsubstituent sei außerdem der Rest -(CH₂)ₙ-Zₘ-(CH₂)ₚ-R⁵ genannt,
worin
- R⁵: für unsubstituiertes oder einfach oder mehrfach, gleich oder verschieden substituiertes Aryl mit 6 bis 10 Kohlenstoffatomen, insbesondere Phenyl oder Naphthyl steht, wobei als Substituenten Cyano, Nitro, Halogen, C₁₋₄-Alkyl, C₁₋₄-Alkoxy, Halogen-C₁₋₄-alkyl oder Halogen-C₁₋₄-alkoxy infrage kommen,
- Z: für Sauerstoff, Schwefel oder für die Gruppe >C=O steht und
- n, m und p: unabhängig voneinander für die Zahlen 0 oder 1 stehen, und
- R³ und R⁶: für Wasserstoff, geradkettiges oder verzweigtes Alkyl mit 1 bis 6 Kohlenstoffatomen, unsubstituiertes oder einfach bis mehrfach, gleich oder verschieden substituiertes Phenyl oder unsubstituiertes oder einfach bis mehrfach, gleich oder verschieden substituiertes Aralkyl mit 6 bis 10 Kohlenstoffatomen im Arylteil und 1 bis 4 Kohlenstoffatomen im Alkylteil stehen, wobei als Substituenten Cyano, Nitro, Halogen, C₁₋₄-Alkyl, C₁₋₄-Alkoxy, Halogen-C₁₋₄-alkyl und Halogen-C₁₋₄-alkoxy infrage kommen,

mit der Maßgabe, daß, wenn X für den Rest OR¹ und gleichzeitig q für O steht, R² nur dann für ortho-substituiertes Phenyl steht, wenn die Substituenten Halogen, Halogenmethyl mit 1 bis 3 gleichen oder verschiedenen Halogenatomen oder jeweils unsubstituiertes oder einfach bis mehrfach gleich oder verschieden substituiertes Phenethenyl oder Phenethinyl sind, mit der Maßgabe, daß R² für substituiertes Phenyl steht, wenn gleichzeitig q für 1 und X für den Rest OR¹ steht und
ausgenommen die Verbindungen 5-[(3,4-Dimethoxyphenyl]-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on und 4-Methoxy-3-(4-methoxyphenyl)-5H-furan-2-on, 3-(2-Chlorphenyl)-4-methoxy-5H-furan-2-on, 3-(2-Fluorphenyl)-4-methoxy-5H-furan-2-on und 4-Methoxy-3-(2-methoxyphenyl)-5H-furan-2-on, 5,5-Dimethyl-4-amino-3-phenyl-5H-furan-2-on, 4-Methoxy-3-phenyl-5H-furan-2-on, 5-Benzyl-4-methoxy-3-phenyl-5H-furan-2-on, 5,5-Diethyl-4-methoxy-3-phenyl-5H-furan-2-on, 5-(α-Methoxycarbonyl-α-hydroxy-benzyl)-4-methoxy-3-(4-methoxyphenyl)-5-H-furan-2-on, 5-(α-Methoxycarbonyl-α-hydroxy-4-methoxybenzyl)-4-methoxy-3-(3,4,5-trimethoxyphenyl)-5-H-furan-2-on, 5-(α-Hydroxy-4-methoxybenzyl)-4-methoxy-3-(4-methoxyphenyl)-5H-furan-2-on.

Besonders bevorzugt ist die Gruppe von Verbindungen der Formel (I), in welcher
- X: für den Rest 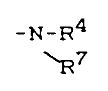 steht,
worin
- R⁴: für Wasserstoff, Methyl, Ethyl, n- oder i-Propyl, n-, i-, s- oder t-Butyl, Allyl, Propenyl-(2), 1-Methallyl, Methoxymethyl, Ethoxymethyl, Methoxyethyl, Ethoxyethyl, Methylcarbonyl, Ethylcarbonyl, n-Propylcarbonyl, i-Propylcarbonyl steht,
- R⁷: für Wasserstoff, Hydroxy, Amino, Formyl, Methyl, Ethyl, n- oder i-Propyl, n-, i-, s- oder t-Butyl, Methoxy, Ethoxy, n- oder i-Propoxy, n-, i-, s- oder t-Butoxy, Alkylcarbonyl mit 1 bis 6 Kohlenstoffatomen, jeweils geradkettiges oder verzweigtes Alkoxyalkyl, Cyanoalkyl, Halogenalkylcarbonyl, Alkoxycarbonylalkyl, Alkylamino, Alkylcarbonyloxy oder Aminocarbonylalkyl mit 1 bis 4 Kohlenstoffatomen in den einzelnen Alkylteilen und gegebenenfalls 1 bis 9, vorzugsweise 1 bis 5, gleichen oder verschiedenen Halogenatomen, für Alkinyl mit 2 bis 4 Kohlenstoffatomen, Cycloalkyl mit 3 bis 6 Kohlenstoffatomen, für jeweils unsubstituiertes oder einfach bis dreifach, gleich oder verschieden substituiertes Phenylcarbonyl, Phenylalkyl, Phenylalkyloxy und 1 bis 4 Kohlenstoffstomen im geradkettigen oder verzweigten Alkylteil, wobei als Substituenten die bei R⁵ genannten Substituenten infrage kommen oder für eine Gruppe 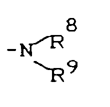 steht,
wobei R⁸ und R⁹ jeweils unabhängig voneinander für Methyl, Ethyl, n- oder i-Propyl, Methylcarbonyl, Ethylcarbonyl oder n- oder i-Propylcarbonyl stehen
oder
- R⁴ und R⁷: gemeinsam mit dem Stickstoffatom, an welches sie gebunden sind, für eine gesättigte Heteroalkylenkette mit 4 oder 5 Kohlenstoffatomen stehen,
- q: für die Zahlen 0 oder 1 steht,
- R²: für unsubstituiertes oder einfach bis dreifach, gleich oder verschieden substituiertes Phenyl steht, wobei als Substituenten genannt seien: Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenethenyl, Phenethinyl, wobei jeweils als Substituenten die schon oben genannten Phenylsubstituenten infrage kommen;
als weiterer Phenylsubstituent sei außerdem der Rest -(CH₂)ₙ-Zₘ-(CH₂)ₚ-R⁵ genannt,
worin
- R⁵: für unsubstituiertes oder einfach bis dreifach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n-oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, infrage kommen,
- Z: für Sauerstoff, Schwefel oder für die Gruppe >C=O steht und
- n, m und p: unabhängig voneinander für die Zahlen 0 oder 1 stehen,
- R³ und R⁶: für Wasserstoff, Methyl, Ethyl, n- oder i-Propyl, n-, i-, s- oder t-Butyl, unsubstituiertes oder einfach bis dreifach, gleich oder verschieden substituiertes Phenyl oder jeweils unsubstituiertes oder einfach bis dreifach, gleich oder verschieden substituiertes Benzyl oder Phenethyl stehen, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, infrage kommen.

Besonders bevorzugt ist auch die Gruppe von Verbindungen der Formel (I), in welcher
- X: für den Rest OR¹ steht, wobei
- R¹: für geradkettiges oder verzweigtes Alkyl mit 1 bis 4 Kohlenstoffatomen oder für jeweils geradkettiges oder verzweigtes Cyanoalkyl, Alkoxyalkyl, Alkylcarbonyl, Halogenalkylcarbonyl oder Alkoxycarbonylalkyl mit 1 bis 6 Kohlenstoffatomen in den einzelnen Alkylteilen und gegebenenfalls 1 bis 9 gleichen oder verschiedenen Halogenatomen steht,
- R³ und R⁶: für Wasserstoff, geradkettiges oder verzweigtes Alkyl mit 1 bis 6 Kohlenstoffatomen, unsubstituiertes oder einfach bis mehrfach, gleich oder verschieden substituiertes Phenyl oder unsubstituiertes oder einfach oder mehrfach, gleich oder verschieden substituiertes Aralkyl mit 6 bis 10 Kohlenstoffatomen im Arylteil und 1 bis 4 Kohlenstoffatomen im Alkylteil stehen, wobei als Substituenten Cyano, Nitro, Halogen, C₁₋₄-Alkyl, C₁₋₄-Alkoxy, Halogen-C₁₋₄-alkyl und Halogen-C₁₋₄-alkoxy infrage kommen, und
entweder
α) R²⁻¹ für in meta- oder para-Position, gleich oder verschieden, mono-, di- oder trisubstituiertes Phenyl steht, wobei als Substituenten genannt seien:
   Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6, insbesondere 1 bis 4, Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen;
   als weiterer Phenylsubstituent sei außerdem der Rest -(CH₂)ₙ-Zₘ-(CH₂)ₚ-R⁵ genannt,
worin
- R⁵: für unsubstituiertes oder einfach bis mehrfach, gleich oder verschieden substituiertes Aryl mit 6 bis 10 Kohlenstoffatomen steht, wobei als Substituenten Cyano, Nitro, Halogen, jeweils geradkettiges oder verzweigtes Alkyl oder Alkoxy mit 1 bis 6, insbesondere 1 bis 4 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen,
- Z: für Sauerstoff, Schwefel oder für die Gruppe >C=O steht und
- n, m und p: für die Zahlen 0 oder 1 stehen und
- q: für die Zahlen 0 oder 1 steht,
ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on, 4-Methoxy-3-(4-methoxyphenyl)-5H-furan-2-on, 5-(α-Methoxycarbonyl-α-hydroxy-4-methoxybenzyl)-4-methoxy-3-(3,4,5-trimethoxyphenyl)-5H-furan-2-on 5-(α-Methoxycarbonyl-α-hydroxy-benzyl)-4-methoxy-3-(4-methoxyphenyl-5H-furan-2-on und 5-(α-Hydroxy-4-methoxybenzyl)-4-methoxy-3-(4-methoxyphenyl)-5H-furan-2-on (vgl. Tetrahedron Lett., 29 (17), 2085-8, 1988; J. Chem. Soc., Chem. Commun. (16), 660-1, 1976; J. Chem. Soc., Perkin Trans. 1, (8), 1567-76, 1985),
oder
β) R²⁻² für einfach in ortho-Position substituiertes Phenyl steht,
   wobei als Substituenten genannt seien: Cyano, Nitro, Halogen, jeweils geradkettiges oder verzweigtes Alkyl oder Alkoxy mit 1 bis 6, insbesondere 1 bis 4 Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6, insbesondere 1 bis 4 Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, infrage kommen;
   als weiterer Phenylsubstituent sei außerdem der Rest -(CH₂)ₙ-Zₘ-(CH₂)ₚ-R⁵ genannt,
- R⁵: für unsubstituiertes oder einfach bis mehrfach, gleich oder verschieden substituiertes Aryl mit 6 bis 10 Kohlenstoffatomen steht, wobei als Substituenten Cyano, Nitro, Halogen, jeweils geradkettiges oder verzweigtes Alkyl oder Alkoxy mit 1 bis 6, insbesondere 1 bis 4 Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, infrage kommen,
- Z: für Sauerstoff, Schwefel oder für die Gruppe >C=O steht und
- n, m und p: für die Zahlen 0 oder 1 stehen und
- q: für die Zahl 1 steht, oder
γ) R²⁻³ für einfach in ortho-Position substituiertes Phenyl steht,
   wobei als Substituenten genannt seien: Halogen, Halogenmethyl mit 1 bis 3 gleichen oder verschiedenen Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6, insbesondere 1 bis 4 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen und
- q: für die Zahl 0 steht,
ausgenommen die Verbindungen 3-(2-Chlorphenyl)-4-methoxy-5H-furan-2-on, 3-(2-Fluorphenyl)-4-methoxy-5H-furan-2-on, 4-Methoxy-3-(2-methoxyphenyl)-5H-furan-2-on (vgl. J. Chem. Soc., Perkin Trans. 1, (8) 1567-76, 1985).

Ganz besonders bevorzugt ist die Gruppe von Verbindungen der Formel (I), in welcher
- X: für den Rest OR¹ steht, wobei
- R¹: für Methyl, Ethyl, n- oder i-Propyl, n-, i-, s- oder t-Butyl oder für jeweils geradkettiges oder verzweigtes Cyanoalkyl, Alkoxyalkyl oder Alkoxycarbonylalkyl mit 1 bis 4 Kohlenstoffatomen in den einzelnen Alkylteilen steht,
- R³ und R⁶: für Wasserstoff, Methyl, Ethyl, n- oder i-Propyl, n-, i-, s- oder t-Butyl, unsubstituiertes oder einfach bis dreifach, gleich oder verschieden substituiertes Phenyl oder unsubstituiertes oder einfach bis dreifach, gleich oder verschieden substituiertes Benzyl oder Phenethyl stehen, wobei als Substituenten jeweils Cyano, Nitro, Fluor, Chlor, Brom, Methyl, Ethyl, n- oder i-Propyl, Methoxy, Ethoxy, n- oder i-Propoxy, jeweils geradkettiges Halogenalkyl oder Halogenalkoxy mit 1 oder 2 Kohlenstoffatomen und 1 bis 5 gleichen oder verschiedenen Fluor- oder Chloratomen, infrage kommen;
entweder
α) R²⁻¹ für in meta- oder para-Position, gleich oder verschieden, einfach, di- oder trisubstituiertes Phenyl steht, wobei als Substituenten genannt seien: Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenylethenyl oder Phenethinyl, wobei jeweils an Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen;
   als weiterer Phenylsubstituent sei außerdem der Rest -(CH₂)ₙ-Zₘ-(CH₂)ₚ-R⁵ genannt,
   worin
   - R⁵: für unsubstituiertes oder einfach bis dreifach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen,
   - Z: für Sauerstoff, Schwefel oder für die Gruppe >C=O steht und
   - n, m und p: für die Zahlen 0 oder 1 stehen und
   - q: für die Zahlen 0 oder 1 steht,
ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on, 4-Methoxy-3-(4-methoxyphenyl)-5H-furan-2-on, 5-(α-Methoxycarbonyl-α-hydroxy-4-methoxybenzyl)-4-methoxy-3-(3,4,5-trimethoxyphenyl)-5H-furan-2-on 5-(α-Methoxycarbonyl-α-hydroxy-benzyl)-4-methoxy-3-(4-methoxyphenyl)-5H-furan-2-on und 5-(α-Hydroxy-4-methoxybenzyl)-4-methoxy-3-(4-methoxyphenyl)-5H-furan-2-on (vgl. Tetrahedron Lett., 29 (17), 2085-8, 1988; J. Chem. Soc., Chem. Commun. (16), 660-1, 1976; J. Chem. Soc., Perkin Trans. 1, (8), 1567-76, 1985),
oder
β) R²⁻² für einfach in ortho-Position substituiertes Phenyl steht,
   wobei als Substituenten genannt seien:
   Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenethenyl oder Phenethinyl, wobei jeweils als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen,
   als weiterer Phenylsubstituent sei außerdem der Rest -(CH₂)ₙ-Zₘ-(CH₂)ₚ-R⁵ genannt,
   worin
   - R⁵: für unsubstituiertes oder einfach bis mehrfach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder oder Chloratomen infrage kommen;
   - Z: für Sauerstoff, Schwefel oder für die Gruppe >C=O steht und
   - n, m und p: für die Zahlen 0 oder 1 stehen und
   - q: für die Zahl 1 steht, oder
γ) R²⁻³ für einfach in ortho-Position substituiertes Phenyl steht,
   wobei als Substituenten genannt seien: Fluor, Chlor, Brom, Halogenmethyl mit 1 bis 3 gleichen oder verschiedenen Fluor- oder Chloratomen, jeweils unsubstituiertes oder einfach bis dreifach, gleich oder verschieden substituiertes Phenethenyl oder Phenethinyl, wobei jeweils als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen und
   - q: für die Zahl 0 steht,
   ausgenommen die Verbindungen 3-(2-Chlorphenyl)-4-methoxy-5H-furan-2-on, 3-(2-Fluorphenyl)-4-methoxy-5H-furan-2-on, 4-Methoxy-3-(2-methoxyphenyl)-5H-furan-2-on (vgl. J. Chem. Soc., Perkin Trans. 1, (8) 1567-76, 1985).

Ganz besonders bevorzugt ist die Gruppe von Verbindungen der Formel (I), in welcher
- X: für den Rest 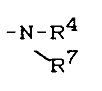 steht,
worin
- R⁴: für Wasserstoff, Methyl, Ethyl, n- oder i-Propyl, Methylcarbonyl oder Ethylcarbonyl steht,
- R⁷: für Wasserstoff, Hydroxy, Amino, Formyl, Methyl, Ethyl, n- oder i-Propyl, n-, i-, s- oder t-Butyl, Methoxy, Ethoxy, n- oder i-Propoxy, n-, i-, s- oder t-Butoxy, Methoxymethyl, Ethoxymethyl, Methoxyethyl, Ethoxyethyl, Cyanomethyl, Cyanoethyl, Methylcarbonyl, Ethylcarbonyl, n- oder i-Propylcarbonyl, n-, i-, s- oder t-Butylcarbonyl, Halogenalkylcarbonyl mit 1 bis 2 Kohlenstoffatomen und 1 bis 5 gleichen oder verschiedenen Fluor- oder Chloratomen, Methoxycarbonylmethyl, Ethoxycarbonylmethyl, Methoxycarbonylethyl, Ethoxycarbonylethyl, Methylamino, Ethylamino, n- oder i-Propylamino, Methylcarbonyloxy, Ethylcarbonyloxy, n- oder i-Propylcarbonyloxy, Aminocabonylmethyl, Aminocarbonylethyl, Propinyl, Butinyl, Cyclopropyl, Cyclopentyl, Cyclohexyl, Phenylcarbonyl, Phenylalkyl und Phenylalkyloxy mit jeweils 1 oder 2 Kohlenstoffatomen im jeweiligen Alkylteil oder für eine Gruppe 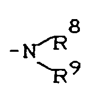 steht
wobei R⁸ und R⁹ jeweils unabhängig voneinander für Methyl, Ethyl, oder Methylcarbonyl stehen
oder
- R⁴ und R⁷: gemeinsam mit dem Stickstoffatom, an welches sie gebunden sind für eine gesättigte Heteroalkylenkette mit 4 oder 5 Kohlenstoffatomen stehen,
- q: für die Zahlen 0 oder 1 steht,
- R²: für unsubstituiertes oder einfach bis dreifach, gleich oder verschieden substituiertes Phenyl steht, wobei mindestens einer der Substituenten aus der Reihe Trifluormethyl, Trifluormethoxy, Fluor, Chlor, Brom in meta-Position steht und gegebenenfalls weitere Substituenten aus der Reihe Fluor, Chlor, Brom, Methyl, Ethyl, n- oder i-Propyl in ortho- oder para-Position stehen; als weiterer Phenylsubstituent sei außerdem der Rest -(CH₂)ₙ-Zₘ-(CH₂)ₚ-R⁵ genannt,
worin
- R⁵: für unsubstituiertes oder einfach bis dreifach, gleich oder verschieden substituiertes Phenyl steht, wobei als Substituenten Fluor, Chlor, Brom, Trifluormethyl, Trifluormethoxy, Methyl, Ethyl, n- oder i-Propyl infrage kommen,
- Z: für Sauerstoff, Schwefel oder für die Gruppe >C=O steht,
- n, m und p: für die Zahlen 0 oder 1 stehen, und
- R³ und R⁶: für Wasserstoff, Methyl, Ethyl, n- oder i-Propyl, n-, i-, s- oder t-Butyl, unsubstituiertes oder einfach oder zweifach, gleich oder verschieden substituiertes Phenyl oder Benzyl stehen, wobei als Substituenten jeweils Fluor, Chlor, Brom, Methyl, Ethyl, n-oder i-Propyl, Trifluormethyl und Trifluormethoxy infrage kommen, ausgenommen die Verbindung 5,5-Dimethyl-4-amino-3-phenyl-5H-furan-2-on.

Verwendet man als Ausgangsstoffe beispielsweise 4-Methoxy-3-(3-trifluormethyl-phenyl)-5H-furan-2-on und Dimethylamin-Hydrochlorid, so läßt sich der Reaktionsablauf zur Herstellung der substituierten 5H-Furan-2-on-Derivate der Formel (I) gemäß Verfahrensvariante a-α) (z.B. für R¹ = R⁴ = CH₃, und X = NR⁴) durch das folgende Formelschema beschreiben: 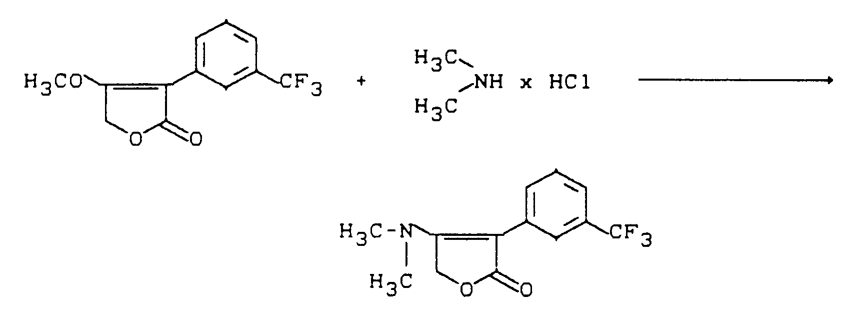
Verwendet man als Ausgangsstoffe beispielsweise 4-Amino-3-(3-trifluormethylphenyl)-5H-furan-2-on und Acetylchlorid, so läßt sich der Reaktionsverlauf des erfindungsgemäßen Verfahrens (a-β) durch das folgende Formelschema beschreiben: 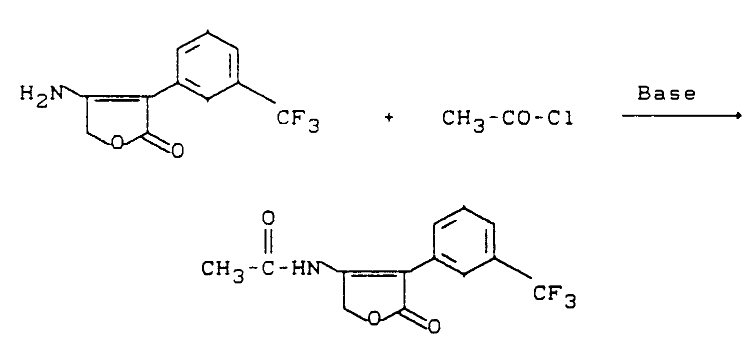
Verwendet man als Ausgangsstoffe beispielsweise 3-(3-Trifluormethylphenyl)-tetronsäure, Dimethylsulfat und Tetrabutylammoniumhydroxid als Katalysator, so läßt sich der Reaktionsablauf zur Herstellung der substituierten 5H-Furan-2-on-Derivate der Formel (I) gemäß Verfahrensvariante b) (z.B. für R¹ = CH₃ und X = O) durch das folgende Formelschema beschreiben: 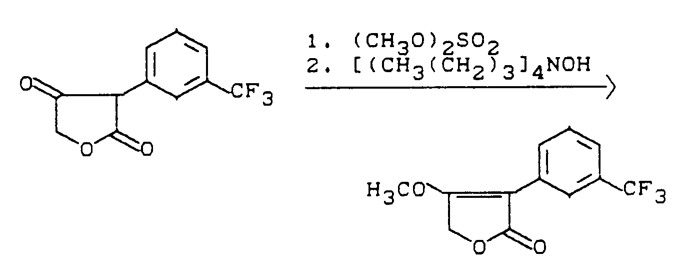
Die als Ausgangsstoffe gemäß Verfahrensvariante a-α) benötigten 5H-Furan-2-on-Derivate der Formel (II) 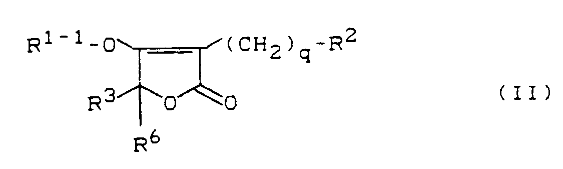
in welcher
- R¹⁻¹, R², R³, R⁶ und q: diejenige Bedeutung haben, die bereits im Zusammenhang mit der Beschreibung der Stoffe der Formel (I) für diese Substituenten genannt wurden,
sind teilweise bekannt (vgl. z.B. Tetrahedron Lett., 29, 2085-2088, 1988; Can. J. Chem., 64, 104-109, 1986; J. Chem. Soc., Perkin Trans. 1, 1567-76, 1985; J.Chem. Soc. Perkin Trans. 1, 1539-45, 1984; Tetrahedron, 35, 2181-2185, 1979; J.Chem. Soc. Perkin Trans. 1, 70-76, 1979; J. Chem. Soc., Perkin Trans. 1, 62-69, 1979; J. Chem. Soc., Perkin Trans. 1, 84-88, 1979; J. Chem. Soc., Chem. Commun. 660-661, 1976; J.Chem. Soc., Chem. Commun. 635-637, 1976; Tetrahedron Lett. 4279-4282, 1975; J. Chem. Soc., Chem. Commun., 876-877, 1975; JP6917901) und/oder erhältlich in Analogie zu bekannten Verfahren.

Die weiterhin als Ausgangsstoffe zur Herstellung der substituierten 5H-Furan-2-on-Derivate der Formel (I) benötigten Amine sind durch die Formel (III) allgemein definiert. In dieser Formel (III) stehen R⁷⁻⁰ und R⁴ vorzugsweise für diejenigen Reste, die bereits im Zusammenhang mit der Beschreibung der erfindungsgemäßen Stoffe der Formel (I) als bevorzugt für R¹ mit Ausnahme von Alkylcarbonyl und Halogenalkylcarbonyl und R⁴ genannt wurden.

Die Amine der Formel (III) sind allgemein bekannte Verbindungen der organischen Chemie.

Die als Ausgangsstoffe für Verfahren (a-β) benötigten 5H-Furan-2-on-Derivate der Formel (Ia) sind erfindungsgemäße Verbindungen und erhältlich nach Verfahren (a-α).

Die weiterhin als Ausgangsstoffe benötigten Acylierungsmitteln der Formel (VI) sind bekannte Verbindungen der organischen Chemie.

In Formel (VI) steht R⁷⁻¹ vorzugsweise für Alkylcarbonyl oder Halogenalkylcarbonyl mit jeweils 1 bis 6, insbesondere 1 bis 4, Kohlenstoffatomen und gegebenenfalls 1 bis 9, insbesondere 1 bis 5 gleichen oder verschiedenen Halogenatomen (vgl. entsprechende Definition für R⁷). E² steht vorzugsweise für Halogen, insbesondere für Chlor oder Brom, Alkoxysulfonyloxy mit vorzugsweise 1 bis 4 Kohlenstoffatomen, insbesondere für Methoxysulfonyloxy oder Ethoxysulfonyloxy, oder für Arylsulfonyloxy, insbesondere für p-Toluolsulfonyloxy.

Die als Ausgangsstoffe gemäß Verfahrensvariante b) zur Herstellung der substituierten 5H-Furan-2-on-Derivate der Formel (I) benötigten substituierten Tetronsäure-Derivate sind durch die Formel (IV) allgemein definiert. In dieser Formel (IV) haben R², R³, R⁶ und q vorzugsweise diejenige Bedeutung, die bereits mit der Beschreibung der erfindungsgemäßen Stoffe der Formel (I) vorzugsweise für diese Substituenten genannt wurden.

Die substituierten Tetronsäure-Derivate der Formel (IV) sind teilweise bekannt und/oder können nach prinzipiell bekannten Verfahren hergestellt werden (vgl. z.B. EP-OS 259 707, Arch. Pharm. (Weinheim) 291, 100 (1958) und Herstellungsbeispiele).

Die zur Durchführung des erfindungsgemäßen Verfahrens gemäß Verfahrensvariante (b) weiterhin als Ausgangsstoffe benötigten Alkylierungs- oder Acylierungsmittel sind durch die Formel (V) allgemein definiert. In dieser Formel (V) steht R¹ vorzugsweise für diejenigen Reste, die bereits im Zusammenhang mit der Beschreibung der erfindungsgemäßen Stoffe der Formel (I) als bevorzugt für diesen Substituenten genannt wurden.
- E¹: steht für eine bei Alkylierungs- oder Acylierungsmitteln übliche Abgangsgruppe, vorzugsweise für einen gegebenenfalls substituierten Alkyl-, Alkoxy- oder Arylsulfonyloxyrest, wie beispielsweise ein Methoxysulfonyloxyrest, ein Ethoxysulfonyloxyrest oder ein p-Toluolsulfonyloxyrest oder für Halogen, insbesondere für Chlor, Brom oder Iod.

Die Alkylierungs- und Acylierungsmittel der Formel (V) sind allgemein bekannte Verbindungen der organischen Chemie.

Das erfindungsgemäße Verfahren (a-α) zur Herstellung der neuen substituierten 5H-Furan-2-on-Derivate der Formel (I) wird vorzugsweise unter Verwendung von Verdünnungsmitteln durchgeführt.

Als Verdünnungsmittel kommen dabei praktisch alle für diese Umsetzung üblichen inerten organischen Lösungsmittel infrage, insbesondere Alkohole, wie Methanol oder Ethanol; Ether, wie Diethylether, Dioxan, Tetrahydrofuran, Ethylenglykoldimethyl- oder -diethylether oder Amide, wie Diemthylformamid, Dimethylacetamid, N-Methylformanilid, N-Methylpyrrolidon oder Hexamethylphosphorsäuretriamid.

Die Reaktionstemperaturen können bei der Durchführung des erfindungsgemäßen Verfahrens (a-α) zur Herstellung der neuen substituierten 5H-Furan-2-on-Derivate der Formel (I) in einem größeren Bereich variiert werden. Im allgemeinen arbeitet man bei Temperaturen zwischen 0°C und 150°C, vorzugsweise bei Temperaturen zwischen 20°C und 140°C.

Bei der Durchführung des erfindungsgemäßen Verfahrens (a-α) zur Herstellung der neuen substituierten 5H-Furan-2-on-Derivate der Formel (I) setzt man pro Mol 5H-Furan-2-on-Derivat der Formel (II) im allgemeinen 1 bis 5 Mol, vorzugsweise 1 bis 3 Mol Amin der Formel (III) ein.

Das erfindungsgemäße Verfahren (a-α) zur Herstellung der neuen substituierten 5H-Furan-2-on-Derivate der Formel (I) können bei Normaldruck, aber auch unter erhöhtem Druck, durchgeführt werden. Im allgemeinen arbeitet man bei einem Druck von 1 bis 50 bar, vorzugsweise bei einem Druck von 1 bis 10 bar.

Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden. Im allgemeinen verfährt man in der Weise, daß man das Reaktionsgemisch filtriert, unter vermindertem Druck einengt und das Produkt durch Chromatographie reinigt.

Das erfindungsgemäße Verfahren zur Herstellung der neuen substituierten 5H-Furan-2-on-Derivate der Formel (I) gemäß Verfahrensvariante (a-β) und (b) wird vorzugsweise unter Verwendung von Verdünnungsmitteln durchgeführt.

Als Verdünnungsmittel kommen alle für diese Umsetzung üblichen inerten organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform oder Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid. Verwendet man als Reaktionspartner in den Verfahren (a-β) und b) Verbindungen der Formeln (V) und (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (a-β) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a-β) in einem größeren Bereich variiert werden. Im allgemeinen arbeitet man zwischen -20°C und +150°C, vorzugsweise zwischen 0°C und +100°C.

Zur Durchführung des erfindungsgemäßen Verfahrens (a-β) setzt man pro Mol an 5H-Furan-2-on-Derivat der Formel (Ia) im allgemeinen 1 bis 20 Mol, vorzugsweise 1 bis 15 Mol, an Acylierungsmittel der Formel (VI) und gegebebenenfalls 1 bis 3 Mol, vorzugsweise 1 bis 2 Mol, an Reaktionshilfsmittel ein.

Das erfindungsgemäße Verfahren zur Herstellung der neuen substituierten 5H-Furan-2-on-Derivate der Formel (I) gemäß Verfahrensvariante (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden.

Als Beispiele für solche Katalysatoren seien genannt: Tetrabutylammoniumiodid, Tetrabutylammoniumbromid, Tributyl-methylphosphoniumbromid, Trimethyl-C₁₃/C₁₅-alkylammoniumchlorid, Dibenzyl-dimethyl-ammoniummethylsulfat, Dimethyl-C₁₂/C₁₄-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die Reaktionstemperaturen können bei der Durchführung des erfindungsgemäßen Verfahrens zur Herstellung der neuen substituierten 5H-Furan-2-on-Derivate der Formel (I) gemäß Verfahrensvariante b) in einem größeren Bereich variiert werden. Im allgemeinen arbeitet man bei Temperaturen zwischen 0°C und 80°C, vorzugsweise bei Temperaturen zwischen 10°C und 50°C.

Bei der Durchführung des erfindungsgemäßen Verfahrens (b) zur Herstellung der neuen substituierten 5H-Furan-2-on-Derivate der Formel (I) setzt man pro Mol an Tetronsäure-Derivat der Formel (IV), im allgemeinen 1,0 bis 3 Mol, vorzugsweise 1,0 bis 1,5 Mol an Alkylierungsmittel der Formel (V) und 0,1 bis 2 Mol, vorzugsweise 0,5 bis 1,5 Mol an Katalysator ein.

Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt jeweils nach üblichen Methoden, Im allgemeinen verfährt man in der Weise, daß man das Reaktionsgemisch entweder unter vermindertem Druck einengt und das Produkt durch Chromatographie reinigt.

Die erfindungsgemäß verwendbaren Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäß verwendbaren Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäß verwendbaren Wirkstoffe eignen sich sehr gut zur selektiven Bekämpfung mono- und dikotyler Unkräuter in dikotylen Kulturen, insbesondere im Vorauflaufverfahren.

Darüber hinaus zeigen einige der erfindungsgemäß zu verwendenden Wirkstoffe auch eine fungizide Wirkung. Sie lassen sich insbesondere gegen Getreidemehltau und Oomyceten mit guter Wirkung einsetzen.

Die erfindungsgemäß verwendbaren Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischung mit 4-(2,4-Dichlorphenoxy)-buttersäure (2,4-DB); Chloressigsäure-N-(methoxymethyl)-2,6-diethylanilid (ALACHLOR); 3-Isopropyl-2,1,3-benzothiadiazin-4-on-2,2-dioxid (BENTAZON); Ethyl-2-{[(4-chlor-6-methoxy-2-pyrimidinyl)-aminocarbonyl]-aminosulfonyl}-benzoat (CHLORIMURON); exo-1-Methyl-4-(1-methylethyl)-2-(2-methylphenyl-methoxy)-7-oxabicyclo-(2,2,1)-heptan (CINMETHYLIN); 2-[(2-Chlorphenyl)-methyl]-4,4-dimethylisoxazolidin-3-on (DIMETHAZONE); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); 2-[4-(5-Trifluormethyl-2-pyridyloxy)-phenoxy]-propansäure oder deren Butylester (FLUAZIFOP); N,N-Dimethyl-N'-(3-trifluormethylphenyl)-harnstoff (FLUOMETURON); 1-Methyl-3-phenyl-5-(3-trifluormethylphenyl)-4-pyridon (FLURIDONE); 2-{4-[(3-Chlor-5-(trifluormethyl)-2-pyridinyl)-oxy]-phenoxy}-propansäure bzw. deren Ethylester (HALOXYFOP); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethylpyridin-3-carbonsäure (IMAZETHAPYR); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); N-(1-Ethylpropyl)-3,4-dimethyl-2,6-dinitroanilin (PENDIMETHALIN) und 2-[4-(6-Chlor-chinoxalin-2-yl-oxy)-phenoxy]-propionsäure-ethylester (QUIZALOFOPETHYL) sind möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

### Herstellungsbeispiele

### Beispiel 1:

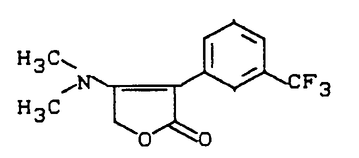

### (Verfahrensvariante a-α)

5,16 g (0,2 Mol) 4-Methoxy-3-(3-trifluormethylphenyl)-1H-furan-2-on werden in 100 ml Methanol gelöst, 0,9 g Dimethylamin in 34 ml Xylol gelöst zugegeben und 0,1 g Dimethylammoniumhydrochlorid zugegeben. Man erhitzt 3 Stunden im Autoklaven bei 110°C, wobei sich ein Druck von 5 bar aufbaut, Nach dem Abkühlen wird entspannt, die Lösung filtriert und das Methanol abdestilliert. Nach Chromatographie über eine Kieselgelsäule (Laufmittel: Methylenchlorid/Methanol 10 : 0,25) erhält man ein Oel, das nach Lösen in 60 ml Diisopropylether in farblosen Kristallen kristallisiert.
Man erhält 4,3 g (79,3 % der Theorie) 4-Dimethylamino-3-(3-trifluormethylphenyl)-5H-furan-2-on vom Schmelzpunkt 110°C.

### Beispiel 2

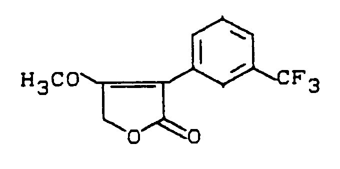

### (Verfahrensvariante b)

2,44 g (0,01 Mol) 3-(3-Trifluormethylphenyl)-tetronsäure werden in 6,5 g (0,01 Mol) 40 %-iger wässriger Tetrabutylammoniumhydroxidlösung, 3 ml Wasser und 4 ml Methylenchlorid gelöst. Man rührt 2 Stunden bei 20°C, trennt die Methylenchloridphase ab, extrahiert die wässrige Phase zweimal mit je 5 ml Methylenchlorid, vereinigt die organischen Phasen und trocknet über Natriumsulfat. Nach dem Abfiltrieren des Trockenmittels werden 1,05 ml (0,011 Mol) Dimethylsulfat zugetropft, 16 Stunden bei 20°C nachgerührt, das Lösungsmittel abdestilliert und das entstehende Oel an Kieselgel mit Methylenchlorid chromatographiert. Das entstehende Oel kristallisiert bei Zusatz von 10 ml Diisopropylether in farblosen Kristallen.
Man erhält 1,5 g (58,1 % der Theorie) 4-Methoxy-3-(3-trifluormethylphenyl)-5H-furan-2-on vom Schmelzpunkt 126°C.

### Beispiel 39

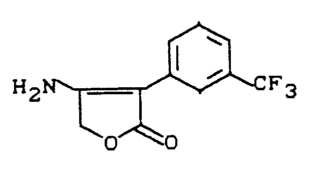

### (Verfahrensvariante a-α)

2,6 g (0,01 Mol) 4-Methoxy-3-(3-trifluormethylphenyl)-5H-furan-2-on werden in 30 ml Methanol suspendiert. Anschließend werden 7 ml (ca. 0,01 Mol) konzentrierte Ammoniaklösung zugegeben. Man rührt ca. 18 h bei Raumtemperatur und dampft dann die klare Lösung ein. Nach Chromatographie über eine Kieselgelsäule (Laufmittel: Methylenchlorid/Methanol 10:0,1) erhält man ein Öl, das auskristallisiert. Die Kristalle werden mit 15 ml Diisopropylether verrührt und abgesaugt.
Man erhält 1,8 g (74 % der Theorie) 4-Amino-3-(3-trifluormethylphenyl)-5H-furan-2-on vom Schmelzpunkt 165°C.

### Beispiel 41

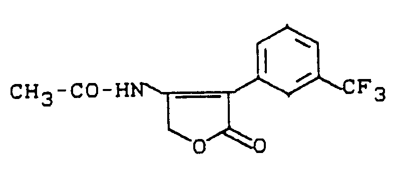

### (Verfahrensvariante a-β)

1,2 g (0,005 Mol) 4-Amino-3-(3-trifluormethylphenyl)-5H-furan-2-on (Beispiel 39) werden in 20 ml Essigsäureanhydrid gelöst. Anschließend wird 0,1 ml Acetylchlorid zugegeben und 22 h bei 130°C gerührt. Die Reaktionsmischung wird dann in 100 ml Wasser eingerührt und zweimal mit je 20 ml Methylenchlorid ausgeschüttelt. Die organischen Phasen werden vereinigt und mit Natriumsulfat getrocknet. Nach dem Abfiltrieren des Trockenmittels wird eingedampft und das entstehende Öl an Kieselgel mit Methylenchlorid chromatographiert. Das entstehende Öl kristallisiert bei Zusatz von 10 ml Diisopropylether in farblosen Kristallen.

Man erhält 0,7 g (49,1 % der Theorie) 4-Methylcarbonylamino-3-(3-trifluormethylphenyl)-5H-furan-2-on vom Schmelzpunkt 140°C.

In analoger Weise zu den in den Beispielen 1, 2, 39 und 41 beschriebenen Methoden und unter Berücksichtigung der Angaben in den Beschreibungen zu den erfindungsgemäßen Verfahren, werden die in der nachfolgenden Tabelle 1 aufgeführten Endprodukte der Formel (I) erhalten.

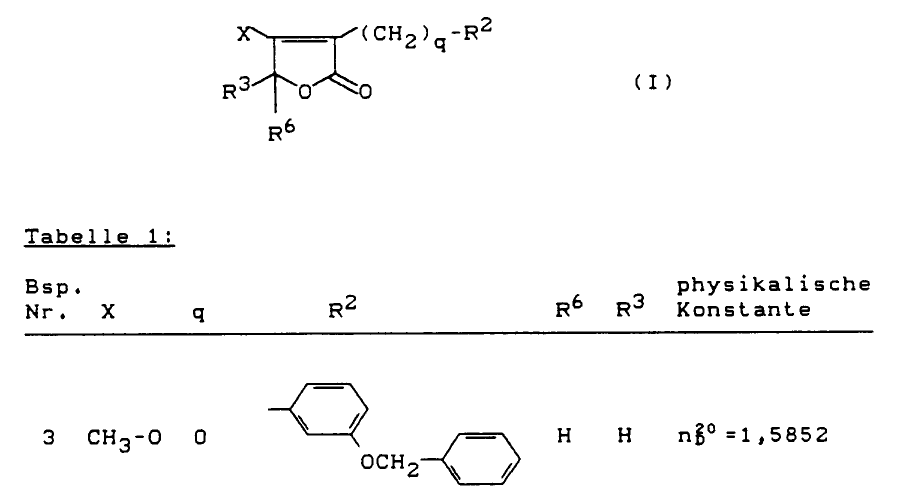

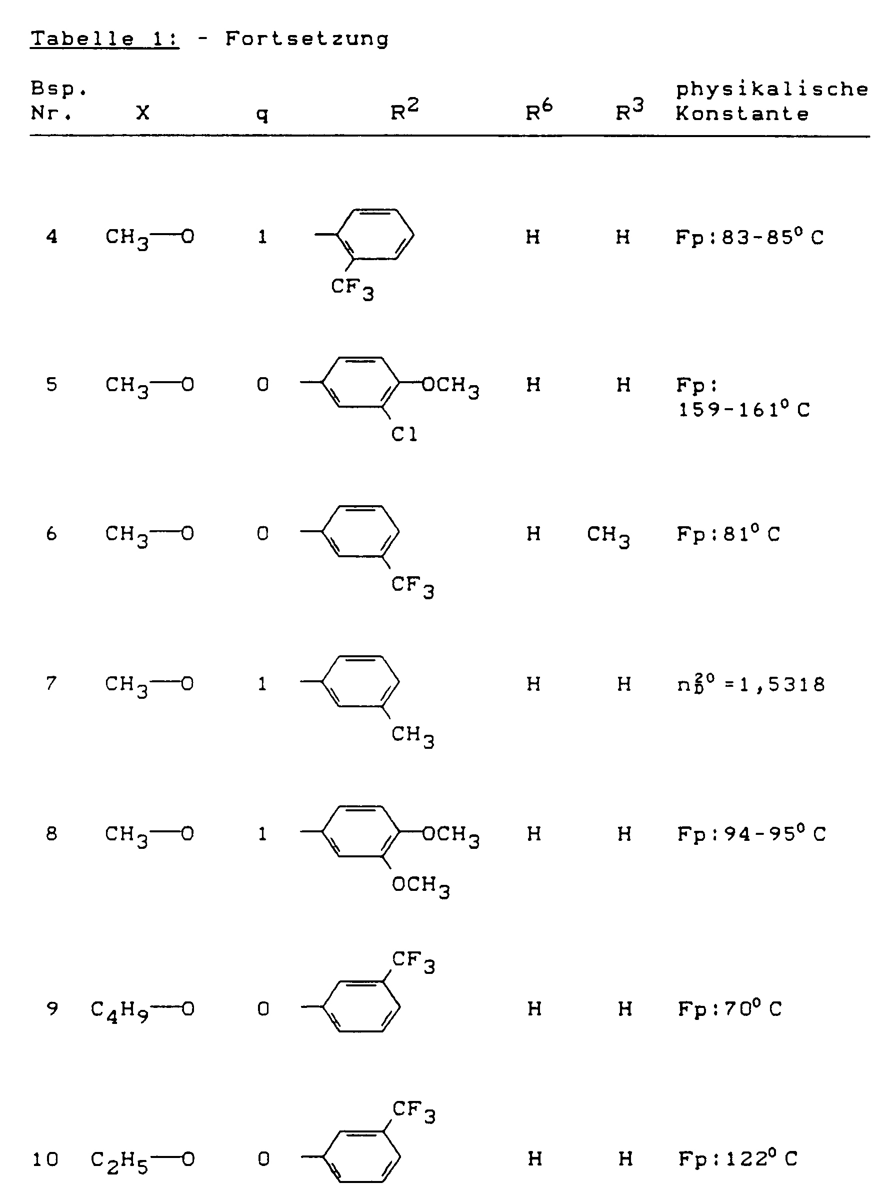

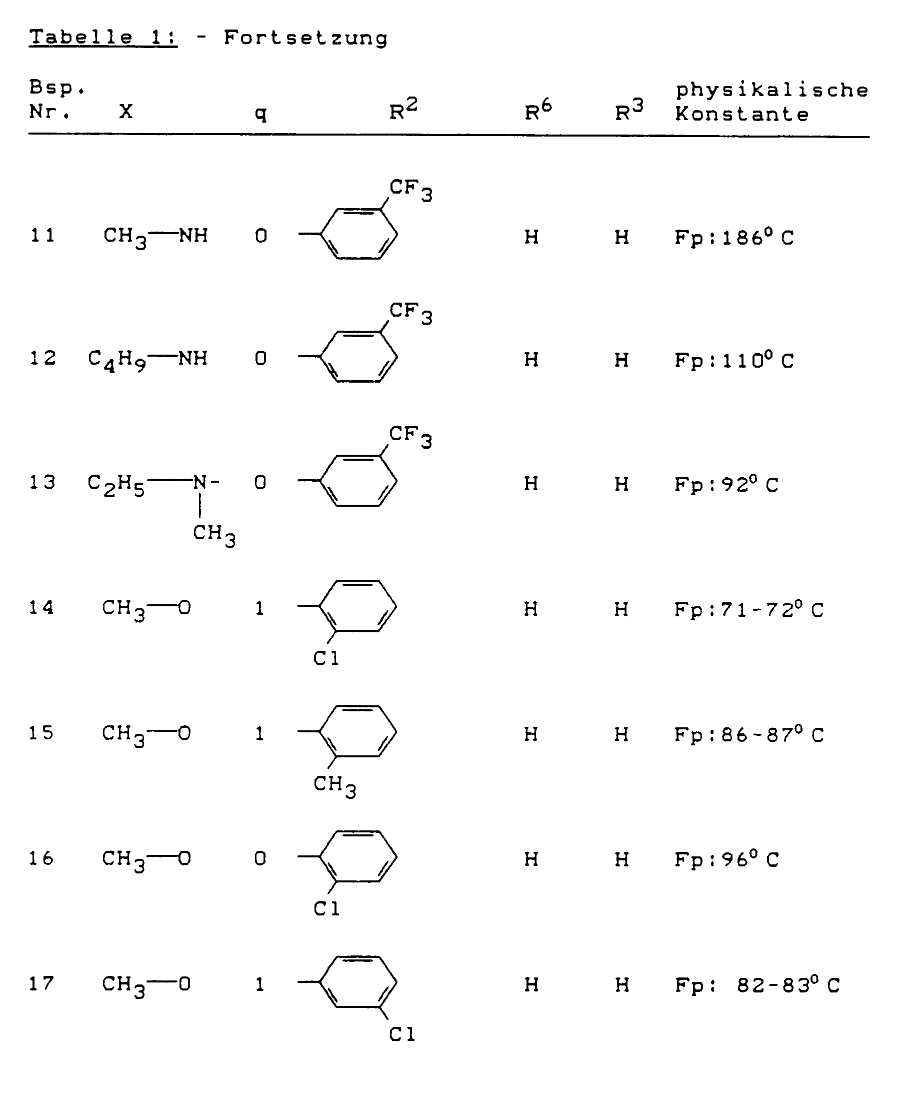

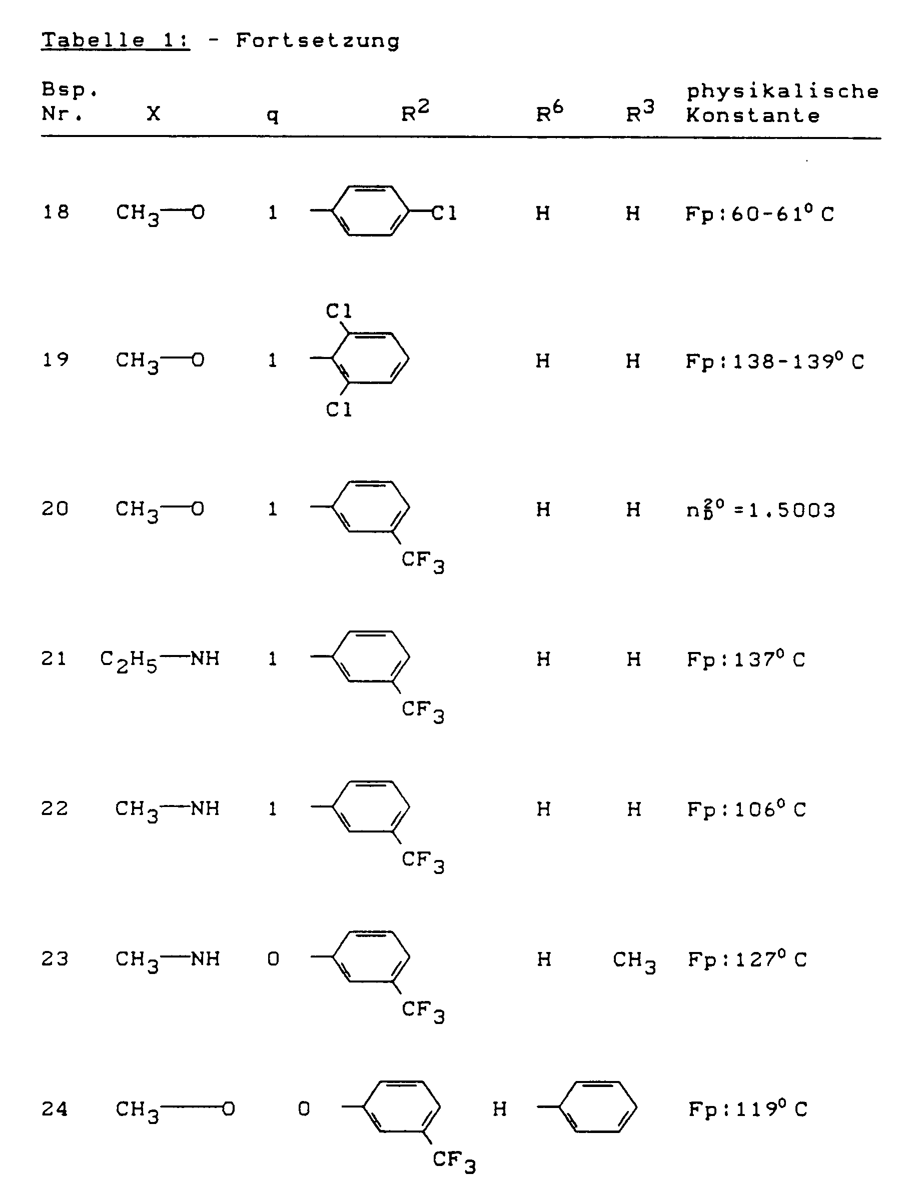

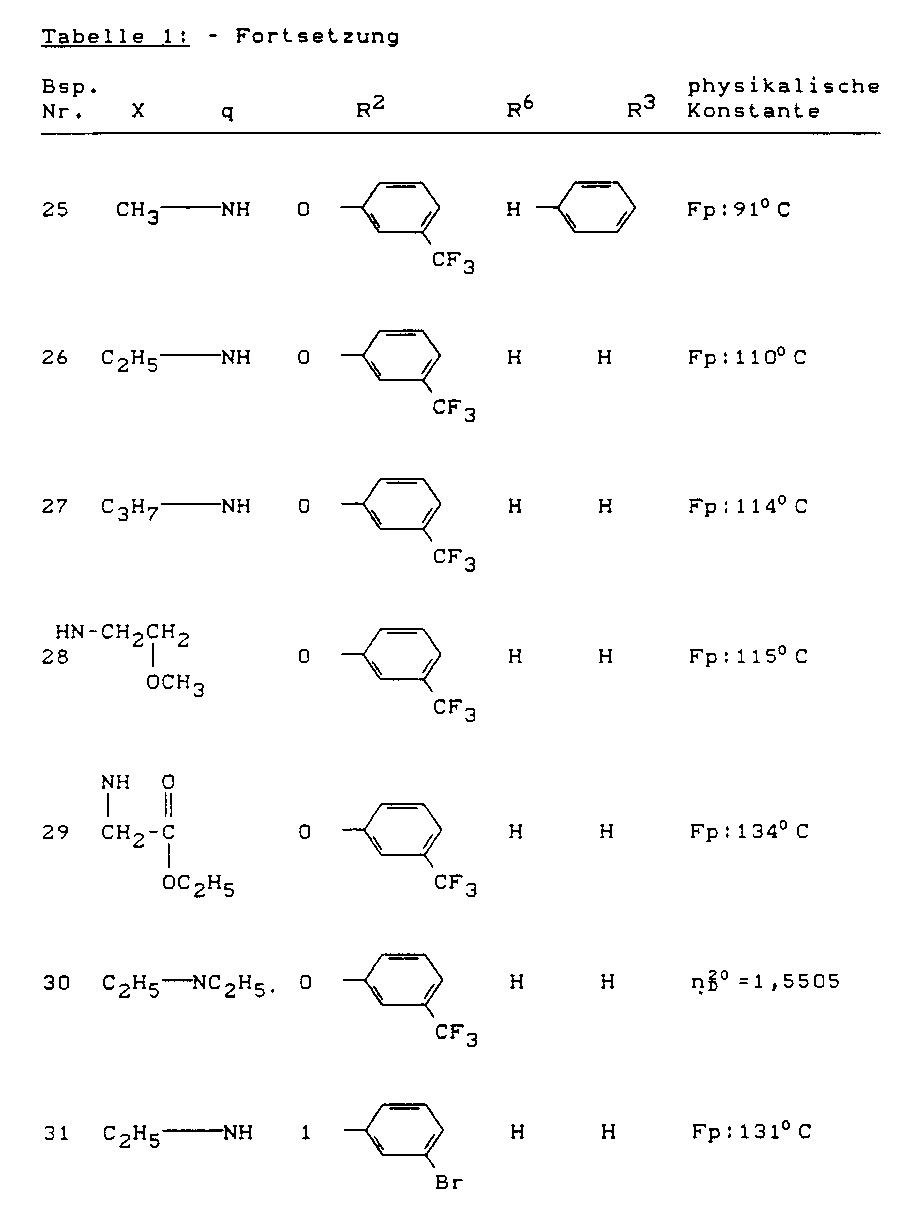

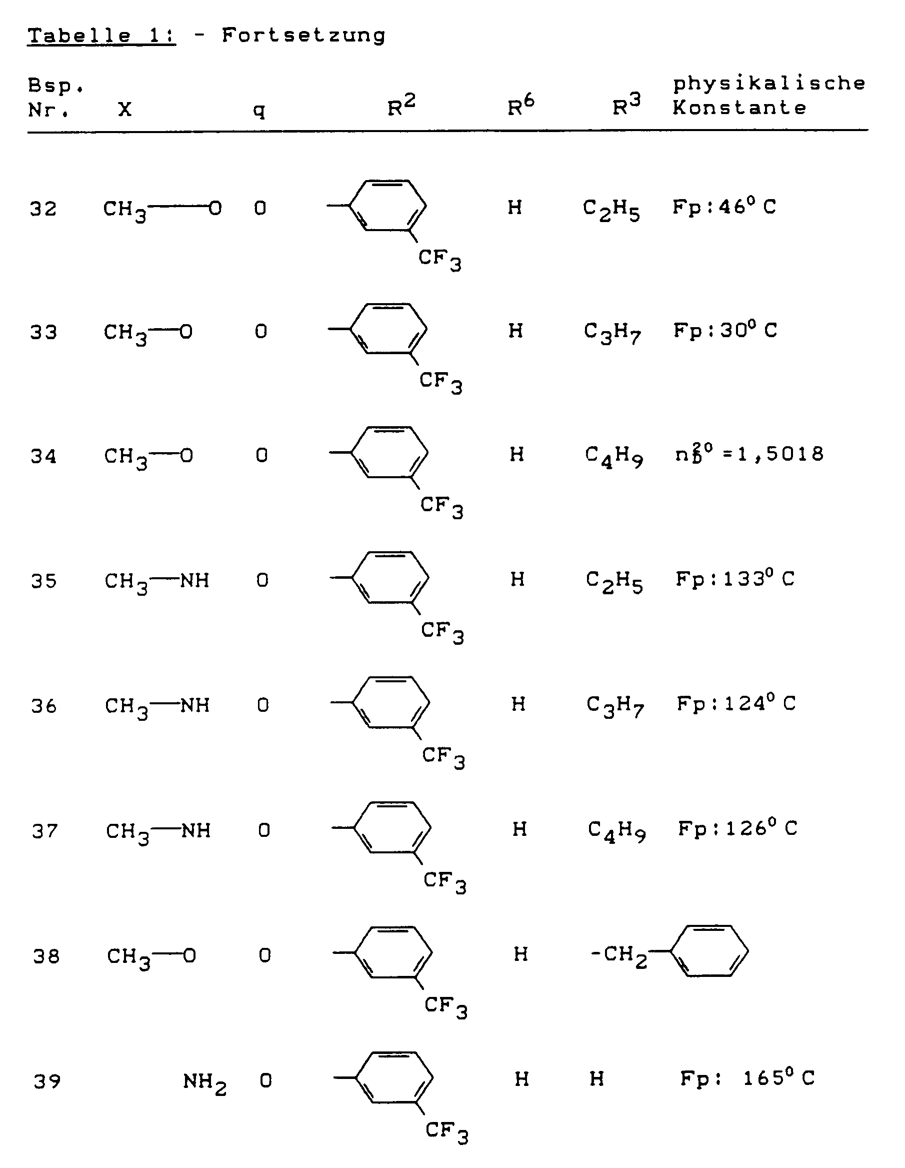

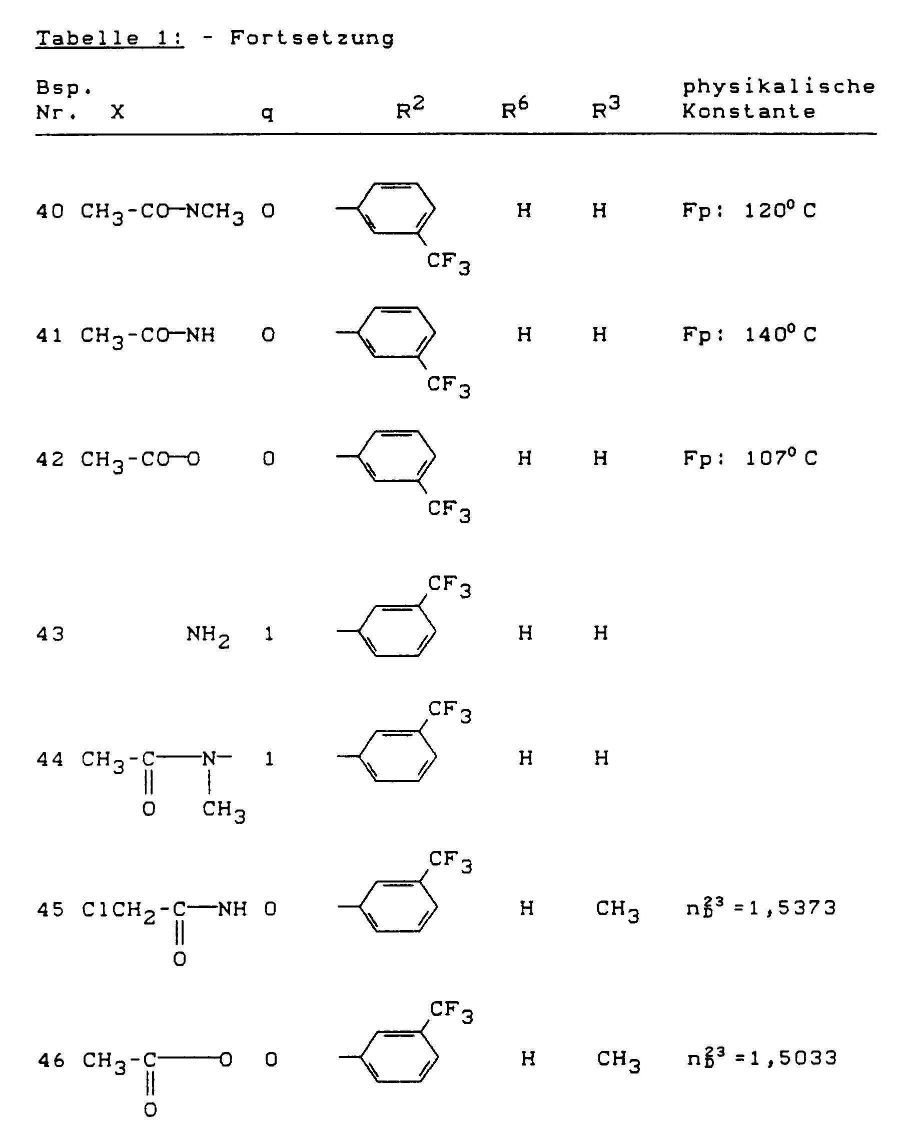

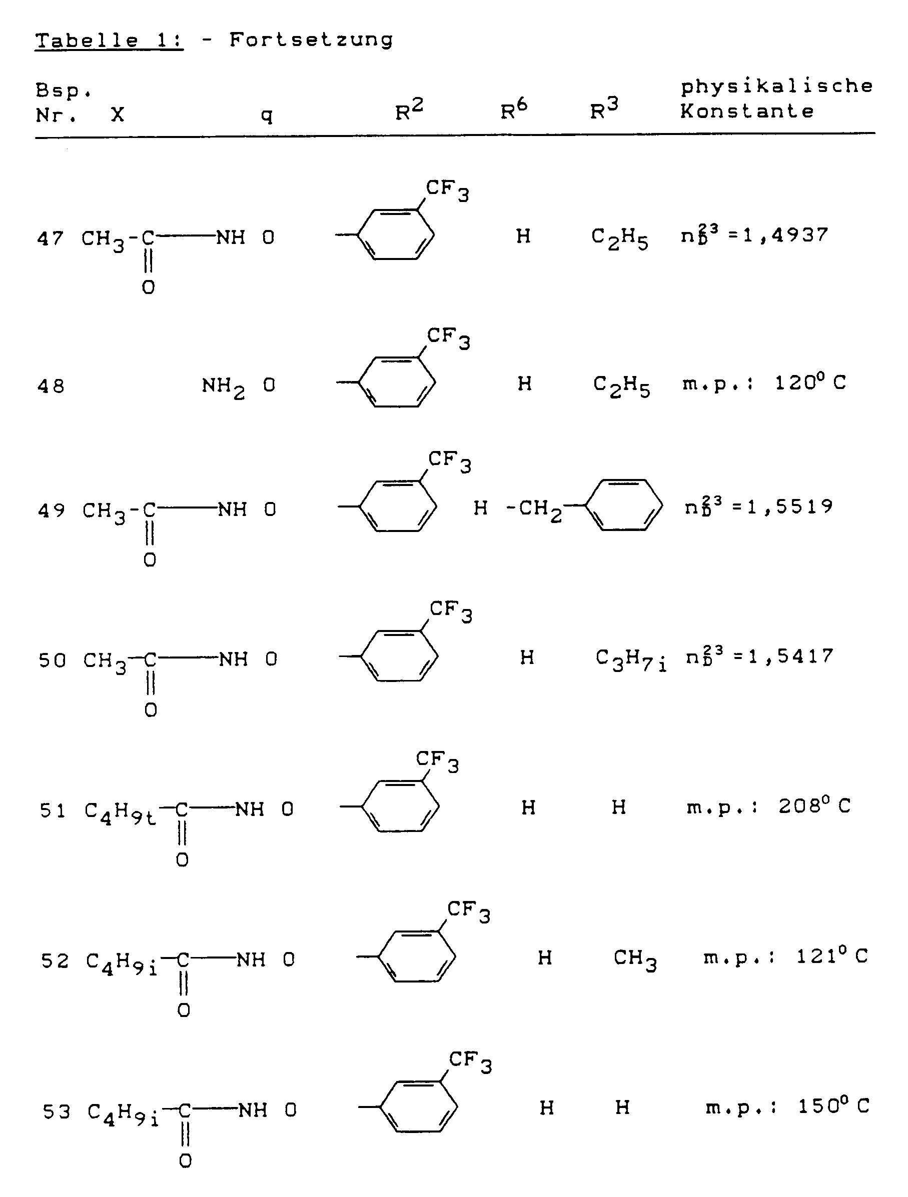

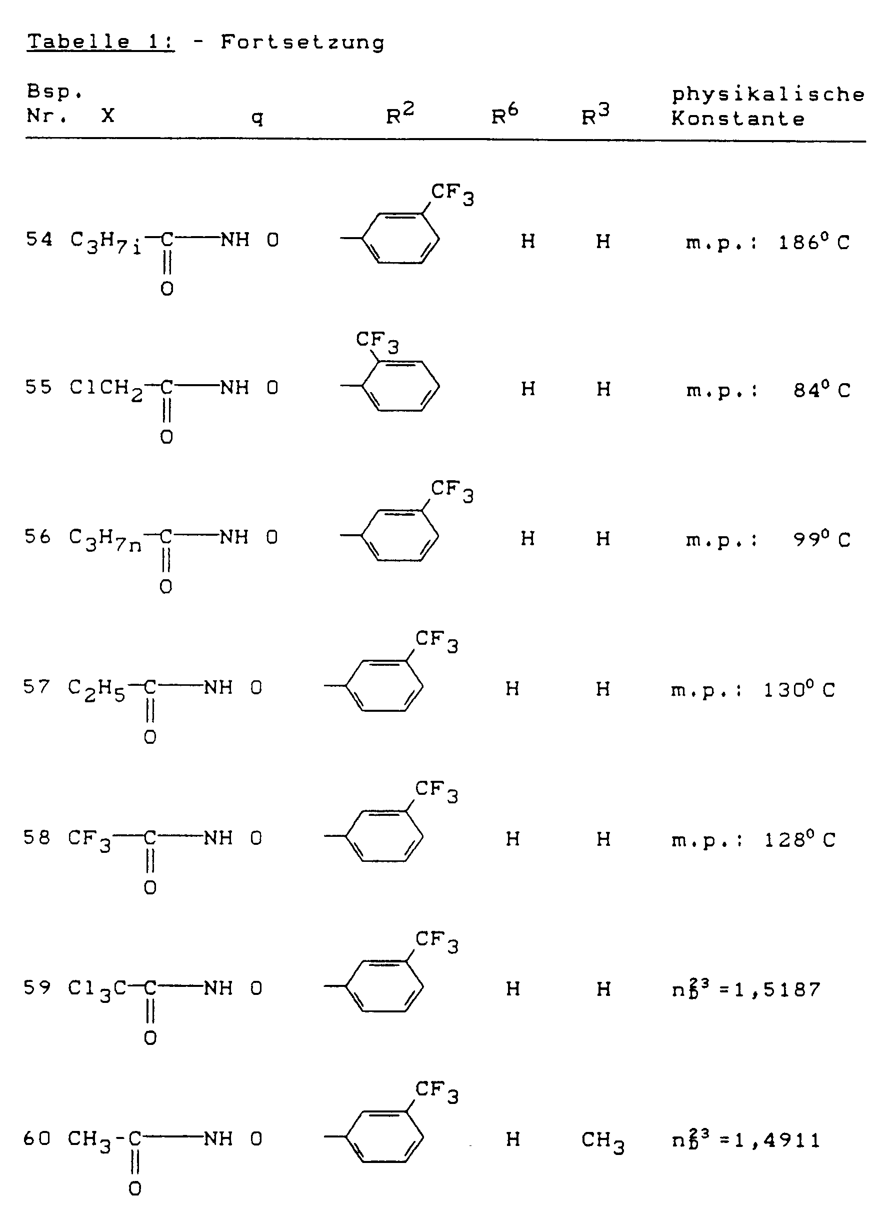

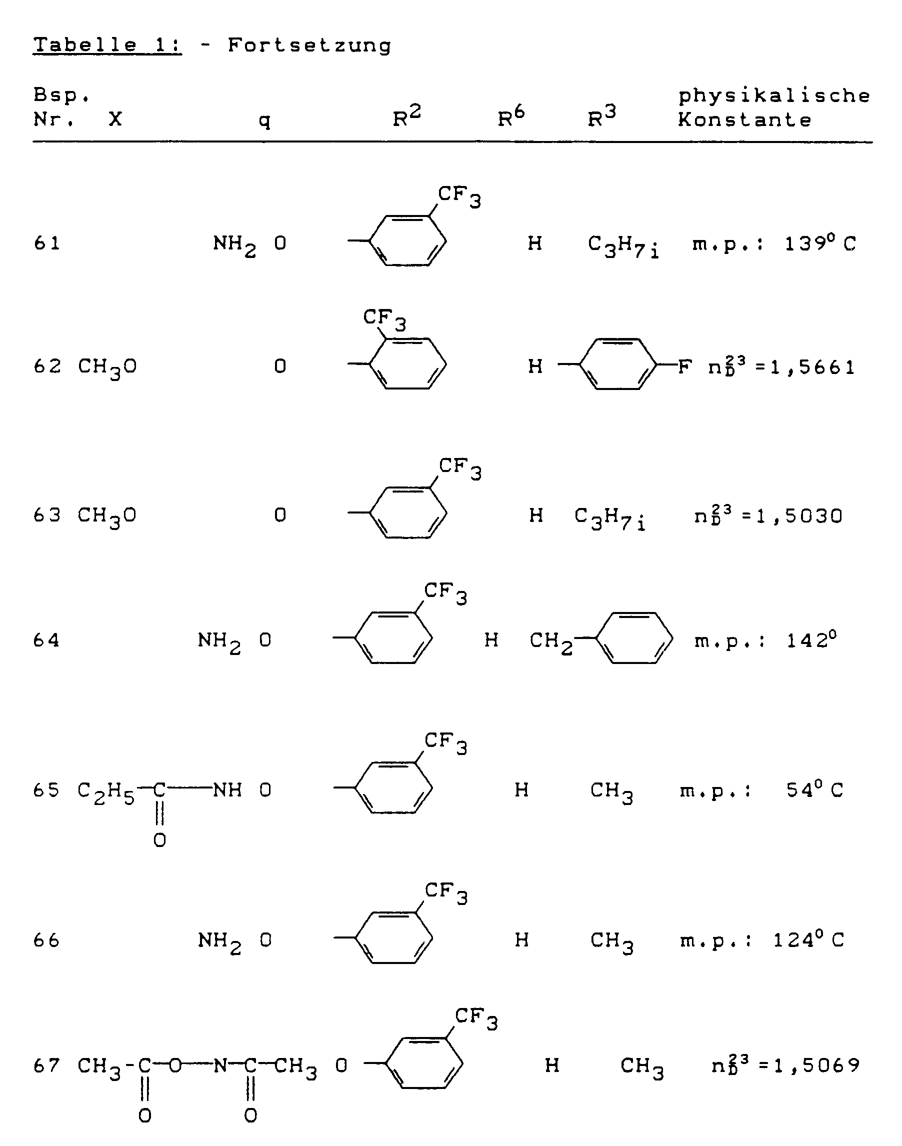

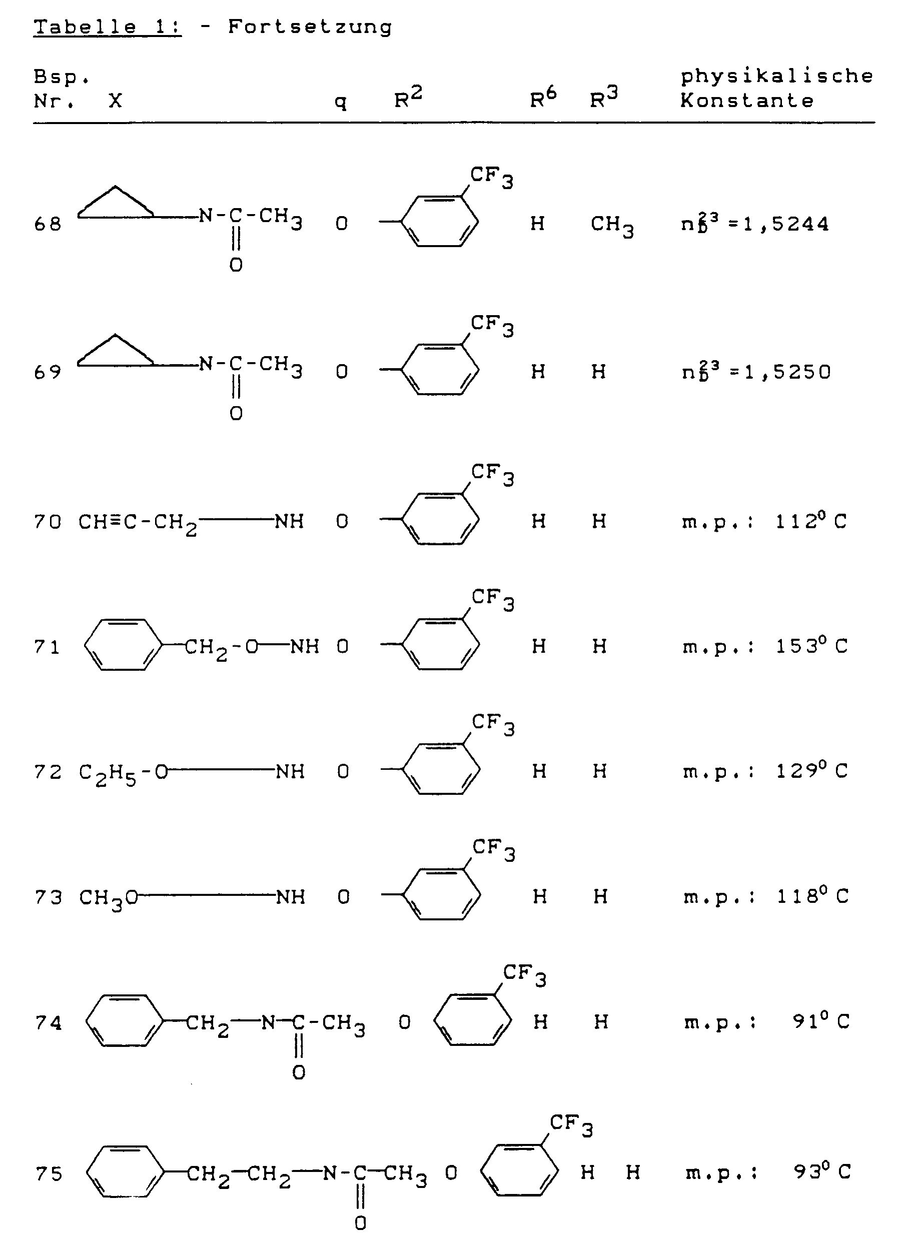

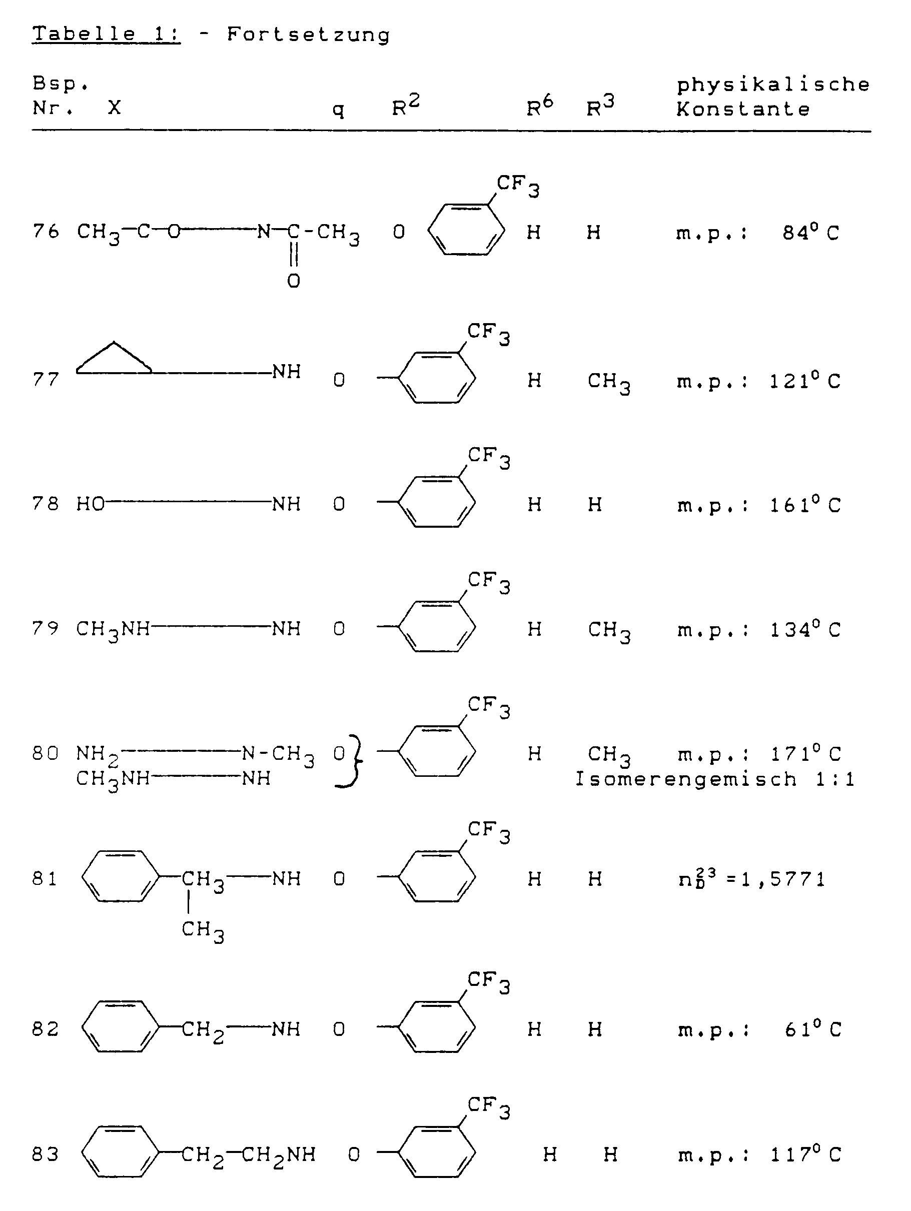

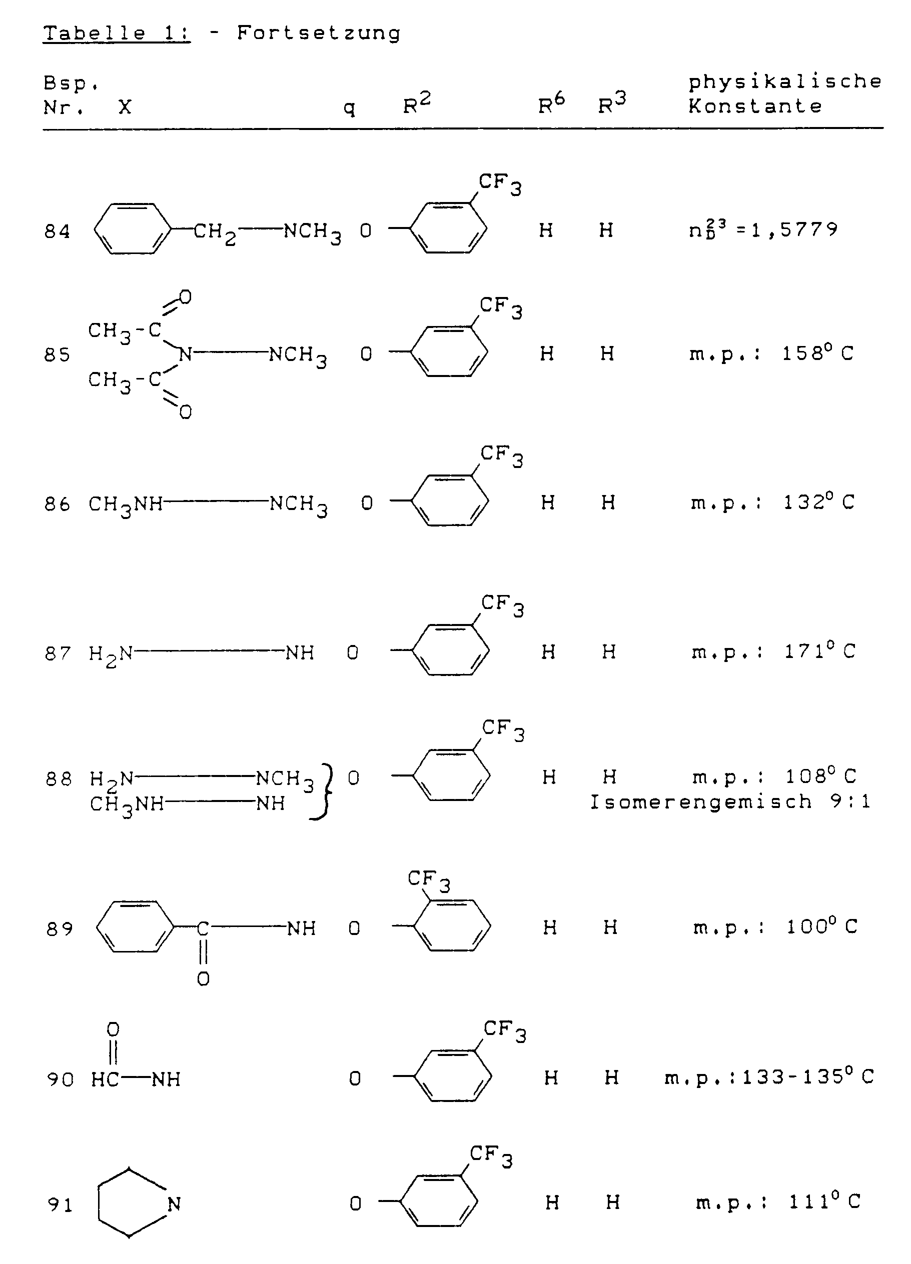

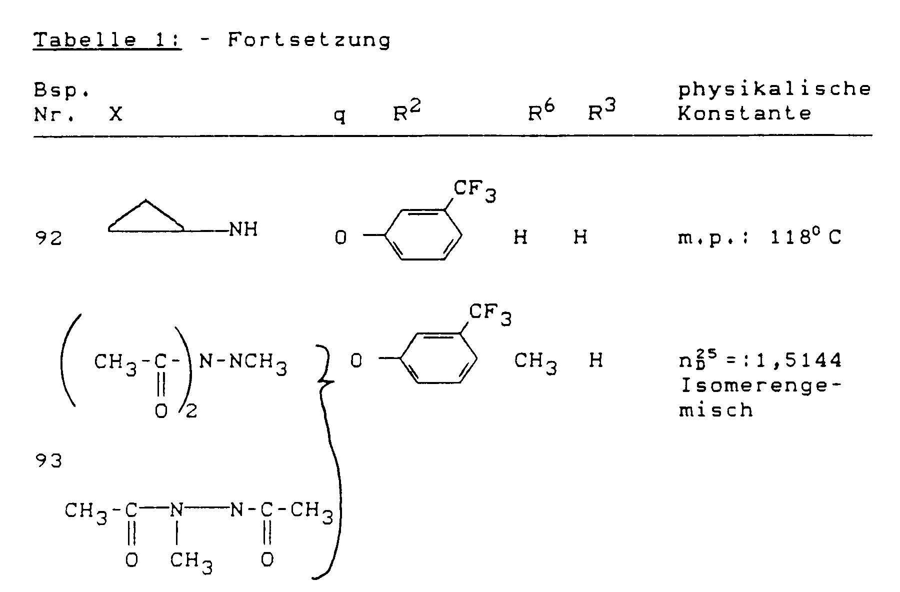

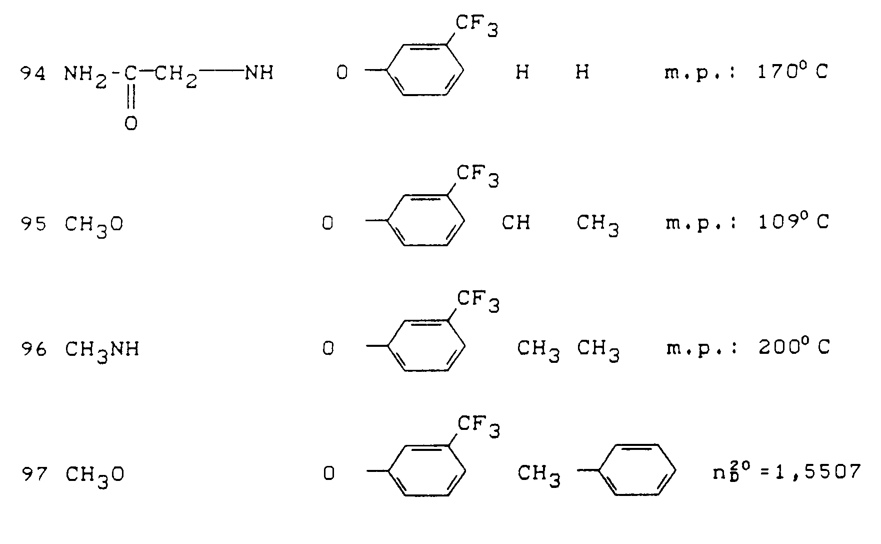

### Herstellung der Ausgangsverbindungen

### Beispiel (IV-1)

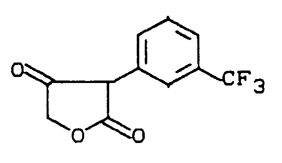

1,9 g (0,017 Mol) Kalium-tert-butylat werden in 20 ml Butanol gelöst, 5,0 g (0,017 Mol) O-(3-Trifluorphenylacetyl)-hydroxyessigsäure-ethylester zugetropft und 16 Stunden bei 80°C gerührt. Nach Abkühlen wird mit 5 ml konzentrierter Salzsäure angesäuert, 3 mal mit je 50 ml Methylenchlorid extrahiert und die Methylenchloridphase über Natriumsulfat getrocknet. Nach Abdestillieren des Lösungsmittels im Wasserstrahlvakuum wird das zurückbleibende Oel mit 10 ml Diisopropylether aufgenommen. Dabei kristallisieren 1,9 g (58,5 % der Theorie) farblose Kristalle von 3-(3-Trifluormethylphenyl)-tetronsäure vom Schmelzpunkt 184°C.

### Beispiel (IV-2)

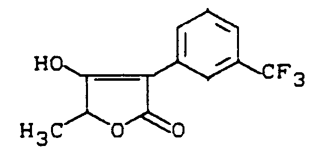

In eine im Eisbad auf 0 - 5°C gekühlte Suspension von 1,3 g (43,3 mMol) 80 %-igem Natriumhydrid in Paraffinöl in 20 ml wasserfreiem Dimethylformamid tropft man unter Rühren eine Lösung von 13,3 g (42,2 mMol) DL-O-(3-Trifluormethylphenylacetyl)-milchsäureethylester in 20 ml wasserfreiem Dimethylformamid innerhalb von 10 Minuten zu. Anschließend läßt man auf Raumtemperatur erwärmen und rührt 16 Stunden bei dieser Temperatur. Danach fügt man 10 ml Wasser zur Reaktionslösung, säuert mit konzentrierter Salzsäure auf pH 1 an und schüttelt dreimal mit je 50 ml Dichlormethan aus. Die vereinigten organischen Phasen werden mit gesättigter Natriumchloridlösung ausgeschüttelt, über Natriumsulfat getrocknet und das Lösungsmittel im Vakuum abgedampft. Das verbliebene Dimethylformamid destilliert man im Ölpumpenvakuum ab. Der erhaltene Rückstand wird mit Petrolether verrieben.

Man erhält 6,8 g (63 % der Theorie) DL-4-Hydroxy-5-methyl-3-(3-trifluormethylphenyl)-5H-furan-2-on als hellgelben Feststoff vom Schmelzpunkt 175°C.

### Beispiel (IV-3)

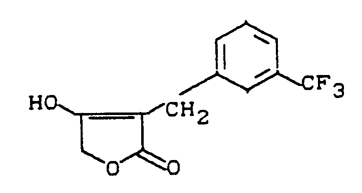

32,0 g (0,125 Mol) 3-(3-Trifluormethylbenzyliden)-2,4-furan-dion werden in 450 ml Essigester in Gegenwart von 4,0 g Palladium/Aktivkohle (4 %ig) bei Raumtemperatur unter 9 bar Wasserstoffatmosphäre hydriert. Nach 90 Minuten wird die Lösung filtriert und das Lösungsmittel abdestilliert Das so erhaltene Rohprodukt wird aus Diethylether umkristallisiert.
Man erhält 16,0 g (50 % der Theorie) 3-Trifluormethylbenzyl-tetronsäure vom Schmelzpunkt 128-129°C.

In analoger Weise zu den in den Beispielen (IV-1) bis (IV-3) beschriebenen Methoden und unter Berücksichtigung der Angaben in der Beschreibung zu der erfindungsgemäßen Verfahrensvariante b), wurden die in der nachfolgenden Tabelle 2 aufgeführten Ausgangsverbindungen der Formel (IV) erhalten.

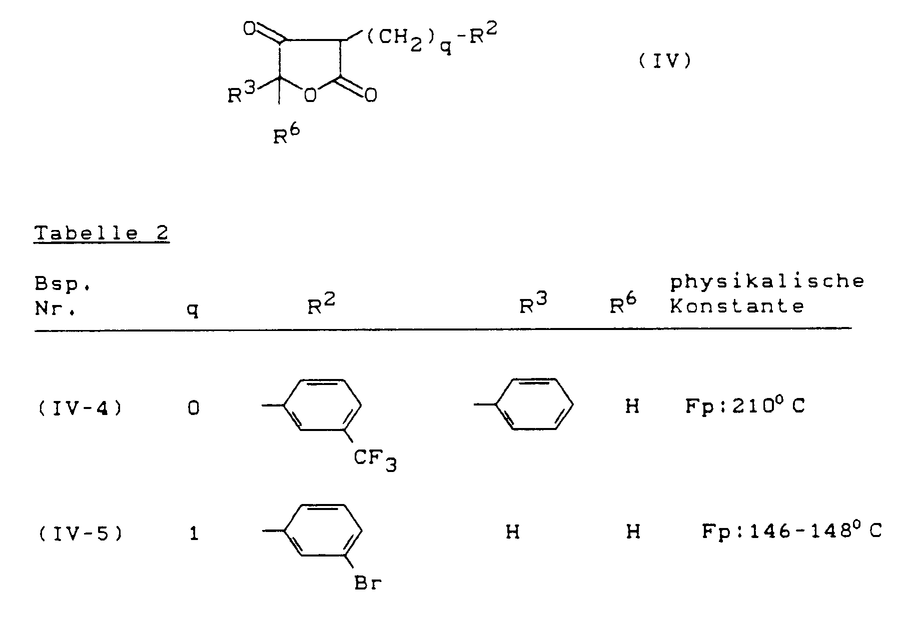

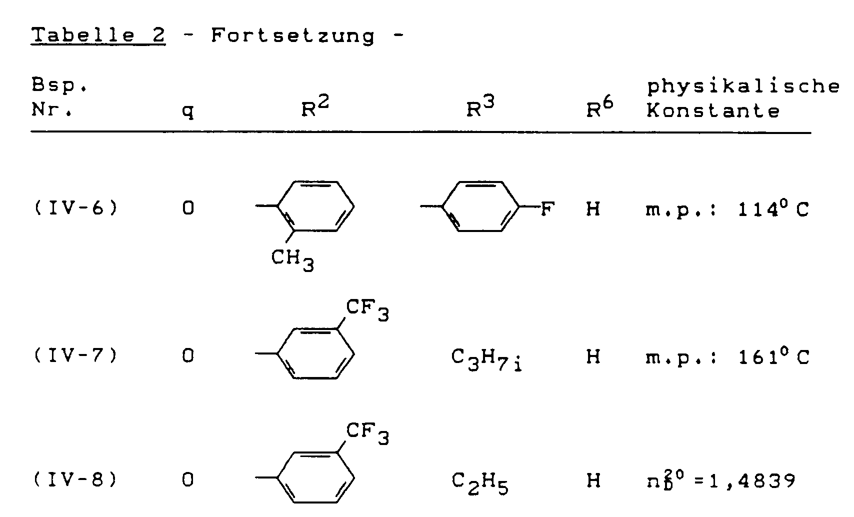

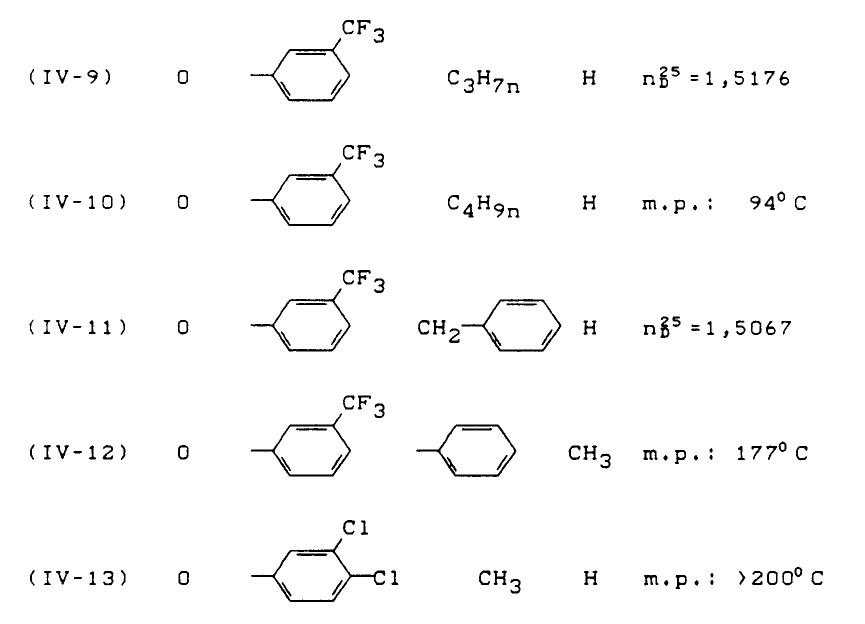

### Herstellung der Vorprodukte

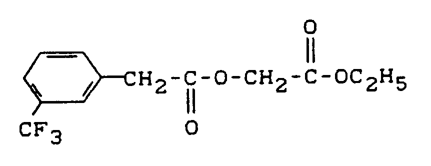

2,4 g (0,105 Mol) Natrium werden bei 20°C bis 50°C zu 100 ml Ethanol portionsweise zugegeben. Man gibt 21 g (0,105 Mol) 3-Trifluorphenylessigsäure portionsweise zu, rührt 30 Minuten bei 20°C nach und tropft 11,7 ml (0,105 Mol) Bromessigsäureethylester zu. Dabei steigt die Temperatur auf 28°C. Danach wird auf 80°C erwärmt und 2 Stunden nachgerührt. Man destilliert das Lösungsmittel im Wasserstrahlvakuum ab, nimmt den Rückstand in 300 ml Methylenchlorid auf und wäscht die organische Phase zweimal mit je 150 ml Wasser, trocknet über Natriumsulfat und destilliert nach dem Filtrieren im Wasserstrahlvakuum das Lösungsmittel ab. Nach Chromatographie über Kieselgel (Laufmittel: Methylenchlorid) erhält man 28,3 g (93 % der Theorie) O-(3-Trifluormethylphenylacetyl)-hydroxyessigsäureethylester als Öl vom Brechungsindex n$\frac{\text{23}}{\text{D}}$ = 1,4535. 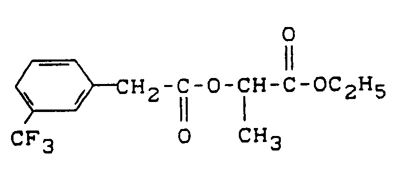
10,0 g (44,9 mMol) 3-Trifluormethylphenylacetylchlorid und 5,3 g (44,9 mMol) DL-Milchsäureethylester werden langsam auf 100°C erwärmt und bis zum Ende der HCl-Entwicklung bei dieser Temperatur gerührt. Nach 2 Stunden ist die Reaktion beendet. Man läßt abkühlen und filtriert die Reaktionslösung über eine Kieselgelsäule (Laufmittel: Methylenchlorid).

Man erhält 13,3 g (97 % der Theorie) DL-O-(3-Trifluormethylphenylacetyl)-milchsäureethylester als hellgelbe Flüssigkeit vom Brechungsindex n$\frac{\text{20}}{\text{D}}$ = 1,4524. 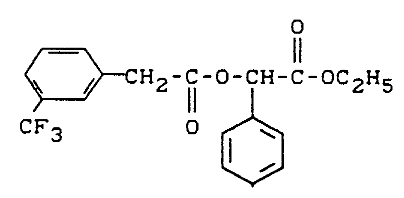
Auf analoge Weise erhält man DL-O-(3-Trifluormethylphenylacetyl)-mandelsäureethylester als gelbes Öl vom Brechungsindex n$\frac{\text{20}}{\text{D}}$ = 1,4978. 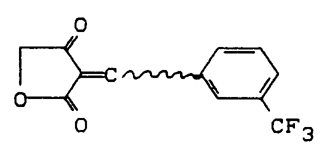
Zu einer Lösung von 104,5 g (0,6 Mol) 3-Trifluormethylbenzaldehyd und 20,0 g (0,2 Mol) Tetronsäure in 60 ml Methylenchlorid werden innerhalb von 30 Minuten unter Rühren 19,4 ml konzentrierte Salzsäure getropft. Nach 24 Stunden wird das Rohprodukt in 150 ml gesättigte Natriumhydrogencarbonat-Lösung eingerührt, die organische Phase abgetrennt und die wässrige Phase zweimal mit je 70 ml Methylenchlorid extrahiert. Die vereinigten organischen Phasen werden über Natriumsulfat getrocknet, das Lösungsmittel abdestilliert und der verbleibende Rückstand aus Diisopropylether umkristallisiert. Man erhält 38,5 g (75 % der Theorie) an 3-(3-Trifluormethylbenzyliden)-2,4-furandion vom Schmelzpunkt 108-109°C. 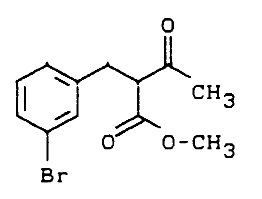
Zu einer Lösung von 13,5 g (0,25 Mol) Natriummethylat in 100 ml Methanol werden 58 g (0,5 Mol) Acetessigester bei 30-35°C schnell zugetropft. Nach kurzem Nachrühren (20 Minuten) tropft man bei 30°C 50 g (0,25 Mol) 3-Brombenzylbromid zu. Nach Ende der exothermen Reaktion erhitzt man weitere 4 Stunden zum Sieden und destilliert anschließend das Lösungsmittel ab. Der Rückstand wird mit 200 ml Wasser versetzt und dreimal mit 75 ml Ether extrahiert. Die vereinigten organischen Phasen werden über Natriumsulfat getrocknet und vom Lösungsmittel befreit. Durch Fraktionierung des Rückstandes erhält man 56,0 g (0,20 Mol, 80 % der Theorie) 2-(3-Brombenzyl)-acetessigsäuremethylester vom Siedepunkt 105°C/0,1 Torr).

### Anwendungsbeispiele:

In dem folgenden Anwendungsbeispiel wurde die nachstehend aufgeführte Verbindung als Vergleichssubstanz eingesetzt: 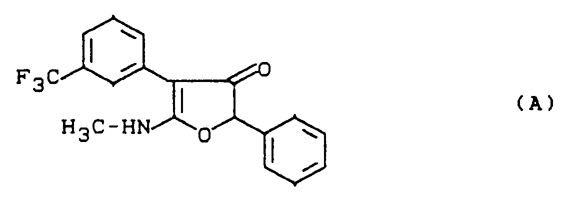
(±)-5-Methylamino-2-phenyl-4-[3-(trifluormethyl)-phenyl]-2H-furan-3-on
(bekannt aus DE-OS 34 22 346, Beispiel 7, Seite 53).

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
O % = keine Wirkung (wie unbehandelte Kontrolle)
100 % = totale Vernichtung
In diesem Test wird z.B. die Verbindung (15) von Soja und Baumwolle deutlich besser toleriert und zeigt eine bessere herbizide Wirkung als die Vergleichsverbindung (A).

Darüber hinaus wird z.B. die Verbindung (41) von Weizen besser toleriert und zeigt eine deutlich bessere herbizide Wirkung als die Vergleichsverbindung (A).

## Patentansprüche

1. 5H-Furan-2-on-Derivate der Formel (I) 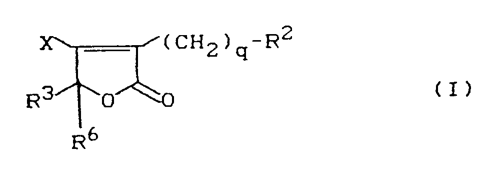 in welcher
X für den Rest OR¹ oder den Rest 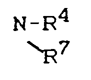 steht,
wobei
R⁴ für Wasserstoff, Alkyl, Alkenyl, Alkoxyalkyl oder Alkylcarbonyl steht, und
R⁷ für Wasserstoff, Hydroxy, Amino, Formyl, Alkyl, Alkoxyalkyl, Cyanoalkyl, Alkylcarbonyl, Halogenalkylcarbonyl, Alkoxycarbonylalkyl, Alkoxy, Alkylamino, Alkylcarbonyloxy, Aminocarbonylalkyl, Alkinyl, Cycloalkyl, jeweils unsubstituiertes oder substituiertes Arylcarbonyl, Aralkyl, Aralkyloxy oder für die Gruppe 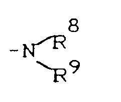 steht
wobei R⁸ und R⁹ jeweils unabhängig voneinander für Alkyl oder Alkylcarbonyl stehen
oder
R⁴ und R⁷ gemeinsam mit dem Stickstoffatom, an welches sie gebunden sind, für einen gesättigten Heterocyclus stehen,
q für die Zahlen 0 oder 1 steht,
R¹ für Alkyl, Alkoxyalkyl, Cyanoalkyl, Alkylcarbonyl, Halogenalkylcarbonyl oder Alkoxycarbonylalkyl steht,
R² für unsubstituiertes oder einfach oder mehrfach, gleich oder verschieden substituiertes Aryl steht, wobei als Substituenten ausgewählt sind:
Cyano, Nitro, Halogen, Alkyl, Alkoxy, Halogenalkyl, Halogenalkoxy, jeweils unsubstituiertes oder einfach oder mehrfach, gleich oder verschieden substituiertes Phenethenyl oder Phenethinyl steht, wobei als Phenylsubstituenten Cyano, Nitro, Halogen, Alkyl, Alkoxy, Halogenalkyl oder Halogenalkoxy genannt seien, als weiterer Phenylsubstituent sei außerdem der Rest -(CH₂)ₙ-Zₘ-(CH₂)ₚ-R⁵ genannt, wobei
R⁵ für unsubstituiertes oder einfach oder mehrfach, gleich oder verschieden substituiertes Aryl steht, wobei als Substituenten genannt seien: Cyano, Nitro, Halogen, Alkyl, Alkoxy, Halogenalkyl oder Halogenalkoxy,
Z für Sauerstoff, Schwefel oder die Gruppe -CO-steht,
n, m und p unabhängig voneinander für die Zahlen 0 oder 1 stehen, und
R³ und R⁶ unabhängig voneinander für Wasserstoff, Alkyl, jeweils unsubstituiertes oder einfach oder mehrfach, gleich oder verschieden substituiertes Aryl oder Aralkyl stehen, wobei als Substituenten im Arylteil ausgewählt sind: Cyano, Nitro, Halogen, Alkyl, Alkoxy, Halogenalkyl oder Halogenalkoxy,
mit der Maßgabe, daß, wenn X für den Rest OR¹ und gleichzeitig q für 0 steht, R² nur dann für orthosubstituiertes Phenyl steht, wenn die Substituenten Halogen, Halogenmethyl mit 1 bis 3 gleichen oder verschiedenen Halogenatomen oder jeweils unsubstituiertes oder einfach bis mehrfach gleich oder verschieden substituiertes Phenethenyl oder Phenethinyl sind, mit der Maßgabe, daß R² für substituiertes Phenyl steht, wenn gleichzeitig q für 1 und X für den Rest OR¹ steht und ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on und 4-Methoxy-3-(4-methoxyphenyl)-5H-furan-2-on, 3-(2-Chlorphenyl)-4-methoxy-5H-furan-2-on, 3-(2-Fluorphenyl)-4-methoxy-5H-furan-2-on, 4-Methoxy-3-(2-methoxyphenyl)-5H-furan-2-on, 5,5-Dimethyl-4-amino-3-phenyl-5H-furan-2-on, 4-Methoxy-3-phenyl-5H-furan-2-on, 5-Benzyl-4-methoxy-3-phenyl-5H-furan-2-on, 5,5-Diethyl-4-methoxy-3-phenyl-5H-furan-2-on, 5-(α-Methoxycarbonyl-α-hydroxy-benzyl)-4-methoxy-3-(4-methoxyphenyl)-5-H-furan-2-on, 5-(α-Methoxycarbonyl-α-hydroxy-4-methoxybenzyl)-4-methoxy-3-(3,4,5-trimethoxyphenyl)-5-H-furan-2-on, 5-(α-Hydroxy-4-methoxybenzyl)-4-methoxy-3-(4-methoxyphenyl)-5H-furan-2-on.

2. 5H-Furan-2-on-Derivate der Formel (I) gemäß Anspruch 1, in welcher
X für den Rest OR¹ oder den Rest 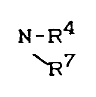 steht,
wobei
R⁴ für Wasserstoff, jeweils geradkettiges oder verzweigtes Alkyl mit 1 bis 4 Kohlenstoffatomen, Alkenyl mit 2 bis 4 Kohlenstoffatomen oder Alkoxyalkyl mit jeweils 1 bis 4 Kohlenstoffatomen im Alkoxy- und Alkylteil oder Alkylcarbonyl mit 1 bis 4 Kohlenstoffatomen steht, und
R⁷ für Wasserstoff, Hydroxy, Amino, Formyl, für jeweils geradkettiges oder verzweigtes Alkyl, Alkoxy, Alkoxyalkyl, Cyanoalkyl, Alkylcarbonyl, Halogenalkylcarbonyl, Alkoxycarbonylalkyl, Alkylamino, Alkylcarbonyloxy oder Aminocarbonylalkyl mit 1 bis 6 Kohlenstoffatomen in den einzelnen Alkylteilen und gegebenenfalls 1 bis 9 gleichen oder verschiedenen Halogenatomen, für Alkinyl mit 2 bis 6 Kohlenstoffatomen, Cycloalkyl mit 3 bis 7 Kohlenstoffatomen, für jeweils unsubstituiertes oder einfach bis mehrfach, gleich oder verschieden substituiertes Arylcarbonyl, Aralkyl, Aralkyloxy mit 3 bis 10 Kohlenstoffatomen und 1 bis 4 Kohlenstoffatomen im geradkettigen oder verzweigten Alkylteil, wobei als Substituenten die bei R⁵ genannten Substituenten infrage kommen oder R⁷ für eine Gruppe 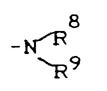 steht,
wobei R⁸ und R⁹ jeweils unabhängig voneinander für Alkyl oder Alkylcarbonyl mit jeweils 1 bis 4 Kohlenstoffatomen stehen, oder
R⁴ und R⁷ gemeinsam mit dem Stickstoffatom, an welches sie gebunden sind, für eine gesättigte Heteroalkylenkette mit 4 bis 5 Kohlenstoffatomen stehen,
q für die Zahlen 0 oder 1 steht,
R¹ für geradkettiges oder verzweigtes Alkyl mit 1 bis 4 Kohlenstoffatomen oder für jeweils geradkettiges oder verzweigtes Cyanoalkyl, Alkoxyalkyl, Alkylcarbonyl, Halogenalkylcarbonyl oder Alkoxycarbonylalkyl mit 1 bis 6 Kohlenstoffatomen in den einzelnen Alkylteilen und gegebenenfalls 1 bis 9 gleichen oder verschiedenen Halogenatomen steht,
R² für unsubstituiertes oder einfach oder mehrfach, gleich oder verschieden substituiertes Phenyl steht, wobei als Substituenten genannt seien: Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen, jeweils unsubstituiertes oder einfach oder mehrfach, gleich oder verschieden substituiertes Phenethenyl, Phenethinyl, wobei jeweils als Substituenten die schon oben genannten Phenylsubstituenten infrage kommen; als weiterer Phenylsubstituent sei außerdem der Rest -(CH₂)ₙ-Zₘ-(CH₂)ₚ-R⁵ genannt,
worin
R⁵ für unsubstituiertes oder einfach oder mehrfach, gleich oder verschieden substituiertes Aryl mit 6 bis 10 Kohlenstoffatomen, insbesondere Phenyl oder Naphthyl steht, wobei als Substituenten Cyano, Nitro, Halogen, C₁₋₄-Alkyl, C₁₋₄-Alkoxy, Halogen-C₁₋₄-alkyl oder Halogen-C₁₋ ₄-alkoxy infrage kommen,
Z für Sauerstoff, Schwefel oder für die Gruppe >C=O steht und
n, m und p unabhängig voneinander für die Zahlen 0 oder 1 stehen, und
R³ und R⁶ für Wasserstoff, geradkettiges oder verzweigtes Alkyl mit 1 bis 6 Kohlenstoffatomen, unsubstituiertes oder einfach bis mehrfach, gleich oder verschieden substituiertes Phenyl oder unsubstituiertes oder einfach bis mehrfach, gleich oder verschieden substituiertes Aralkyl mit 6 bis 10 Kohlenstoffatomen im Arylteil und 1 bis 4 Kohlenstoffatomen im Alkylteil stehen, wobei als Substituenten Cyano, Nitro, Halogen, C₁₋₄-Alkyl, C₁₋₄-Alkoxy, Halogen-C₁₋₄-alkyl und Halogen-C₁₋₄-alkoxy infrage kommen,
mit der Maßgabe, daß, wenn X für den Rest OR¹ und gleichzeitig q für O steht, R² nur dann für orthosubstituiertes Phenyl steht, wenn die Substituenten Halogen, Halogenmethyl mit 1 bis 3 gleichen oder verschiedenen Halogenatomen oder jeweils unsubstituiertes oder einfach bis mehrfach gleich oder verschieden substituiertes Phenethenyl oder Phenethinyl sind, mit der Maßgabe, daß R² für substituiertes Phenyl steht, wenn gleichzeitig q für 1 und X für den Rest OR¹ steht und
ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on und 4-Methoxy-3-(4-methoxyphenyl)-5H-furan-2-on, 3-(2-Chlorphenyl)-4-methoxy-5H-furan-2-on, 3-(2-Fluorphenyl)-4-methoxy-5H-furan-2-on und 4-Methoxy-3-(2-methoxyphenyl)-5H-furan-2-on, 5,5-Dimethyl-4-amino-3-phenyl-5H-furan-2-on, 4-Methoxy-3-phenyl-5H-furan-2-on, 5-Benzyl-4-methoxy-3-phenyl-5H-furan-2-on, 5,5-Diethyl-4-methoxy-3-phenyl-5H-furan-2-on, 5-(α-Methoxycarbonyl-α-hydroxy-benzyl)-4-methoxy-3-(4-methoxyphenyl)-5-H-furan-2-on, 5-(α-Methoxycarbonyl-α-hydroxy-4-methoxybenzyl)-4-methoxy-3-(3,4,5-trimethoxyphenyl)-5-H-furan-2-on, 5-(α-Hydroxy-4-methoxybenzyl)-4-methoxy-3-(4-methoxyphenyl)-5H-furan-2-on.

3. 5H-Furan-2-on-Derivate der Formel (I) gemäß Anspruch 1, in welcher
X für den Rest 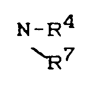 steht,
worin
R⁴ für Wasserstoff, Methyl, Ethyl, n- oder i-Propyl, n-, i-, s- oder t-Butyl, Allyl, Propenyl-(2), 1-Methallyl, Methoxymethyl, Ethoxymethyl, Methoxyethyl, Ethoxyethyl Methylcarbonyl, Ethylcarbonyl, n-Propylcarbonyl, oder i-Propylcarbonyl steht,
R⁷ für Wasserstoff, Hydroxy, Amino, Formyl, Methyl, Ethyl, n- oder i-Propyl, n-, i-, s- oder t-Butyl, Methoxy, Ethoxy, n- oder i-Propoxy, n-, i-, s- oder t-Butoxy, Alkylcarbonyl mit 1 bis 6 Kohlenstoffatomen, jeweils geradkettiges oder verzweigtes Alkoxyalkyl, Cyanoalkyl, Halogenalkylcarbonyl, Alkoxycarbonylalkyl, Alkylamino, Alkylcarbonyloxy oder Aminocarbonylalkyl mit 1 bis 4 Kohlenstoffatomen in den einzelnen Alkylteilen und gegebenenfalls 1 bis 9, vorzugsweise 1 bis 5, gleichen oder verschiedenen Halogenatomen, für Alkinyl mit 2 bis 4 Kohlenstoffatomen, Cycloalkyl mit 3 bis 6 Kohlenstoffatomen, für jeweils unsubstituiertes oder einfach bis dreifach, gleich oder verschieden substituiertes Phenylcarbonyl, Phenylalkyl, Phenylalkyloxy und 1 bis 4 Kohlenstoffstomen im im geradkettigen oder verzweigten Alkylteil, wobei als Substituenten die bei R⁵ genannten Substituenten infrage kommen oder für eine Gruppe 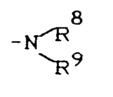 steht,
wobei R⁸ und R⁹ jeweils unabhängig voneinander für Methyl, Ethyl, n- oder i-Propyl, Methylcarbonyl, Ethylcarbonyl oder n- oder i-Propylcarbonyl stehen
oder
R⁴ und R⁷ gemeinsam mit dem Stickstoffatom, an welches sie gebunden sind, für eine gesättigte Heteroalkylenkette mit 4 oder 5 Kohlenstoffatomen stehen,
q für die Zahlen 0 oder 1 steht,
R² für unsubstituiertes oder einfach bis dreifach, gleich oder verschieden substituiertes Phenyl steht, wobei als Substituenten genannt seien; Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenethenyl, Phenethinyl, wobei jeweils als Substituenten die schon oben genannten Phenylsubstituenten infrage kommen;
als weiterer Phenylsubstituent sei außerdem der Rest -(CH₂)ₙ-Zₘ-(CH₂)ₚ-R⁵ genannt, worin
R⁵ für unsubstituiertes oder einfach bis dreifach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, infrage kommen,
Z für Sauerstoff, Schwefel oder für die Gruppe >C=O steht und
n, m und p unabhängig voneinander für die Zahlen 0 oder 1 stehen,
R³ und R⁶ für Wasserstoff, Methyl, Ethyl n- oder i-Propyl, n-, i-, s- oder t-Butyl, unsubstituiertes oder einfach bis dreifach, gleich oder verschieden substituiertes Phenyl oder jeweils unsubstituiertes oder einfach bis dreifach, gleich oder verschieden substituiertes Benzyl oder Phenethyl stehen, wobei als Substituenten Cyano, Nitro, Fluor, Chlor, Brom, Methyl, Ethyl, n- oder i-Propyl, Methoxy, Ethoxy, n-oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, infrage kommen, ausgenommen die Verbindung 5,5-Dimethyl-4-amino-3-phenyl-5H-furan-2-on.

4. 5H-Furan-2-on-Derivate der Formel (I) gemäß Anspruch 1, in welcher
X für den Rest OR¹ steht, wobei
R¹ für geradkettiges oder verzweigtes Alkyl mit 1 bis 4 Kohlenstoffatomen oder für jeweils geradkettiges oder verzweigtes Cyanoalkyl, Alkoxyalkyl, Alkylcarbonyl, Halogenalkylcarbonyl oder Alkoxycarbonylalkyl mit 1 bis 6 Kohlenstoffatomen in den einzelnen Alkylteilen und gegebenenfalls 1 bis 9 gleichen oder verschiedenen Halogenatomen steht,
R³ und R⁶ für Wasserstoff, geradkettiges oder verzweigtes Alkyl mit 1 bis 6 Kohlenstoffatomen, unsubstituiertes oder einfach bis mehrfach, gleich oder verschieden substituiertes Phenyl oder unsubstituiertes oder einfach oder mehrfach, gleich oder verschieden substituiertes Aralkyl mit 6 bis 10 Kohlenstoffatomen im Arylteil und 1 bis 4 Kohlenstoffatomen im Alkylteil steht, wobei als Substituenten Cyano, Nitro, Halogen, C₁₋₄-Alkyl, C₁₋₄-Alkoxy, Halogen-C₁₋₄-alkyl und Halogen-C₁₋₄-alkoxy infrage kommen, und
entweder
α) R²⁻¹ für in meta- oder para-Position, gleich oder verschieden, mono-, di- oder trisubstituiertes Phenyl steht, wobei als Substituenten genannt seien:
Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen;
als weiterer Phenylsubstituent sei außerdem der Rest -(CH₂)ₙ-Zₘ-(CH₂)ₚ-R⁵ genannt,
worin
R⁵ für unsubstituiertes oder einfach bis mehrfach, gleich oder verschieden substituiertes Aryl mit 6 bis 10 Kohlenstoffatomen steht, wobei als Substituenten Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen,
Z für Sauerstoff, Schwefel oder für die Gruppe >C=O steht und
n, m und p für die Zahlen 0 oder 1 stehen und
q für die Zahlen 0 oder 1 steht,
ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on, 4-Methoxy-3-(4-methoxyphenyl)-5H-furan-2-on, 5-(α-Methoxycarbonyl-α-hydroxy-4-methoxybenzyl)-4-methoxy-3-(3,4,5-trimethoxyphenyl)-5H-furan-2-on, 5-(α-Methoxycarbonyl-α-hydroxy-benzyl)-4-methoxy-3-(4-methoxyphenyl)-5H-furan-2-on und 5-(α-Hydroxy-4-methoxybenzyl)-4-methoxy-3-(4-methoxyphenyl)-5H-furan-2-on, oder
β) R²⁻² für einfach in ortho-Position substituiertes Phenyl steht,
wobei als Substituenten genannt seien:
Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6, Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, infrage kommen;
als weiterer Phenylsubstituent sei außerdem der Rest
-(CH₂)ₙ-Zₘ-(CH₂)ₚ-R⁵ genannt,
R⁵ für unsubstituiertes oder einfach bis mehrfach, gleich oder verschieden substituiertes Aryl mit 6 bis 10 Kohlenstoffatomen steht, wobei als Substituenten Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschieden Halogenatomen, infrage kommen,
Z für Sauerstoff, Schwefel oder für die Gruppe >C=O steht und
n, m und p für die Zahlen 0 oder 1 stehen und
q für die Zahl 1 steht, oder
γ) R²⁻³ für einfach in ortho-Position substituiertes Phenyl steht,
wobei als Substituenten genannt seien:
Halogen, Halogenmethyl mit 1 bis 3 gleichen oder verschiedenen Halogenatomen, jeweils unsubstituiertes oder einfach bis mehrfach, gleich oder verschieden substituiertes Phenethenyl oder Phenethinyl, wobei jeweils als Substituenten Cyano, Nitro, Halogen, jeweils geradkettiges oder verzweigtes Alkyl oder Alkoxy mit 1 bis 6 Kohlenstoffatomen, jeweils geradkettiges oder verzweigtes Halogenalkyl oder Halogenalkoxy mit 1 bis 4 Kohlenstoffatomen und 1 bis 9 gleichen oder verschiedenen Halogenatomen infrage kommen und
q für die Zahl 0 steht,
ausgenommen die Verbindungen 3-(2-Chlorphenyl)-4-methoxy-5H-furan-2-on, 3-(2-Fluor-phenyl)-4-methoxy-5H-furan-2-on, 4-Methoxy-3-(2-methoxyphenyl)-5H-furan-2-on.

5. 5H-Furan-2-on-Derivate der Formel (I) gemäß Anspruch 1, in welcher
X für den Rest OR¹ steht,
R¹ für Methyl, Ethyl, n- oder i-Propyl, n-, i-, s- oder t-Butyl oder für jeweils geradkettiges oder verzweigtes Cyanoalkyl, Alkoxyalkyl oder Alkoxycarbonylalkyl mit 1 bis 4 Kohlenstoffatomen in den einzelnen Alkylteilen steht,
R³ und R⁶ für Wasserstoff, Methyl, Ethyl n- oder i-Propyl, n-, i-, s- oder t-Butyl, unsubstituiertes oder einfach bis dreifach, gleich oder verschieden substituiertes Phenyl oder unsubstituiertes oder einfach bis dreifach, gleich oder verschieden substituiertes Benzyl oder Phenethyl stehen, wobei als Substituenten jeweils Cyano, Nitro, Fluor, Chlor, Brom, Methyl, Ethyl, n- oder i-Propyl, Methoxy, Ethoxy, n- oder i-Propoxy, jeweils geradkettiges Halogenalkyl oder Halogenalkoxy mit 1 oder 2 Kohlenstoffatomen und 1 bis 5 gleichen oder verschiedenen Fluor- oder Chloratomen, infrage kommen;
entweder
α) R²⁻¹ für in meta- oder para-Position, gleich oder verschieden, einfach, di- oder trisubstituiertes Phenyl steht, wobei als Substituenten genannt seien: Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenylethenyl oder Phenethinyl, wobei jeweils an Substituenten Cyano, Nitro, Fluor, Chlor, Brom, Methyl, Ethyl, n-oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen;
als weiterer Phenylsubstituent sei außerdem der Rest -(CH₂)ₙ-Zₘ-(CH₂)ₚ-R⁵ genannt,
worin
R⁵ für unsubstituiertes oder einfach bis dreifach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen,
Z für Sauerstoff, Schwefel oder für die Gruppe >C=O steht und
n, m und p für die Zahlen 0 oder 1 stehen und
q für die Zahlen 0 oder 1 steht,
ausgenommen die Verbindungen 5-[(3,4-Dimethoxyphenyl-methyl]-4-methoxy-3-(4-methoxyphenyl)-5H-furan-2-on, 4-Methoxy-3-(3,4,5-trimethoxyphenyl)-5H-furan-2-on, 3-(3-Chlorphenyl)-4-methoxy-5H-furan-2-on, 3-(4-Chlorphenyl)-4-methoxy-5H-furan-2-on, 3-(3-Fluorphenyl)-4-methoxy-5H-furan-2-on, 3-(4-Fluorphenyl)-4-methoxy-5H-furan-2-on, 4-Methoxy-3-(3-methoxyphenyl)-5H-furan-2-on, 3-(4-Bromphenyl)-4-methoxy-5H-furan-2-on, 3-(3,4-Dichlorphenyl)-4-methoxy-5H-furan-2-on, 3-[1,1'-Biphenyl]-4-yl-4-methoxy-5H-furan-2-on, 4-Methoxy-3-(4-methylphenyl)-5H-furan-2-on, 4-Methoxy-3-(4-methoxyphenyl)-5H-furan-2-on, 5-(α-Methoxycarhonyl-α-hydroxy-4-methoxybenzyl)-4-methoxy-3-(3,4,5-trimethoxyphenyl)-5H-furan-2-on, 5-(α-Methoxycarbonyl-α-hydroxy-benzyl)-4-methoxy-3-(4-methoxyphenyl)-5H-furan-2-on und 5-(α-Hydroxy-4-methoxybenzyl)-4-methoxy-3-(4-methoxyphenyl)-5H-furan-2-on, oder
β) R²⁻² für einfach in ortho-Position substituiertes Phenyl steht, wobei als Substituenten genannt seien:
Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen, jeweils unsubstituiertes oder einfach bis dreifach, gleich oder verschieden substituiertes Phenethenyl oder Phenethinyl, wobei jeweils als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen,
als weiterer Phenylsubstituent sei außerdem der Rest
-(CH₂)ₙ-Zₘ-(CH₂)ₚ-R⁵ genannt,
worin
R⁵ für unsubstituiertes oder einfach bis mehrfach, gleich oder verschieden substituiertes Phenyl steht, wobei als Substituenten Cyano, Nitro, Fluor, Chlor, Brom, Methyl, Ethyl, n- oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen;
Z für Sauerstoff, Schwefel oder für die Gruppe >C=O steht und
n, m und p für die Zahlen 0 oder 1 stehen und
q für die Zahl 1 steht, oder
γ) R²⁻³ für einfach in ortho-Position substituiertes Phenyl steht,
wobei als Substituenten genannt seien:
Fluor, Chlor, Brom, Halogenmethyl mit 1 bis 3 gleichen oder verschiedenen Fluor- oder Chloratomen, jeweils unsubstituiertes oder einfach bis dreifach, gleich oder verschieden substituiertes Phenethenyl oder Phenethinyl, wobei jeweils als Substituenten Cyano, Nitro, Fluor, Chlor, Brom, Methyl, Ethyl, n-oder i-Propyl, Methoxy, Ethoxy, n- oder i-Propoxy, jeweils geradkettiges oder verzweigtes Halogenalkyl oder Halogenalkoxy mit 1 oder 2 Kohlenstoffatomen und 1 bis 5 gleichen oder verschiedenen Fluor- oder Chloratomen infrage kommen und
q für die Zahl 0 steht,
ausgenommen die Verbindungen 3-(2-Chlorphenyl)-4-methoxy-5H-furan-2-on, 3-(2-Fluorphenyl)-4-methoxy-5H-furan-2-on, 4-Methoxy-3-(2-methoxyphenyl)-5H-furan-2-on.

6. 5H-Furan-2-on-Derivate der Formel (I) gemäß Anspruch 1, in welcher
X für den Rest 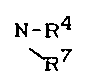 steht,
worin
R⁴ für Wasserstoff, Methyl, Ethyl, n- oder i-Propyl, Methylcarbonyl oder Ethylcarbonyl steht, und
R⁷ für Wasserstoff, Hydroxy, Amino, Formyl, Methyl, Ethyl, n- oder i-Propyl, n-, i-, s- oder t-Butyl, Methoxy, Ethoxy, n- oder i-Propoxy, n-, i-, s- oder t-Butoxy, Methoxymethyl, Ethoxymethyl, Methoxyethyl, Ethoxyethyl, Cyanomethyl, Cyanoethyl, Methylcarbonyl, Ethylcarbonyl, n- oder i-Propylcarbonyl, n-, i-, s- oder t-Butylcarbonyl, Halogenalkylcarbonyl mit 1 bis 2 Kohlenstoffatomen und 1 bis 5 gleichen oder verschiedenen Fluor- oder Chloratomen, Methoxycarbonylmethyl, Ethoxycarbonylmethyl, Methoxycarbonylethyl, Ethoxycarbonylethyl, Methylamino, Ethylamino, n- oder i-Propylamino, Methylcarbonyloxy, Ethylcarbonyloxy, n- oder i-Propylcarbonyloxy, Aminocabonylmethyl, Aminocarbonylethyl, Propinyl, Butinyl, Cyclopropyl, Cyclopentyl, Cyclohexyl, Phenylcarbonyl, Phenylalkyl und Phenylalkyloxy mit jeweils 1 oder 2 Kohlenstoffatomen im jeweiligen Alkylteil oder für eine Gruppe 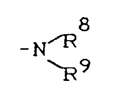 steht
wobei R⁸ und R⁹ jeweils unabhängig voneinander für Methyl, Ethyl, oder Methylcarbonyl, stehen
oder
R⁴ und R⁷ gemeinsam mit dem Stickstoffatom, an welches sie gebunden sind, für eine gesättigte Heteroalkylenkette mit 4 oder 5 Kohlenstoffatomen stehen,
q für die Zahlen 0 oder 1 steht,
R² für unsubstituiertes oder einfach bis dreifach, gleich oder verschieden substituiertes Phenyl steht, wobei mindestens einer der Substituenten aus der Reihe Trifluormethyl, Trifluormethoxy, Fluor, Chlor, Brom in meta-Position steht und gegebenenfalls weitere Substituenten aus der Reihe Fluor, Chlor, Brom, Methyl, Ethyl, n- oder i-Propyl in ortho- oder para-Position stehen; als weiterer Phenylsubstituent sei außerdem der Rest -(CH₂)ₙ-Zₘ-(CH₂)ₚ-R⁵ genannt,
worin
R⁵ für unsubstituiertes oder einfach bis dreifach, gleich oder verschieden substituiertes Phenyl steht, wobei als Substituenten Fluor, Chlor, Brom, Trifluormethyl, Trifluormethoxy, Methyl, Ethyl, n- oder i-Propyl infrage kommen,
Z für Sauerstoff, Schwefel oder für die Gruppe
>C=O steht,
n, m und p für die Zahlen 0 oder 1 stehen, und
R³ und R⁶ für Wasserstoff, Methyl, Ethyl, n- oder i-Propyl, n-, i-, s- oder t-Butyl, unsubstituiertes oder einfach oder zweifach,
gleich oder verschieden substituiertes Phenyl oder Benzyl steht, wobei als Substituenten jeweils Fluor, Chlor, Brom, Methyl, Ethyl, n- oder i-Propyl, Trifluormethyl und Trifluormethoxy infrage kommen, ausgenommen die Verbindung 5,5-Dimethyl-4-amino-3-phenyl-5H-furan-2-on.

7. Verfahren zur Herstellung von 5H-Furan-2-on-Derivaten der Formel (I), 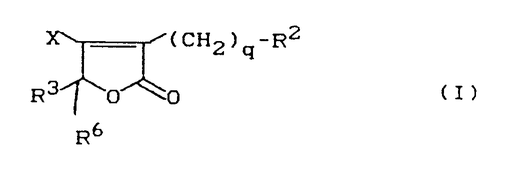 in welcher
X, R², R³, R⁶ und q die in Anspruch 1 angegebene Bedeutung haben,
dadurch gekennzeichnet, daß man
a) für den Fall, daß in der Formel (I)
X für den Rest 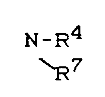 steht und
R², R³, R⁴, R⁶ und q die oben angegebene Bedeutung haben und
R⁷⁻⁰ für Wasserstoff, Alkyl, Alkoxyalkyl, Cyanoalkyl, Alkoxycarbonylalkyl, Alkoxy, Amino, Alkylamino, Hydroxy, Aralkyl, Aminocarbonylalkyl, Cycloalkyl, Arylalkyloxy, Alkinyl oder für die Gruppe 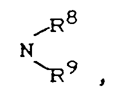 wobei R⁸ und R⁹ die oben angebene Bedeutung haben, steht, oder R⁷⁻⁰ und R⁴ gemeinsame mit dem Stickstoffatom, an das sie gebunden sind, für einen gesättigten Heterocyclus stehen,
a-α) 5H-Furan-2-on-Derivate der Formel (II) 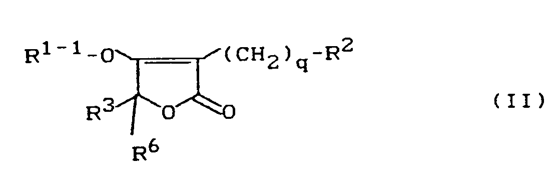 in welcher
R¹⁻¹ für Alkyl, insbesondere Methyl oder Ethyl steht und
R², R³, R⁶ und q die oben angegebenen Bedeutungen haben,
mit Aminen der Formel (III) 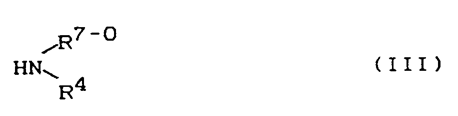 in welcher
R⁷⁻⁰ und R⁴ die oben angegebene Bedeutung haben,
oder deren Hydrochloride,
gegebenenfalls in Gegenwart eines Verdünnungsmittels und gegebenenfalls unter Druck umsetzt oder
a-β) die nach Verfahren (a-α) erhaltenen 5H-Furan-2-on-Derivate der Formel (Ia) 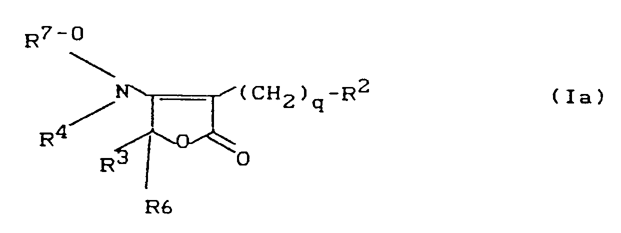 in welcher
R⁷⁻⁰, R², R³, R⁴, R⁶ und q die oben angegebene Bedeutung haben,
mit Acylierungsmitteln der Formel (VI)
R⁷⁻¹-E² (VI)
in welcher
R⁷⁻¹ für Alkylcarbonyl, Alkylcarbonyloxy, Halogenalkylcarbonyl oder Arylcarbonyl steht und
E² für eine elektronenanziehende Abgangsgruppe steht,
oder mit Orthoestern der Formel (VIa)
(RO)₃CH (VIa)
in welcher
R für Methyl oder Ethyl steht,
gegebenenfalls in Gegenwart eines Verdünnungsmittels und gegebenenfalls in Gegenwart eines Reaktionshilfsmittels umsetzt, oder daß man
b) für den Fall, daß in der Formel (I)
X für den Rest OR¹ steht und
R¹, R², R³, R⁶ und q die oben angegebenen Bedeutungen haben,
substituierte Tetronsäure-Derivate der Formel (IV) 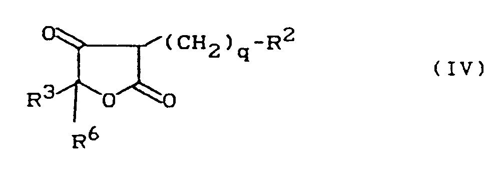 in welcher
R², R³, R⁶ und q die oben angegebenen Bedeutungen haben,
mit Alkylierungs- oder Acylierungsmitteln der Formel (V)
R¹ - E¹ (V)
in welcher
R¹ die oben angegebene Bedeutung hat und
E¹ für eine elektronenanziehende Abgangsgruppe steht,
gegebenenfalls in Gegenwart eines Verdünnungsmittels und gegebenenfalls in Gegenwart eines Katalysators bzw. Reaktionshilfsmittels umsetzt.

8. Herbizide Mittel, gekennzeichnet durch einen Gehalt an mindestens einem 5H-Furan-2-on-Derivat der Formel (I) gemäß Anspruch 1.

9. Verfahren zur Bekämpfung von unerwünschten Pflanzen, dadurch gekennzeichnet, daß man 5H-Furan-2-on-Derivate der Formel (I) gemäß Anspruch 1 auf die Pflanzen und/oder ihren Lebensraum einwirken läßt.

10. Verwendung von 5H-Furan-2-on-Derivaten der Formel (I) gemäß Anspruch 1 zur Bekämpfung von unerwünschten Pflanzen.

11. Verfahren zur Herstellung von herbiziden Mitteln, dadurch gekennzeichnet, daß man 5H-Furan-2-on-Derivate der Formel (I) gemäß Anspruch 1 mit Streckmitteln und/oder oberflächenaktiven Substanzen vermischt.

## Claims

1. 5H-Furan-2-one derivatives of the formula (I) 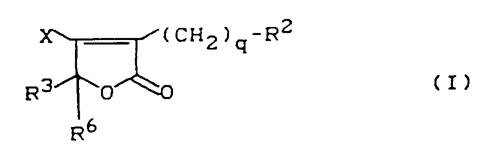 in which
X represents the radical OR¹ or the radical 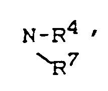 where
R⁴ represents hydrogen, alkyl, alkenyl, alkoxyalkyl or alkylcarbonyl, and
R⁷ represents hydrogen, hydroxyl, amino, formyl, alkyl, alkoxyalkyl, cyanoalkyl, alkylcarbonyl, halogenoalkylcarbonyl, alkoxycarbonylalkyl, alkoxy, alkylamino, alkylcarbonyloxy, aminocarbonylalkyl, alkinyl, cycloalkyl, or arylcarbonyl, aralkyl or aralkyloxy each of which is unsubstituted or substituted or the group 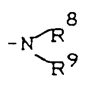 where R⁸ and R⁹ each independently of one another represent alkyl or alkylcarbonyl or
R⁴ and R⁷, together with the nitrogen atom to which they are bonded, represent a saturated heterocycle,
q represents the numbers 0 or 1,
R¹ represents alkyl, alkoxyalkyl, cyanoalkyl, alkylcarbonyl, halogenoalkylcarbonyl or alkoxycarbonylalkyl,
R² represents aryl which is unsubstituted or monosubstituted or polysubstituted by identical or different substituents, the substituents selected being: cyano, nitro, halogen, alkyl, alkoxy, halogenoalkyl, halogenoalkoxy, or phenethenyl or phenethinyl each of which is unsubstituted or monosubstituted or polysubstituted by identical or different substituents, phenyl substituents which may be mentioned being cyano, nitro, halogen, alkyl, alkoxy, halogenoalkyl or halogenoalkoxy, and another phenyl substituent which may be mentioned is furthermore the radical -(CH₂)ₙ-Zₘ-(CH₂)ₚ-R⁵,
where
R⁵ represents aryl which is unsubstituted or monosubstituted or polysubstituted by identical or different substituents, substituents which may be mentioned being: cyano, nitro, halogen, alkyl, alkoxy, halogenoalkyl or halogenoalkoxy,
Z represents oxygen, sulphur or the group -CO-,
n, m and p independently of one another represent the numbers 0 or 1, and
R³ and R⁶ independently of one another represent hydrogen, alkyl, or aryl or aralkyl each of which is unsubstituted or monosubstituted or polysubstituted by identical or different substituents, substituents in the aryl moiety which are selected being: cyano, nitro, halogen, alkyl, alkoxy, halogenoalkyl or halogenoalkoxy,
with the proviso that, if X represents the radical OR¹ and simultaneously q represents 0, R² can only represent ortho-substituted phenyl when the substituents are halogen, halogenomethyl having 1 to 3 identical or different halogen atoms or phenethenyl or phenethinyl each of which is unsubstituted or monosubstituted to polysubstituted by identical or different substituents, with the proviso that R² represents substituted phenyl when q represents 1 and X simultaneously represents the radical OR¹, and with the exception of the compounds 5-[(3,4-dimethoxyphenyl)-methyl]-4-methoxy-3-(4-methoxyphenyl)-5H-furan-2-one, 4-methoxy-3-(3,4,5-trimethoxyphenyl)-5H-furan-2-one, 3-(3-chlorophenyl)-4-methoxy-5H-furan-2-one, 3-(4-chlorophenyl)-4-methoxy-5H-furan-2-one, 3-(3-fluorophenyl)-4-methoxy-5H-furan-2-one, 3-(4-fluorophenyl)-4-methoxy-5H-furan-2-one, 4-methoxy-3-(3-methoxyphenyl)-5H-furan-2-one, 3-(4-bromophenyl)-4-methoxy-5H-furan-2-one, 3-(3,4-dichlorophenyl)-4-methoxy-5H-furan-2-one, 3-[1,1'-biphenyl]-4-yl-4-methoxy-5H-furan-2-one, 4-methoxy-3-(4-methylphenyl)-5H-furan-2-one and 4-methoxy-3-(4-methoxyphenyl)-5H-furan-2-one, 3-(2-chlorophenyl)-4-methoxy-5H-furan-2-one, 3-(2-fluorophenyl)-4-methoxy-5H-furan-2-one, 4-methoxy-3-(2-methoxyphenyl)-5H-furan-2-one, 5,5-dimethyl-4-amino-3-phenyl-5H-furan-2-one, 4-methoxy-3-phenyl-5H-furan-2-one, 5-benzyl-4-methoxy-3-phenyl-5H-furan-2-one, 5,5-diethyl-4-methoxy-3-phenyl-5H-furan-2-one, 5-(α-methoxycarbonyl-α-hydroxy-benzyl)-4-methoxy-3-(4-methoxyphenyl)-5H-furan-2-one, 5-(α-methoxycarbonyl-α-hydroxy-4-methoxybenzyl)-4-methoxy-3-(3,4,5-trimethoxyphenyl)-5H-furan-2-one, 5-(α-hydroxy-4-methoxybenzyl)-4-methoxy-3-(4-methoxyphenyl)-5H-furan-2-one.

2. 5H-Furan-2-one derivatives of the formula (I) according to Claim 1, in which
X represents the radical OR¹ or the radical 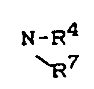 where
R⁴ represents hydrogen, in each case straight-chain or branched alkyl having 1 to 4 carbon atoms, alkenyl having 2 to 4 carbon atoms or alkoxyalkyl having in each case 1 to 4 carbon atoms in the alkoxy moiety and alkyl moiety or alkylcarbonyl having 1 to 4 carbon atoms, and
R⁷ represents hydrogen, hydroxyl, amino, formyl, or alkyl, alkoxy, alkoxyalkyl, cyanoalkyl, alkylcarbonyl, halogenoalkylcarbonyl, alkoxycarbonylalkyl, alkylamino, alkylcarbonyloxy or aminocarbonylalkyl each of which is straight-chain or branched and has 1 to 6 carbon atoms in the individual alkyl moieties and optionally 1 to 9 identical or different halogen atoms, represents alkinyl having 2 to 6 carbon atoms, cycloalkyl having 3 to 7 carbon atoms, or represents arylcarbonyl, aralkyl or aralkyloxy having 7 to 10 carbon atoms and 1 to 4 carbon atoms in the straight-chain or branched alkyl moiety and each of which is unsubstituted or monosubstituted to polysubstituted by identical or different substituents, suitable substituents being the substituents mentioned in the case of R⁵ or R⁷ represents a group 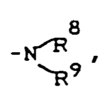 where R⁸ and R⁹ each independently of one another represent alkyl or alkylcarbonyl each having 1 to 4 carbon atoms, or
R⁴ and R⁷, together with the nitrogen atom to which they are bonded, represent a saturated heteroalkylene chain having 4 to 5 carbon atoms,
q represents the numbers 0 or 1,
R¹ represents straight-chain or branched alkyl having 1 to 4 carbon atoms, or represents in each case straight-chain or branched cyanoalkyl, alkoxyalkyl, alkylcarbonyl, halogenoalkylcarbonyl or alkoxycarbonylalkyl having 1 to 6 carbon atoms in the individual alkyl moieties and where appropriate 1 to 9 identical or different halogen atoms,
R² represents phenyl which is unsubstituted or monosubstituted or polysubstituted by identical or different substituents, substituents which may be mentioned being: cyano, nitro, halogen, in each case straight-chain or branched alkyl or alkoxy having 1 to 6 carbon atoms, in each case straight-chain or branched halogenoalkyl or halogenoalkoxy having 1 to 4 carbon atoms and 1 to 9 identical or different halogen atoms, or phenethenyl or phenethinyl, each of which is unsubstituted or monosubstituted or polysubstituted by identical or different substituents, suitable substituents in each case being the phenyl substituents already mentioned above; another phenyl substituent which may be mentioned is furthermore the radical -(CH₂)ₙ-Zₘ-(CH₂)ₚ-R⁵,
where
R⁵ represents aryl which has 6 to 10 carbon atoms, in particular phenyl or naphthyl, and which is unsubstituted or monosubstituted or polysubstituted by identical or different substituents, suitable substituents being cyano, nitro, halogen, C₁₋₄-alkyl, C₁₋₄-alkoxy, halogeno-C₁₋₄-alkyl or halogeno-C₁₋₄-alkoxy,
Z represents oxygen or sulphur or represents the group >C=O and
n, m and p independently of one another represent the numbers 0 or 1, and
R³ and R⁶ represent hydrogen, straight-chain or branched alkyl having 1 to 6 carbon atoms, phenyl which is unsubstituted or monosubstituted to polysubstituted by identical or different substituents, or aralkyl which has 6 to 10 carbon atoms in the aryl moiety and 1 to 4 carbon atoms in the alkyl moiety and which is unsubstituted or monosubstituted to polysubstituted by identical or different substituents, suitable substituents being cyano, nitro, halogen, C₁₋₄-alkyl, C₁₋₄-alkoxy, halogeno-C₁₋₄-alkyl and halogeno-C₁₋₄-alkoxy,
with the proviso that, if X represents the radical OR¹ and simultaneously q represents 0, R² can only represent ortho-substituted phenyl when the substituents are halogen, halogenomethyl having 1 to 3 identical or different halogen atoms or phenethenyl or phenethinyl each of which is unsubstituted or monosubstituted to polysubstituted by identical or different substituents, with the proviso that R² represents substituted phenyl when q represents 1 and X simultaneously represents the radical OR¹, and
with the exception of the compounds 5-[(3,4-dimethoxyphenyl)-methyl]-4-methoxy-3-(4-methoxyphenyl)-5H-furan-2-one, 4-methoxy-3-(3,4,5-trimethoxyphenyl)-5H-furan-2-one, 3-(3-chlorophenyl)-4-methoxy-5H-furan-2-one, 3-(4-chlorophenyl)-4-methoxy-5H-furan-2-one, 3-(3-fluorophenyl)-4-methoxy-5H-furan-2-one, 3-(4-fluorophenyl)-4-methoxy-5H-furan-2-one, 4-methoxy-3-(3-methoxyphenyl)-5H-furan-2-one, 3-(4-bromophenyl)-4-methoxy-5H-furan-2-one, 3-(3,4-dichlorophenyl)-4-methoxy-5H-furan-2-one, 3-[1,1'-biphenyl]-4-yl-4-methoxy-5H-furan-2-one, 4-methoxy-3-(4-methylphenyl)-5H-furan-2-one and 4-methoxy-3-(4-methoxyphenyl)-5H-furan-2-one, 3-(2-chlorophenyl)-4-methoxy-5H-furan-2-one, 3-(2-fluorophenyl)-4-methoxy-5H-furan-2-one and 4-methoxy-3-(2-methoxyphenyl)-5H-furan-2-one, 5,5-dimethyl-4-amino-3-phenyl-5H-furan-2-one, 4-methoxy-3-phenyl-5H-furan-2-one, 5-benzyl-4-methoxy-3-phenyl-5H-furan-2-one, 5,5-diethyl-4-methoxy-3-phenyl-5H-furan-2-one, 5-(α-methoxycarbonyl-α-hydroxy-benzyl)-4-methoxy-3-(4-methoxyphenyl)-5H-furan-2-one, 5-(α-methoxycarbonyl-α-hydroxy-4-methoxybenzyl)-4-methoxy-3-(3,4,5-trimethoxyphenyl)-5H-furan-2-one, 5-(α-hydroxy-4-methoxybenzyl)-4-methoxy-3-(4-methoxyphenyl)-5H-furan-2-one.

3. 5H-Furan-2-one derivatives of the formula (I) according to Claim 1, in which
X represents the radical 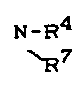 where
R⁴ represents hydrogen, methyl, ethyl, n- or i-propyl, n-, i-, s- or t-butyl, allyl, 2-propenyl, 1-methallyl, methoxymethyl, ethoxymethyl, methoxyethyl, ethoxyethyl, methylcarbonyl, ethylcarbonyl, n-propylcarbonyl or i-propylcarbonyl,
R⁷ represents hydrogen, hydroxyl, amino, formyl, methyl, ethyl, n- or i-propyl, n-, i-, s- or t-butyl, methoxy, ethoxy, n- or i-propoxy, n-, i-, s- or t-butoxy, alkylcarbonyl having 1 to 6 carbon atoms, or alkoxyalkyl, cyanoalkyl, halogenoalkylcarbonyl, alkoxycarbonylalkyl, alkylamino, alkylcarbonyloxy or aminocarbonylalkyl each of which is straight-chain or branched and has 1 to 4 carbon atoms in the individual alkyl moieties and optionally 1 to 9, preferably 1 to 5, identical or different halogen atoms, represents alkinyl having 2 to 4 carbon atoms, cycloalkyl having 3 to 6 carbon atoms, or represents phenylcarbonyl, phenylalkyl or phenylalkyloxy having 7 to 11 carbon atoms and 1 to 4 carbon atoms in the straight-chain or branched alkyl moiety and each of which is unsubstituted or monosubstituted to trisubstituted by identical or different substituents, suitable substituents being those mentioned in the case of R⁵, or represents a group 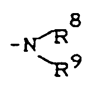 where R⁸ and R⁹ each independently of one another represent methyl, ethyl, n- or i-propyl, methylcarbonyl, ethylcarbonyl or n- or i-propylcarbonyl
or
R⁴ and R⁷, together with the nitrogen atom to which they are bonded, represent a saturated heteroalkylene chain having 4 or 5 carbon atoms,
q represents the numbers 0 or 1,
R² represents phenyl which is unsubstituted or monosubstituted to trisubstituted by identical or different substituents, suitable substituents which may be mentioned being: cyano, nitro, fluorine, chlorine, bromine, methyl, ethyl, n- or i-propyl, methoxy, ethoxy, n- or i-propoxy, in each case straight-chain or branched halogenoalkyl or halogenoalkoxy having 1 or 2 carbon atoms and 1 to 5 identical or different fluorine or chlorine atoms, or phenethenyl or phenethinyl each of which is unsubstituted or monosubstituted to trisubstituted by identical or different substituents, suitable substituents in each case being the phenyl substituents already mentioned above;
as another phenyl substituent, the radical -(CH₂)ₙ-Zₘ-(CH₂)ₚ-R⁵ may also be mentioned,
where
R⁵ represents phenyl which is unsubstituted or monosubstituted to trisubstituted by identical or different substituents, suitable substituents being cyano, nitro, fluorine, chlorine, bromine, methyl, ethyl, n- or i-propyl, methoxy, ethoxy, n- or i-propoxy and in each case straight-chain or branched halogenoalkyl or halogenoalkoxy having 1 or 2 carbon atoms and 1 to 5 identical or different fluorine or chlorine atoms,
Z represents oxygen or sulphur or represents the group >C=O, and
n, m and p independently of one another represent the numbers 0 or 1,
R³ and R⁶ represent hydrogen, methyl, ethyl, n- or i-propyl, n-, i-, s- or t-butyl, phenyl which is unsubstituted or monosubstituted to trisubstituted by identical or different substituents, or benzyl or phenethyl each of which is unsubstituted or monosubstituted to trisubstituted by identical or different substituents, suitable substituents being cyano, nitro, fluorine, chlorine, bromine, methyl, ethyl, n- or i-propyl, methoxy, ethoxy, n- or i-propoxy, and in each case straight-chain or branched halogenoalkyl or halogenoalkoxy having 1 or 2 carbon atoms and 1 to 5 identical or different fluorine or chlorine atoms, with the exception of the compound 5,5-dimethyl-4-amino-3-phenyl-5H-furan-2-one.

4. 5H-Furan-2-one derivatives of the formula (I) according to Claim 1, in which
X represents the radical OR¹, where
R¹ represents straight-chain or branched alkyl having 1 to 4 carbon atoms, or represents in each case straight-chain or branched cyanoalkyl, alkoxyalkyl, alkylcarbonyl, halogenoalkylcarbonyl or alkoxycarbonylalkyl having 1 to 6 carbon atoms in the individual alkyl moieties and where appropriate 1 to 9 identical or different halogen atoms,
R³ and R⁶ represent hydrogen, straight-chain or branched alkyl having 1 to 6 carbon atoms, phenyl which is unsubstituted or monosubstituted to polysubstituted by identical or different substituents, or aralkyl which has 6 to 10 carbon atoms in the aryl moiety and 1 to 4 carbon atoms in the alkyl moiety and which is unsubstituted or monosubstituted or polysubstituted by identical or different substituents, suitable substituents being cyano, nitro, halogen, C₁₋₄-alkyl, C₁₋₄-alkoxy, halogeno-C₁₋₄-alkyl and halogeno-C₁₋₄-alkoxy, and
either
α) R²⁻¹ represents phenyl which is mono-, di- or trisubstituted in the meta- or para-position by identical or different substituents, substituents which may be mentioned being: cyano, nitro, halogen, in each case straight-chain or bran-ched alkyl or alkoxy having 1 to 6 carbon atoms, in each case straight-chain or branched halogenoalkyl or halogenoalkoxy having 1 to 4 carbon atoms and 1 to 9 identical or different halogen atoms, or phenethenyl or phenethinyl each of which is unsubstituted or monosubstituted to polysubstituted by identical or different substituents, suitable substituents in each case being cyano, nitro, halogen, in each case straight-chain or branched alkyl or alkoxy having 1 to 6, carbon atoms, and in each case straight-chain or branched halogenoalkyl or halogenoalkoxy having 1 to 4 carbon atoms and 1 to 9 identical or different halogen atoms;
a further phenyl substituent which may also be mentioned is the radical -(CH₂)ₙ-Zₘ-(CH₂)ₚ-R⁵,
where
R⁵ represents aryl which has 6 to 10 carbon atoms and which is unsubstituted or monosubstituted to polysubstituted by identical or different substituents, suitable substituents being cyano, nitro, halogen, in each case straight-chain or branched alkyl or alkoxy having 1 to 6 carbon atoms, or in each case straight-chain or branched halogenoalkyl or halogenoalkoxy having 1 to 4 carbon atoms and 1 to 9 identical or different halogen atoms,
Z represents oxygen or sulphur, or represents the group >C=O, and
n, m and p represent the numbers 0 or 1, and
q represents the numbers 0 or 1,
with the exception of the compounds 5-[(3,4-dimethoxyphenyl)-methyl]-4-methoxy-3-(4-methoxyphenyl)-5H-furan-2-one, 4-methoxy-3-(3,4,5-trimethoxyphenyl)-5H-furan-2-one, 3-(3-chlorophenyl)-4-methoxy-5H-furan-2-one, 3-(4-chlorophenyl)-4-methoxy-5H-furan-2-one, 3-(3-fluorophenyl)-4-methoxy-5H-furan-2-one, 3-(4-fluorophenyl)-4-methoxy-5H-furan-2-one, 4-methoxy-3-(3-methoxyphenyl)-5H-furan-2-one, 3-(4-bromophenyl)-4-methoxy-5H-furan-2-one, 3-(3,4-dichlorophenyl)-4-methoxy-5H-furan-2-one, 3-[1,1'-biphenyl]-4-yl-4-methoxy-5H-furan-2-one, 4-methoxy-3-(4-methylphenyl)-5H-furan-2-one, 4-methoxy-3-(4-methoxyphenyl)-5H-furan-2-one, 5-(α-methoxycarbonyl-α-hydroxy-4-methoxybenzyl)-4-methoxy-3-(3,4,5-trimethoxyphenyl)-5H-furan-2-one, 5-(α-methoxycarbonyl-α-hydroxy-benzyl)-4-methoxy-3-(4-methoxyphenyl)-5H-furan-2-one and 5-(α-hydroxy-4-methoxybenzyl)-4-methoxy-3-(4-methoxyphenyl)-5H-furan-2-one,
or
β) R²⁻² represents phenyl which is monosubstituted in the ortho-position,
substituents which may be mentioned being: cyano, nitro, halogen, in each case straight-chain or branched alkyl or alkoxy having 1 to 6 carbon atoms, in each case straight-chain or branched halogenoalkyl or halogenoalkoxy having 1 to 4 carbon atoms and 1 to 9 identical or different halogen atoms, or phenethenyl or phenethinyl each of which is unsubstituted or monosubstituted to polysubstituted by identical or different substituents, suitable substituents in each case being cyano, nitro, halogen, in each case straight-chain or branched alkyl or alkoxy having 1 to 6 carbon atoms, in each case straight-chain or branched halogenoalkyl or halogenoalkoxy having 1 to 4 carbon atoms and 1 to 9 identical or different halogen atoms; another phenyl substituent which may furthermore be mentioned is the radical -(CH₂)ₙ-Zₘ-(CH₂)ₚ-R⁵,
R⁵ represents aryl which has 6 to 10 carbon atoms and which is unsubstituted or monosubstituted to polysubstituted by identical or different substituents, suitable substituents being cyano, nitro, halogen, in each case straight-chain or branched alkyl or alkoxy having 1 to 6 carbon atoms, and in each case straight-chain or branched halogenoalkyl or halogenoalkoxy having 1 to 4 carbon atoms and 1 to 9 identical or different halogen atoms,
Z represents oxygen or sulphur, or represents the group >C=O, and
n, m and p represent the numbers 0 or 1 and
q represents the number 1 or
γ) R²⁻³ represents phenyl which is monosubstituted in the ortho-position, substituents which may be mentioned being: halogen, halogenomethyl having 1 to 3 identical or different halogen atoms, or phenethenyl or phenethinyl, each of which is unsubstituted or monosubstituted to polysubstituted by identical or different substituents, suitable substituents in each case being cyano, nitro, halogen, in each case straight-chain or branched alkyl or alkoxy having 1 to 6 carbon atoms, in each case straight-chain or branched halogenoalkyl or halogenoalkoxy having 1 to 4 carbon atoms and 1 to 9 identical or different halogen atoms, and
q represents the number 0,
with the exception of the compounds 3-(2-chlorophenyl)-4-methoxy-5H-furan-2-one, 3-(2-fluorophenyl)-4-methoxy-5H-furan-2-one and 4-methoxy-3-(2-methoxyphenyl)-5H-furan-2-one.

5. 5H-Furan-2-one derivatives of the formula (I) according to Claim 1, in which
X represents the radical OR¹,
R¹ represents methyl, ethyl, n- or i-propyl or n-, i-, s- or t-butyl, or represents in each case straight-chain or branched cyanoalkyl, alkoxyalkyl or alkoxycarbonylalkyl having 1 to 4 carbon atoms in the individual alkyl moieties,
R³ and R⁶ represent hydrogen, methyl, ethyl, n- or i-propyl, n-, i-, s- or t-butyl, phenyl which is unsubstituted or monosubstituted to trisubstituted by identical or different substituents, or benzyl or phenethyl, each of which is unsubstituted or monosubstituted to trisubstituted by identical or different substituents, suitable substituents in each case being cyano, nitro, fluorine, chlorine, bromine, methyl, ethyl, n- or i-propyl, methoxy, ethoxy, n- or i-propoxy, or in each case straight-chain or branched halogenoalkyl or halogenoalkoxy having 1 or 2 carbon atoms and 1 to 5 identical or different fluorine or chlorine atoms;
either
α) R²⁻¹ represents phenyl which is monosubstituted, di- or trisubstituted in the meta- or para-position by identical or different substituents, substituents which may be mentioned being: cyano, nitro, fluorine, chlorine, bromine, methyl, ethyl, n- or i-propyl, methoxy, ethoxy, n- or i-propoxy, in each case straight-chain or branched halogenoalkyl or halogenoalkoxy having 1 or 2 carbon atoms and 1 to 5 identical or different fluorine or chlorine atoms, or phenylethenyl or phenethinyl, each of which is unsubstituted or monosubstituted to trisubstituted by identical or different substituents, suitable substituents in each case being cyano, nitro, fluorine, chlorine, bromine, methyl, ethyl, n- or i-propyl, methoxy, ethoxy, n- or i-propoxy, or in each case straight-chain or branched halogenoalkyl or halogenoalkoxy having 1 or 2 carbon atoms and 1 to 5 identical or different fluorine or chlorine atoms;
as another phenyl substituent, the radical -(CH₂)ₙ-Zₘ-(CH₂)ₚ-R⁵ may also be mentioned,
where
R⁵ represents phenyl which is unsubstituted or monosubstituted to trisubstituted by identical or different substituents, suitable substituents being cyano, nitro, fluorine, chlorine, bromine, methyl, ethyl, n- or i-propyl, methoxy, ethoxy, n- or i-propoxy or in each case straight-chain or branched halogenoalkyl or halogenoalkoxy having 1 or 2 carbon atoms and 1 to 5 identical or different fluorine or chlorine atoms;
Z represents oxygen or sulphur, or represents the group >C=O and
n, m and p represent the numbers 0 or 1 and
q represents the numbers 0 or 1,
with the exception of the compounds 5-[(3,4-dimethoxyphenyl)-methyl]-4-methoxy-3-(4-methoxyphenyl)-5H-furan-2-one, 4-methoxy-3-(3,4,5-trimethoxyphenyl)-5H-furan-2-one, 3-(3-chlorophenyl)-4-methoxy-5H-furan-2-one, 3-(4-chlorophenyl)-4-methoxy-5H-furan-2-one, 3-(3-fluorophenyl)-4-methoxy-5H-furan-2-one, 3-(4-fluorophenyl)-4-methoxy-5H-furan-2-one, 4-methoxy-3-(3-methoxyphenyl)-5H-furan-2-one, 3-(4-bromophenyl)-4-methoxy-5H-furan-2-one, 3-(3,4-dichlorophenyl)-4-methoxy-5H-furan-2-one, 3-[1,1'-biphenyl]-4-yl-4-methoxy-5H-furan-2-one, 4-methoxy-3-(4-methylphenyl)-5H-furan-2-one, 4-methoxy-3-(4-methoxyphenyl)-5H-furan-2-one, 5-(α-methoxycarbonyl-α-hydroxy-4-methoxybenzyl)-4-methoxy-3-(3,4,5-trimethoxyphenyl)-5H-furan-2-one, 5-(α-methoxycarbonyl-α-hydroxy-benzyl)-4-methoxy-3-(4-methoxyphenyl)-5H-furan-2-one and 5-(α-hydroxy-4-methoxybenzyl)-4-methoxy-3-(4-methoxyphenyl)-5H-furan-2-one,
or
β) R²⁻² represents phenyl which is monosubstituted in the ortho-position,
substituents which may be mentioned being: cyano, nitro, fluorine, chlorine, bromine, methyl, ethyl, n- or i-propyl, methoxy, ethoxy, n- or i-propoxy, in each case straight-chain or branched halogenoalhyl or halogenoalkoxy having 1 or 2 carbon atoms and 1 to 5 identical or different fluorine or chlorine atoms, or phenethenyl or phenethinyl, each of which is unsubstituted or monosubstituted to trisubstituted by identical or different substituents, suitable substituents in each case being cyano, nitro, fluorine, chlorine, bromine, methyl, ethyl, n- or i-propyl, methoxy, ethoxy, n- or i-propoxy, or in each case straight-chain or branched halogenoalkyl or halogenoalkoxy having 1 or 2 carbon atoms and 1 to 5 identical or different fluorine or chlorine atoms,
another phenyl substituent which may furthermore be mentioned is the radical -(CH₂)ₙ-Zₘ-(CH₂)ₚ-R⁵
where
R⁵ represents phenyl which is unsubstituted or monosubstituted to polysubstituted by identical or different substituents, suitable substituents being cyano, nitro, fluorine, chlorine, bromine, methyl, ethyl, n- or i-propyl, methoxy, ethoxy, n- or i-propoxy, or in each case straight-chain or branched halogenoalkyl or halogenoalkoxy having 1 or 2 carbon atoms and 1 to 5 identical or different fluorine or chlorine atoms;
Z represents oxygen or sulphur or represents the group >C=O and
n, m and p represent the numbers 0 or 1, and
q represents the number 1, or
γ) R²⁻³ represents phenyl which is monosubstituted in the ortho-position,
the following being mentioned as substituents: fluorine, chlorine, bromine, halogenomethyl having 1 to 3 identical or different fluorine or chlorine atoms, phenethenyl or phenethinyl, each of which is unsubstituted or monosubstituted to trisubstituted by identical or different substituents, suitable substituents in each case being cyano, nitro, fluorine, chlorine, bromine, methyl, ethyl, n- or i-propyl, methoxy, ethoxy, n-or i-propoxy, or in each case straight-chain or branched halogenoalkyl or halogenoalkoxy having 1 or 2 carbon atoms and 1 to 5 identical or different fluorine or chlorine atoms, and
q represents the number 0,
with the exception of the compounds 3-(2-chlorophenyl)-4-methoxy-5H-furan-2-one, 3-(2-fluorophenyl)-4-methoxy-5H-furan-2-one, 4-methoxy-3-(2-methoxyphenyl)-5H-furan-2-one.

6. 5H-Furan-2-one derivatives of the formula (I) according to Claim 1, in which
X represents the radical 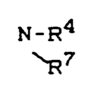 where
R⁴ represents hydrogen, methyl, ethyl, n- or i-propyl, methylcarbonyl or ethylcarbonyl, and
R⁷ represents hydrogen, hydroxyl, amino, formyl, methyl, ethyl, n- or i-propyl, n-, i-, s- or t-butyl, methoxy, ethoxy, n- or i-propoxy, n-, i-, s- or t-butoxy, methoxymethyl, ethoxymethyl, methoxyethyl, ethoxyethyl, cyanomethyl, cyanoethyl, methylcarbonyl, ethylcarbonyl, n- or i-propylcarbonyl, n-, i-, s- or t-butylcarbonyl, halogenoalkylcarbonyl having 1 to 2 carbon atoms and 1 to 5 identical or different fluorine or chlorine atoms, methoxycarbonylmethyl, ethoxycarbonylmethyl, methoxycarbonylethyl, ethoxycarbonylethyl, methylamino, ethylamino, n- or i-propylamino, methylcarbonyloxy, ethylcarbonyloxy, n- or i-propylcarbonyloxy, aminocarbonylmethyl, aminocarbonylethyl, propinyl, butinyl, cyclopropyl, cyclopentyl, cyclohexyl, phenylcarbonyl, phenylalkyl and phenylalkyloxy in each case having 1 or 2 carbon atoms in the respective alkyl moiety or represents a group 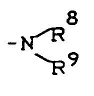 where R⁸ and R⁹ in each case independently of one another represent methyl, ethyl or methylcarbonyl,
or
R⁴ and R⁷, together with the nitrogen atom to which they are bonded, represent a saturated heteroalkylene chain having 4 or 5 carbon atoms,
q represents the numbers 0 or 1,
R² represents phenyl which is unsubstituted or monosubstituted to trisubstituted by identical or different substituents, where at least one of the substituents from the series comprising trifluoromethyl, trifluoromethoxy, fluorine, chlorine and bromine is in the meta-position and, if appropriate, other substituents from the series comprising fluorine, chlorine, bromine, methyl, ethyl or n- or i-propyl are in the ortho- or para-position; another phenyl substituent which may furthermore be mentioned is the radical -(CH₂)ₙ-Zₘ-(CH₂)ₚ-R⁵,
where
R⁵ represents phenyl which is unsubstituted or monosubstituted to trisubstituted by identical or different substituents, suitable substituents being fluorine, chlorine, bromine, trifluoromethyl, trifluoromethoxy, methyl, ethyl or n- or i-propyl,
Z represents oxygen or sulphur or represents the group >C=O,
n, m and p represent the numbers 0 or 1, and
R³ and R⁶ represent hydrogen, methyl, ethyl, n- or i-propyl, n-, i-, s- or t-butyl, or phenyl or benzyl, each of which is unsubstituted or monosubstituted or disubstituted by identical or different substituents, suitable substituents in each case being fluorine, chlorine, bromine, methyl, ethyl, n- or i-propyl, trifluoromethyl or trifluoromethoxy, with the exception of the compound 5,5-dimethyl-4-amino-3-phenyl-5H-furan-2-one.

7. Process for the preparation of 5H-furan-2-one derivatives of the formula (I) 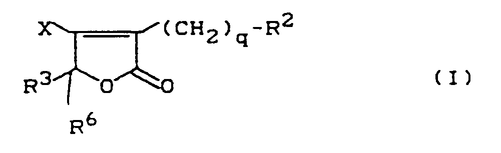 in which
X, R², R³, R⁶ and q have the meaning given in Claim 1,
characterized in that
a) in the event that X in formula (I) represents the radical 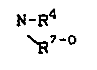 and
R², R³, R⁴, R⁶ and q have the abovementioned meaning and
R⁷⁻⁰ represents hydrogen, alkyl, alkoxyalkyl, cyanoalkyl, alkoxycarbonylalkyl, alkoxy, amino, alkylamino, hydroxy, aralkyl, aminocarbonylalkyl, cycloalkyl, arylalkyloxy, alkinyl or represents the group 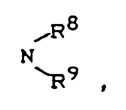 where R⁸ and R⁹ have the abovementioned meaning, or R⁷⁻⁰ and R⁴, together with the nitrogen atom to which they are bonded, represent a saturated heterocycle,
a-α) 5H-furan-2-one derivatives of the formula (II) 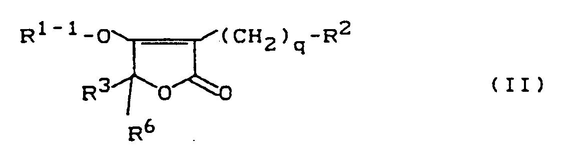 in which
R¹⁻¹ represents alkyl, in particular methyl or ethyl, and
R², R³, R⁶ and q have the abovementioned meanings,
are reacted with amines of the formula (III) 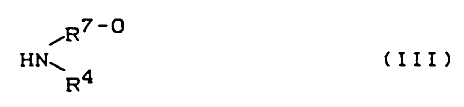 in which
R⁷⁻⁰ and R⁴ have the abovementioned meaning,
or their hydrochlorides,
if appropriate in the presence of a diluent and if appropriate under pressure, or
a-β) the 5H-furan-2-one derivatives, obtained by process (a-α), of the formula (Ia) 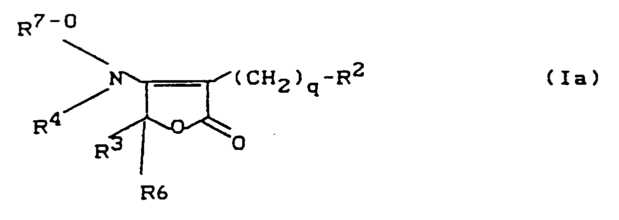 in which
R⁷⁻⁰, R², R³, R⁴, R⁶ and q have the abovementioned meaning,
are reacted with acylating agents of the formula (VI)
R⁷⁻¹E² (VI)
in which
R⁷⁻¹ represents alkylcarbonyl, alkylcarbonyloxy, halogenoalkylcarbonyl or arylcarbonyl and
E² represents an electron-attracting leaving group,
or with ortho esters of the formula (VIa)
(RO)₃CH (VIa)
in which
R represents methyl or ethyl,
if appropriate in the presence of a diluent and if appropriate in the presence of a reaction auxiliary, or in that
b) in the event that X in formula (I) represents the radical OR¹ and
R¹, R², R³, R⁶ and q have the abovementioned meanings,
substituted tetronic acid derivatives of the formula (IV) 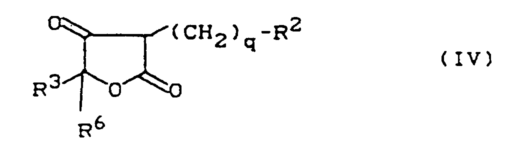 in which
R², R³, R⁶ and q have the abovementioned meanings,
are reacted with alkylating or acylating agents of the formula (V)
R¹ - E¹ (V)
in which
R¹ has the abovementioned meaning and
E¹ represents an electron-attracting leaving group,
if appropriate in the presence of a diluent and if appropriate in the presence of a catalyst or reaction auxiliary.

8. Herbicidal agents, characterized in that they contain at least one 5H-furan-2-one derivative of the formula (I) according to Claim 1.

9. Method of combating undesired plants, characterized in that 5H-furan-2-one derivatives of the formula (I) according to Claim 1 are allowed to act on the plants and/or their environment.

10. Use of 5H-furan-2-one derivatives of the formula (I) according to Claim 1 for combating undesired plants.

11. Process for the preparation of herbicidal agents, characterized in that 5H-furan-2-one derivatives of the formula (I) according to Claim 1 are mixed with extenders and/or surface-active substances.

## Revendications

1. Dérivés de 5H-furanne-2-one de formule (I) 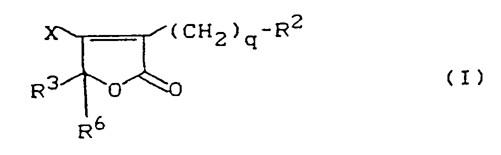 dans laquelle
X représente le reste OR¹ ou le reste 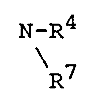 dans lequel
R⁴ est de l'hydrogène, un radical alkyle, alcényle, alkoxyalkyle ou alkylcarbonyle, et
R⁷ est de l'hydrogène, un radical hydroxy, amino, formyle, alkyle, alkoxyalkyle, cyanalkyle, alkylcarbonyle, halogénalkylcarbonyle, alkoxycarbonylalkyle, alkoxy, alkylamino, alkylcarbonyloxy, aminocarbonylalkyle, alcynyle, cycloalkyle, un groupe arylcarbonyle, aralkyle, aralkyloxy dont chacun est dépourvu de substituant ou substitué, ou le groupe 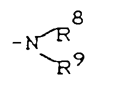 où R⁸ et R⁹ représentent chacun indépendamment l'un de l'autre, un groupe alkyle ou alkylcarbonyle
ou bien
R⁴ et R⁷ forment conjointement avec l'atome d'azote auquel ils sont liés, un hétérocycle saturé
q représente le nombre 0 ou 1,
R¹ est un groupe alkyle, alkoxyalkyle, cyanalkyle, alkylcarbonyle, halogénalkylcarbonyle ou alkoxycarbonylalkyle,
R² est un groupe aryle non substitué ou portant un ou plusieurs substituants égaux ou différents, et on choisi alors comme substituants :
un radical cyano, nitro, halogéno, alkyle, alkoxy, halogénalkyle, halogénalkoxy, un radical phénéthényle ou phénéthynyle dont chacun est dépourvu de substituant ou porte un ou plusieurs substituants identiques ou différents, avec comme substituants de la partie phényle un radical cyano, nitro, halogéno, alkyle, alkoxy, halogénalkyle ou halogénalkoxy, et en outre comme autre constituant de la partie phényle, le reste -(CH₂)ₙ-Zₘ-(CH₂)ₚ-R⁵, où
R⁵ est un groupe aryle non substitué ou portant un ou plusieurs substituants identiques ou différents, et on mentionne alors comme substituants : un radical cyano, nitro, halogéno, alkyle, alkoxy, halogénalkyle ou halogénalkoxy,
Z est de l'oxygène, du soufre, ou le groupe -CO-,
n, m et p représentent indépendamment les uns des autres le nombre 0 ou 1 et
R³ et R⁶ sont, indépendamment l'un de l'autre, de l'hydrogène, un groupe alkyle, un groupe aryle ou aralkyle ne portant pas de substituant ou portant chacun le cas échéant un ou plusieurs substituants identiques ou différents, et on choisi alors comme substituants de la partie aryle : un radical cyano, nitro, halogéno, alkyle, alkoxy, halogénalkyle ou halogénalkoxy,
sous réserve que lorsque X représente le reste OR¹ et que q est en même temps égal à O, R² ne représente un groupe phényle substitué en ortho que lorsque les substituants sont un halogène, un radical halogénométhyle ayant 1 à 3 atomes d'halogènes identiques ou différents ou un groupe phénéthényle ou phénéthynyle dont chacun est dépourvu de substituant ou porte un ou plusieurs substituants identiques ou différents, sous réserve que R² représente un groupe phényle substitué lorsque, en même temps, q est égal à 1 et X représente le reste OR¹ et à l'exception des composés 5-[(3,4-diméthoxyphényl-méthyl)-4-méthoxy-3-(4-méthoxyphényl)-5H-furanne-2-one, 4-méthoxy-3-(3,4,5-triméthoxyphényl)-5H-furanne-2-one, 3-(3-chlorophényl)-4-méthoxy-5H-furanne-2-one, 3-(4-chlorophényl)-4-méthoxy-5H-furanne-2-one, 3-(3-fluorophényl)4-méthoxy-5H-furanne-2-one, 3-(4-fluorophényl)-4-méthoxy-5H-furanne-2-one, 4-méthoxy-3-(3-méthoxyphényl)-5H-furanne-2-one,3-(4-bromophényl)-4-méthoxy-5H-furanne-2-one, 3-(3,4-dichlorophényl)-4-méthoxy-5H-furanne-2-one, 3-[1,1'-biphényl]-4-yl-4-méthoxy-5H-furanne-2-one, 4-méthoxy-3-(4-méthylphényl)-5H-furanne-2-one et 4-méthoxy-3-(4-méthoxyphényl)-5H-furanne-2-one, 3-(2-chlorophényl)-4-méthoxy-5H-furanne-2-one, 3-(2-fluorophényl)-4-méthoxy-5H-furanne-2-one, 4-méthoxy-3-(2-méthoxyphényl)-5H-furanne-2-one, 5,5-diméthyl-4-amino-3-phényl-5H-furanne-2-one, 4-méthoxy-3-phényl-5H-furanne-2-one, 5-benzyl-4-méthoxy-3-phényl-5H-furanne-2-one, 5,5-diéthyl-4-méthoxy-3-phényl-5H-furanne-2-one, 5-(α-méthoxycarbonyl-α-hydroxybenzyl)-4-méthoxy-3-(4-méthoxyphényl)-5H-furanne-2-one, 5-(α-méthoxycarbonyl-α-hydroxy-4-méthoxybenzyl)-4-méthoxy-3-(3,4,5-triméthoxyphényl)-5H-furanne-2-one, 5-(α-hydroxy-4-méthoxybenzyl)-4-méthoxy-3-(4-méthoxyphényl)-5H-furanne-2-one.

2. Dérivés de 5H-furanne-2-one de formule (I) suivant la revendication 1, dans laquelle
X représente le reste OR¹ ou le reste 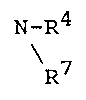 dans lequel
R⁴ est de l'hydrogène, un groupe alkyle de 1 à 4 atomes de carbone, alcényle de 2 à 4 atomes de carbone dans chacune des parties alkoxy et alkyle ou un groupe (alkyle en C₁ à C₄)carbonyle, chacun étant linéaire ou ramifié, et
R⁷ est de l'hydrogène, un groupe hydroxy, amino, formyle, un groupe alkyle, alkoxy, alkoxyalkyle, cyanalkyle, alkoxycarbonyle, halogénalkylcarbonyle, alkoxycarbonylalkyle, alkylamino, alkylcarbonyloxy ou aminocarbonylalkyle, chacun étant linéaire ou ramifié et ayant 1 à 6 atomes de carbone dans les parties alkyle individuelles et le cas échéant 1 à 9 atomes d'halogènes identiques ou différents, un groupe alcynyle de 2 à 6 atomes de carbone, cycloalkyle de 3 à 7 atomes de carbone, un groupe arylcarbonyle, aralkyle, aralkyloxy ayant 3 à 10 atomes de carbone et 1 à 4 atomes de carbone dans la partie alkyle linéaire ou ramifiée, chacun étant dépourvu de substituant ou portant un ou plusieurs substituants identiques ou différents, en considérant comme substituants les substituants mentionnés dans le cas de R⁵, ou bien R⁷ est un groupe de formule 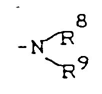 dans laquelle R⁸ et R⁹ représentent chacun, indépendamment l'un de l'autre, un radical alkyle ou alkylcarbonyle ayant chacun 1 à 4 atomes de carbone, ou bien
R⁴ et R⁷ forment conjointement avec l'atome d'azote auquel ils sont liés une chaîne hétéroalkylénique saturée ayant 4 ou 5 atomes de carbone,
q représente le nombre 0 ou 1,
R¹ est un groupe alkyle linéaire ou ramifié ayant 1 à 4 atomes de carbone, ou représente un groupe cyanalkyle, alkoxyalkyle, alkylcarbonyle, halogénalkylcarbonyle ou alkoxycarbonylalkyle linéaire ou ramifié ayant chacun 1 à 6 atomes de carbone dans les parties alkyle individuelles et le cas échéant 1 à 9 atomes d'halogènes identiques ou différents,
R² est un groupe phényle non substitué ou portant un ou plusieurs substituants identiques ou différents, en considérant comme substituants : un reste cyano, nitro, halogéno, un reste alkyle ou alkoxy linéaire ou ramifié ayant chacun 1 à 6 atomes de carbone, un reste halogénalkyle ou halogénalkoxy linéaire ou ramifié ayant chacun 1 à 4 atomes de carbone et 1 à 9 atomes d'halogènes identiques ou différents, un reste phénéthényle ou phénéthynyle ne portant pas de substituant ou portant chacun un ou plusieurs substituants identiques ou différents, en considérant dans chaque cas comme substituants les substituants de la partie phényle qui ont déjà été mentionnés ci-dessus ; avec en outre comme autre substituant de la partie phényle le reste -(CH₂)ₙ-Zₘ-(CH₂)ₚ-R⁵,
dans lequel
R⁵ est un groupe aryle de 6 à 10 atomes de carbone, notamment phényle ou naphtyle, non substitué ou portant un ou plusieurs substituants identiques ou différents, en considérant comme substituants des restes cyano, nitro, halogéno, alkyle en C₁ à C₄, alkoxy en C₁ à C₄, halogénalkyle en C₁ à C₄ ou halogénalkoxy en C₁ à C₄,
Z est de l'oxygène, du soufre ou le groupe >C=O, et
n, m et p représentent indépendamment les uns des autres le nombre 0 ou 1, et
R³ et R⁶ sont de l'hydrogène, un groupe alkyle linéaire ou ramifié ayant 1 à 6 atomes de carbone, un groupe phényle non substitué ou portant un ou plusieurs substituants identiques ou différents ou un groupe aralkyle non substitué ou portant un ou plusieurs substituants identiques ou différents et ayant 6 à 10 atomes de carbone dans la partie aryle et 1 à 4 atomes de carbone dans la partie alkyle, en considérant comme substituants les restes cyano, nitro, halogéno, alkyle en C₁ à C₄, alkoxy en C₁ à C₄, halogénalkyle en C₁ à C₄ et halogénalkoxy en C₁ à C₄,
sous réserve que lorsque X représente le reste OR¹, en même temps que q est égal à 0, R² ne soit un groupe phényle substitué en ortho que lorsque les substituants sont un halogène, un groupe halogénométhyle ayant 1 à 3 atomes d'halogènes identiques ou différents ou un groupe phénéthényle ou phénéthynyle dont chacun est dépourvu de substituant ou porte un ou plusieurs substituants identiques ou différents, sous réserve que R² soit un groupe phényle substitué lorsque, en même temps, q est égal à 1 et X représente le reste OR¹, et
à l'exception des composés 5-[(3,4-diméthoxyphényl-méthyl]-4-méthoxy-3-(4-méthoxyphényl)-5H-furanne-2-one, 4-méthoxy-3-(3,4,5-triméthoxyphényl)-5H-furanne-2-one, 3-(3-chlorophényl)-4-méthoxy-5H-furanne-2-one, 3-(4-chlorophényl)-4-méthoxy-5H-furanne-2-one, 3-(3-fluorophényl)-4-méthoxy-5H-furanne-2-one, 3-(4-fluorophényl)-4-méthoxy-5H-furanne-2-one, 4-méthoxy-3-(3-méthoxyphényl)-5H-furanne-2-one, 3-(4-bromophényl)-4-méthoxy-5H-furanne-2-one, 3-(3,4-dichlorophényl)-4-méthoxy-5H-furanne-2-one, 3-[1,1'-biphényl]-4-yl-4-méthoxy-5H-furanne-2-one,4-méthoxy-3-(4-méthylphényl)-5H-furanne-2- one et 4-méthoxy-3-(4-méthoxyphényl)-5H-furanne-2-one, 3-(2-chlorophényl)-4-méthoxy-5H-furanne-2-one, 3-(2-fluorophényl)-4-méthoxy-5H-furanne-2-one, et 4-méthoxy-3-(2-méthoxyphényl)-5H-furanne-2-one, 5,5-diméthyl-4-amino-3-phényl-5H-furanne-2-one, 4-méthoxy-3-phényl-5H-furanne-2-one, 5-benzyl-4-méthoxy-3-phényl-5H-furanne-2-one, 5,5-diéthyl-4-méthoxy-3-phényl-5H-furanne-2-one, 5-(α-méthoxycarbonyl-α-hydroxybenzyl)-4-méthoxy-3-(4-méthoxyphényl)-5H-furanne-2-one, 5-(α-méthoxycarbonyl-α-hydroxy-4-méthoxybenzyl)-4-méthoxy-3-(3,4,5-triméthoxyphényl)-5H-furanne-2-one, 5-(α-hydroxy-4-méthoxybenzyl)-4-méthoxy-3-(4-méthoxyphényl)-5H-furanne-2-one.

3. Dérivés de 5H-furanne-2-one de formule (I) suivant la revendicatioN 1, dans laquelle
X représente le reste 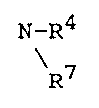 où
R⁴ est de l'hydrogène, un radical méthyle, éthyle, n-propyle, iso-propyle, n-butyle, isobutyle, sec.-butyle, tertio-butyle, allyle, propényle-(2), 1-méthallyle, méthoxyméthyle, éthoxyméthyle, méthoxyéthyle, éthoxyéthyle, méthylcarbonyle, éthylcarbonyle, n-propylcarbonyle ou isopropylcarbonyle,
R⁷ est de l'hydrogène, un groupe hydroxy, amino, formyle, méthyle, éthyle, n-propyle, iso-propyle, n-butyle, iso-butyle, sec.-butyle, tertiobutyle, méthoxy, éthoxy, n-propoxy, iso-propoxy, n-butoxy, iso-butoxy, sec.-butoxy, tertio-butoxy, alkylcarbonyle ayant 1 à 6 atomes de carbone, un groupe alkoxyalkyle, cyanalkyle, halogénalkylcarbonyle, alkoxycarbonylalkyle, alkylamino, alkylcarbonyloxy ou aminocarbonylalkyle linéaire ou ramifié ayant chacun 1 à 4 atomes de carbone dans les parties alkyle individuelles et le cas échéant 1 à 9, de préférence 1 à 5 atomes d'halogènes identiques ou différents, un groupe alcynyle de 2 à 4 atomes de carbone, cycloalkyle de 3 à 6 atomes de carbone, un groupe phénylcarbonyle, phénylalkyle, phénylalkyloxy dépourvus chacun de substituant ou portant 1 à 3 substituants identiques ou différents et ayant 1 à 4 atomes de carbone dans la partie alkyle linéaire ou ramifiée, en considérant comme substituants les substituants mentionnés pour R⁵, ou un groupe 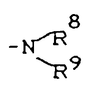 dans lequel R⁸ et R⁹ représentent chacun indépendamment l'un de l'autre un groupe méthyle, éthyle, n-propyle, iso-propyle, méthylcarbonyle, éthylcarbonyle, n-propylcarbonyle ou iso-propylcarbonyle
ou bien
R⁴ et R⁷ forment conjointement avec l'atome d'azote auquel ils sont liés une chaîne hétéroalkylénique saturée ayant 4 ou 5 atomes de carbone,
q représente le nombre 0 ou 1
R² est un groupe phényle dépourvu de substituant ou portant 1 à 3 substituants identiques ou différents, en considérant comme substituants : les radicaux cyano, nitro, fluoro, chloro, bromo, méthyle, éthyle, n-propyle, iso-propyle, méthoxy, éthoxy, n-propoxy, iso-propoxy, halogénalkyle ou halogénalkoxy linéaire ou ramifié ayant chacun 1 ou 2 atomes de carbone et 1 à 5 atomes de fluor ou de chlore identiques ou différents, phénéthényle ou phénéthynyle ne portant pas de substituant ou portant chacun 1 à 3 substituants identiques ou différents, en considérant comme substituants les substituants du groupe phényle déjà mentionnés ci-dessus ; avec en outre, comme autre substituant de la partie phényle, le reste -(CH₂)ₙ-Zₘ-(CH₂)ₚ-R⁵, où
R⁵ est un groupe phényle non substitué ou portant 1 à 3 substituants identiques ou différents, en considérant comme substituants des restes cyano, nitro, fluoro, chloro, bromo, méthyle, éthyle, n-propyle, iso-propyle, méthoxy, éthoxy, n-propoxy, iso-propoxy, halogénalkyle ou halogénalkoxy linéaire ou ramifié ayant chacun 1 ou 2 atomes de carbone et 1 à 5 atomes de fluor ou de chlore identiques ou différents,
Z est de l'oxygène, du soufre ou le groupe >C=O et
n, m et p représentent indépendamment les uns des autres le nombre 0 ou 1,
R³ et R⁶ représentent de l'hydrogène, un groupe méthyle, éthyle, n-propyle, iso-propyle, n-butyle, iso-butyle, sec.-butyle, tertio-butyle, un groupe phényle non substitué ou portant 1 à 3 substituants identiques ou différents ou un groupe benzyle ou phénéthyle non substitué ou portant chacun 1 à 3 substituants identiques ou différents, en considérant comme substituants les restes cyano, nitro, fluoro, chloro, bromo, méthyle, éthyle, n-propyle, iso-propyle, méthoxy, éthoxy, n-propoxy, iso-propoxy, halogénalkyle ou halogénalkoxy linéaire ou ramifié ayant chacun 1 ou 2 atomes de carbone et 1 à 5 atomes de fluor ou de chlore identiques ou différents, excepté le composé 5-5-diméthyl-4-amino-3-phényl-5H-furanne-2-one.

4. Dérivés de 5H-furanne-2-one de formule (I) suivant la revendication 1, dans laquelle
X représente le reste OR¹, où
R¹ est un groupe alkyle linéaire ou ramifié ayant 1 à 4 atomes de carbone ou un groupe cyanalkyle, alkoxyalkyle, alkylcarbonyle, halogénalkylcarbonyle ou alkoxycarbonylalkyle linéaire ou ramifié ayant chacun 1 à 6 atomes de carbone dans les parties alkyle individuelles et le cas échéant 1 à 9 atomes d'halogènes identiques ou différents,
R³ et R⁶ représentent de l'hydrogène, un groupe alkyle linéaire ou ramifié ayant 1 à 6 atomes de carbone, un groupe phényle non substitué ou portant un ou plusieurs substituants identiques ou différents ou un groupe aralkyle non substitué ou portant un ou plusieurs substituants identiques ou différents, ayant 6 à 10 atomes de carbone dans la partie aryle et 1 à 4 atomes de carbone dans la partie alkyle, en considérant comme substituants des restes cyano, nitro, halogéno, alkyle en C₁ à C₄, alkoxy en C₁ à C₄, halogénalkyle en C₁ à C₄ et halogénalkoxy en C₁ à C₄, et
α) R²⁻¹ représente un groupe phényle portant en position méta ou para un, deux ou trois substituants identiques ou différents, en considérant comme substituants :
un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents, phénéthényle ou phénéthynyle non-substitué ou portant chacun 1 ou plusieurs substituants identiques ou différents, en considérant dans chaque cas comme substituants un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents ;
avec en outre comme autre substituant de la partie phényle le reste -(CH₂)ₙ-Zₘ-(CH₂)ₚ-R⁵,
dans lequel
R⁵ est un groupe aryle de 6 à 10 atomes de carbone non substitué ou portant 1 ou plusieurs substituants identiques ou différents, en considérant comme substituants un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents,
Z est de l'oxygène, du soufre ou le groupe >C=O et
n, m et p représentent le nombre 0 ou 1 et
q représente le nombre 0 ou 1,
à l'exception des composés 5-[(3,4-diméthoxyphényl-méthyl]-4-méthoxy-3-(4-méthoxyphényl)-5H- furanne-2-one,4-méthoxy-3-(3,4,5-triméthoxyphényl)-5H-furanne-2-one, 3-(3-chlorophényl)-4-méthoxy-5H-furanne-2-one, 3-(4-chlorophényl)-4-méthoxy-5H-furanne-2-one, 3-(3-fluorophényl)-4-méthoxy-5H-furanne-2-one, 3-(4-fluorophényl)-4-méthoxy-5H-furanne-2-one, 4-méthoxy-3-(3-méthoxyphényl)-5H-furanne-2-one,3-(4-bromophényl)-4-méthoxy-5H-furanne-2-one, 3-(3,4-dichlorophényl)-4-méthoxy-5H-furanne-2-one, 3-[1,1'-biphényl]-4-yl-4-méthoxy-5H-furanne-2-one,4-méthoxy-3-(4-méthylphényl)-5H-furanne-2-one, 4-méthoxy-3-(4-méthoxyphényl)-5H-furanne-2-one, 5-(α-méthoxycarbonyl-α-hydroxy-4-méthoxybenzyl)-4-méthoxy-3-(3,4,5-triméthoxyphényl)-5H-furanne-2-one, 5-(α-méthoxycarbonyl-α-hydroxybenzyl)-4-méthoxy-3-(4-méthoxyphényl)-5H-furanne-2-one,et 5-(α-hydroxy-4-méthoxybenzyl)-4-méthoxy-3-(4-méthoxyphényl)-5H-furanne-2-one, ou bien
β) R²⁻² est un groupe phényle portant un substituant en position ortho-,
en considérant comme substituants :
un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents, phénéthényle ou phénéthynyle ne portant pas de substituant ou portant chacun un ou plusieurs substituants identiques ou différents, en considérant dans chaque cas comme substituants un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents ;
avec en outre comme autre substituant de la partie phényle le reste -(CH₂)ₙ-Zₘ-(CH₂)ₚ-R⁵,
R⁵ est un groupe aryle de 6 à 10 atomes de carbone non substitué ou portant un ou plusieurs substituants identiques ou différents, en considérant comme substituants un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents,
Z est de l'oxygène, du soufre ou le groupe >C=O et
n, m et p représentent le nombre 0 ou 1 et
q est le nombre 1, ou bien
γ) R²⁻³ est un groupe phényle portant un substituant en position ortho, en considérant comme substituants :
un reste halogéno, halogénométhyle portant 1 à 3 atomes d'halogènes identiques ou différents, un reste phénéthényle ou phénéthynyle non substitué ou portant chacun un ou plusieurs substituants identiques ou différents, en considérant dans chaque cas comme substituants un reste cyano, nitro, halogéno, alkyle ou alkoxy linéaire ou ramifié ayant chacun 1 à 6 atomes de carbone, halogénalkyle ou halogénalkoxy linéaire ou ramifié ayant chacun 1 à 4 atomes de carbone et 1 à 9 atomes d'halogènes identiques ou différents et
q représente le nombre 0,
à l'exception des composés 3-(2-chlorophényl)-4-méthoxy-5H-furanne-2-one,3-(2-fluorophényl)-4-méthoxy-5H-furanne-2-one, 4-méthoxy-3-(2-méthoxyphényl)-5H-furanne-2-one.

5. Dérivés de 5H-furanne-2-one de formule (I) suivant la revendication 1, dans laquelle
X représente le reste OR¹,
R¹ est un groupe méthyle, éthyle, n-propyle, iso-propyle, n-butyle, iso-butyle, sec.-butyle, tertio-butyle ou un groupe cyanalkyle, alkoxyalkyle ou alkoxycarbonylalkyle linéaire ou ramifié ayant chacun 1 à 4 atomes de carbone dans les parties alkyle individuelles,
R³ et R⁶ représentent de l'hydrogène, un groupe méthyle, éthyle, n-propyle, iso-propyle, n-butyle, iso-butyle, sec.-butyle, tertio-butyle, un groupe phényle non substitué ou portant 1 à 3 substituants identiques ou différents ou un groupe benzyle ou phénéthyle non substitué ou portant 1 à 3 substituants identiques ou différents, en considérant dans chaque cas comme substituants un reste cyano, nitro, fluoro, chloro, bromo, méthyle, éthyle, n-propyle, iso-propyle, méthoxy, éthoxy, n-propoxy, iso-propoxy, un reste halogénalkyle ou halogénalkoxy linéaire ayant chacun 1 ou 2 atomes de carbone et 1 à 5 atomes de fluor ou de chlore identiques ou différents ;
α) R²⁻¹ est un groupe phényle portant en position méta ou para un, deux ou trois substituants identiques ou différents, en considérant comme substituants : un reste cyano, nitro, fluoro, bromo, méthyle, éthyle, n-propyle, iso-propyle, méthoxy, éthoxy, n-propoxy, iso-propoxy, un reste halogénalkyle ou halogénalkoxy linéaire ou ramifié ayant chacun 1 ou 2 atomes de carbone et 1 à 5 atomes de fluor ou de chlore identiques ou différents, un reste phényléthényle ou phénéthynyle ne portant pas de substituant ou portant chacun 1 à 3 substituants identiques ou différents, en considérant dans chaque cas comme substituants un reste cyano, nitro, fluoro, chloro, bromo, méthyle, éthyle, n-propyle, iso-propyle, méthoxy, éthoxy, n-propoxy, iso-propoxy, un reste halogénalkyle ou halogénalkoxy linéaire ou ramifié ayant chacun 1 ou 2 atomes de carbone et 1 à 5 atomes de fluor ou de chlore identiques ou différents ;
avec en outre comme autre substituant de la partie phényle le reste -(CH₂)ₙ-Zₘ-(CH₂)ₚ-R⁵,
où
R⁵ est un groupe phényle non substitué ou portant 1 à 3 substituants identiques ou différents, en considérant comme substituants le reste cyano, nitro, fluoro, chloro, bromo, méthyle, éthyle, n-propyle, iso-propyle, méthoxy, éthoxy, n-propoxy, iso-propoxy, un reste halogénalkyle ou halogénalkoxy linéaire ou ramifié ayant chacun 1 ou 2 atomes de carbone et 1 à 5 atomes de fluor ou de chlore identiques ou différents,
Z est de l'oxygène, du soufre ou le groupe >C=O et
n, m et p représentent le nombre 0 ou 1 et
q représente le nombre 0 ou 1,
à l'exception des composés 5-[(3,4-diméthoxyphényl-méthyl]-4-méthoxy-3-(4-méthoxyphényl)-5H-furanne-2-one,4-méthoxy-3-(3,4,5-triméthoxyphényl)-5H-furanne-2-one, 3-(3-chlorophényl)-4-méthoxy-5H-furanne-2-one, 3-(4-chlorophényl)-4-méthoxy-5H-furanne-2-one, 3-(3-fluorophényl)-4-méthoxy-5H-furanne-2-one, 3-(4-fluorophényl)-4-méthoxy-5H-furanne-2-one, 4-méthoxy-3-(3-méthoxyphényl)-5H-furanne-2-one,3-(4-bromophényl)-4-méthoxy-5H-furanne-2-one, 3-(3,4-dichlorophényl)-4-méthoxy-5H-furanne-2-one, 3-[1,1'-biphényl]-4-yl-4-méthoxy-5H-furanne-2-one,4-méthoxy-3-(4-méthylphényl)-5H-furanne-2-one, 4-méthoxy-3-(4-méthoxyphényl)-5H-furanne-2-one, 5-(α-méthoxycarbonyl-α-hydroxy-4-méthoxybenzyl)-4-méthoxy-3-(3,4,5-triméthoxyphényl)-5H-furanne-2-one, 5-(α-méthoxycarbonyl-α-hydroxybenzyl)-4-méthoxy-3-(4-méthoxyphényl)-5H-furanne-2-one,et 5-(α-hydroxy-4-méthoxybenzyl)-4-méthoxy-3-(4-méthoxyphényl)-5H-furanne-2-one,
ou bien
β) R²⁻² est un reste phényle portant un substituant en position ortho-, en considérant comme substituants :
un reste cyano, nitro, fluoro, chloro, bromo, méthyle, éthyle, n-propyle- iso-propyle, méthoxy, éthoxy, n-propoxy, iso-propoxy, halogénalkyle ou halogénalkoxy linéaire ou ramifié ayant chacun 1 ou 2 atomes de carbone et 1 à 5 atomes de fluor ou de chlore identiques ou différents, phénéthényle ou phénéthynyle non substitué ou portant 1 à 3 substituants identiques ou différents, en considérant dans chaque cas comme substituants le reste cyano, nitro, fluoro, chloro, bromo, méthyle, éthyle, n-propyle, iso-propyle, méthoxy, éthoxy, n-propoxy, iso-propoxy, halogénalkyle ou halogénalkoxy linéaire ou ramifié ayant chacun 1 ou 2 atomes de carbone et 1 à 5 atomes de fluor ou de chlore identiques ou différents ;
avec en outre comme autre substituant de la partie phényle le reste -(CH₂)ₙ-Zₘ-(CH₂)ₚ-R⁵,
R⁵ est un groupe phényle non substitué ou portant un ou plusieurs substituants identiques ou différents, en considérant comme substituants un reste cyano, nitro, fluoro, chloro, bromo, méthyle, éthyle, n-propyle, iso-propyle, méthoxy, éthoxy, n-propoxy, iso-propoxy, un reste halogénalkyle ou halogénalkoxy linéaire ou ramifié ayant chacun 1 ou 2 atomes de carbone et 1 à 5 atomes de fluor ou de chlore identiques ou différents,
Z est de l'oxygène, du soufre ou le groupe >C=O et
n, m et p représentent le nombre 0 ou 1 et
q est le nombre 1, ou bien
γ) R²⁻³ est un groupe phényle portant un substituant en position ortho, en considérant comme substituants :
un reste fluoro, chloro, bromo, halogénométhyle ayant 1 à 3 atomes de fluor ou de chlore identiques ou différents, un reste phénéthényle ou phénéthynyle non substitué ou portant chacun 1 à 3 substituants identiques ou différents, en considérant dans chaque cas comme substituants un reste cyano, nitro, fluoro, bromo, chloro, méthyle, éthyle, n-propyle, iso-propyle, méthoxy, éthoxy, n-propoxy, iso-propoxy, un reste halogénalkyle ou halogénalkoxy linéaire ou ramifié ayant chacun 1 ou 2 atomes de carbone et 1 à 5 atomes de fluor ou de chlore identiques ou différents et
q est le nombre 0,
excepté les composés 3-(2-chlorophényl)-4-méthoxy-5H-furanne-2-one, 3-(2-fluorophényl)-4-méthoxy-5H-furanne-2-one, 4-méthoxy-3-(2-méthoxyphényl)-5H-furanne-2-one.

6. Dérivés de 5H-furanne-2-one de formule (I) suivant la revendication 1, dans laquelle :
X est le reste 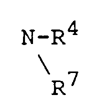 où
R⁴ est de l'hydrogène, un radical méthyle, éthyle, n-propyle, iso-propyle, méthylcarbonyle ou éthylcarbonyle, et
R⁷ est de l'hydrogène, un radical hydroxy, amino, formyle, méthyle, éthyle, n-propyle, iso-propyle, n-butyle, iso-butyle, sec.-butyle, tertio-butyle, méthoxy, éthoxy, n-propoxy, iso-propoxy, n-butoxy, iso-butoxy, sec.-butoxy, tertio-butoxy, méthoxyméthyle, éthoxyméthyle, méthoxyéthyle, éthoxyéthyle, cyanométhyle, cyanéthyle, méthylcarbonyle, éthylcarbonyle, n-propylcarbonyle, iso-propylcarbonyle, n-butylcarbonyle, iso-butylcarbonyle, sec.-butylcarbonyle, tertio-butylcarbonyle, halogénalkylcarbonyle ayant 1 ou 2 atomes de carbone et 1 à 5 atomes de fluor ou de chlore identiques ou différents, méthoxycarbonylméthyle, éthoxycarbonylméthyle, méthoxycarbonyléthyle, éthoxycarbonyléthyle, méthylamino, éthylamino, n-propylamino, iso-propylamino, méthylcarbonyloxy, éthylcarbonyloxy, n-propylcarbonyloxy, iso-propylcarbonyloxy, aminocarbonylméthyle, aminocarbonyléthyle, propynyle, butynyle, cyclopropyle, cyclopentyle, cyclohexyle, phénylcarbonyle, phénylalkyle et phénylalkyloxy ayant chacun 1 ou 2 atomes de carbone dans chaque partie alkyle ou un groupe 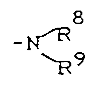 où R⁸ et R⁹ représentent chacun, indépendamment l'un de l'autre, un radical méthyle, éthyle, ou méthylcarbonyle,
ou bien
R⁴ et R⁷ forment conjointement avec l'atome d'azote auquel ils sont liés une chaîne hénéroalkylénique saturée ayant 4 ou 5 atomes de carbone,
q représente le nombre 0 ou 1,
R² est un groupe phényle non substitué ou portant 1 à 3 substituants identiques ou différents, l'un au moins des substituants de la série trifluorométhyle, trifluorométhoxy, fluoro, chloro, bromo étant en position méta- et le cas échéant d'autres substituants de la série fluoro, chloro, bromo, méthyle, éthyle, n-propyle ou iso-propyle étant en position ortho ou para ; avec en outre comme autre substituant de la partie phényle le reste -(CH₂)ₙ-Zₘ-(CH₂)ₚ-R⁵,
où
R⁵ est un groupe phényle non substitué ou portant 1 à 3 substituants identiques ou différents, en considérant comme substituants un reste fluoro, chloro, bromo, trifluorométhyle, trifluorométhoxy, méthyle, éthyle, n-propyle ou iso-propyle,
Z est de l'oxygène, du soufre ou le groupe >C=O,
n, m et p représentent le nombre 0 ou 1 et
R³ et R⁶ représentent de l'hydrogène, un radical méthyle, éthyle, n-propyle, iso-propyle, n-butyle, isobutyle, sec.-butyle, tertio-butyle, un groupe phényle ou benzyle non substitué ou portant chacun 1 ou 2 substituants identiques ou différents, en considérant dans chaque cas comme substituants le fluor, le chlore, le brome, un reste méthyle, éthyle, n-propyle, iso-propyle, trifluorométhyle, trifluorométhoxy, à l'exception du composé 5,5-diméthyl-4-amino-3-phényl-5H-furanne-2-one.

7. Procédé de production de dérivés de 5H-furanne-2-one de formule (I) 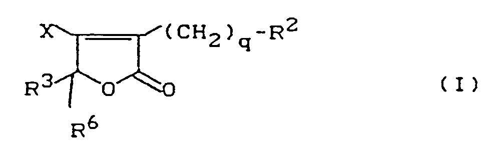 dans laquelle
X, R², R³, R⁶ et q ont la définition indiquée dans la revendication 1, caractérisé en ce que
a) au cas où dans la formule (I) X représente le reste 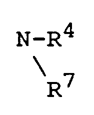 et
R², R³, R⁴, R⁶ et q ont la définition donnée ci-dessus et
R⁷⁻⁰ est de l'hydrogène, un groupe alkyle, alkoxyalkyle, cyanalkyle, alkoxycarbonylalkyle, alkoxy, amino, alkylamino, hydroxy, aralkyle, aminocarbonylalkyle, cycloalkyle, arylalkyloxy, alcynyle ou le groupe 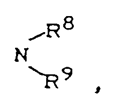 dans lequel R⁸ et R⁹ ont la définition indiquée ci-dessus, ou bien R⁷⁻⁰ et R⁴ forment conjointement avec l'atome d'azote auquel ils sont liés un hétérocycle saturé,
a-α) On fait réagir des dérivés de 5H-furanne-2-one de formule (II) 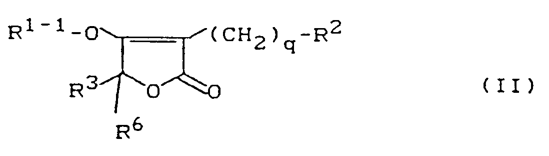 dans laquelle
R¹⁻¹ est un groupe alkyle, notamment méthyle ou éthyle et
R², R³, R⁶ et q ont les définitions indiquées ci-dessus,
avec des amines de formule (III) 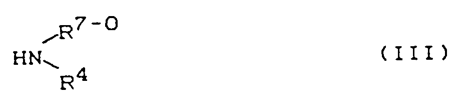 dans laquelle
R⁷⁻⁰ et R⁴ ont la définition indiquée ci-dessus, ou leurs chlorhydrates,
le cas échéant en présence d'un diluant et éventuellement sous pression, ou bien
a-β) On fait réagir les dérivés de 5H-furanne-2-one obtenus par le procédé (a-α), de formule (Ia) 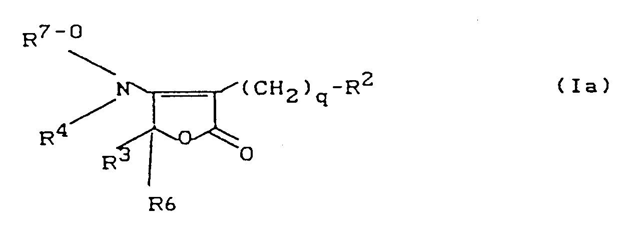 dans laquelle
R⁷⁻⁰, R², R³, R⁴, R⁶ et q ont la définition indiquée ci-dessus,
avec des agents acylants de formule (VI)
R⁷⁻¹-E² (VI)
dans laquelle
R⁷⁻¹ est un groupe alkylcarbonyle, alkylcarbonyloxy, halogénalkylcarbonyle ou arylcarbonyle et
E² est un groupe partant attirant les électrons,
ou avec des orthoesters de formule (VIa)
(RO)₃CH (VIa)
dans laquelle
R est un groupe méthyle ou éthyle,
le cas échéant en présence d'un diluant et en la présence éventuelle d'une substance auxiliaire de réaction, ou bien
b) au cas où dans la formule (I)
X représente le reste OR¹ et
R¹, R², R³, R⁶ et q ont les définitions indiquées ci-dessus,
on fait réagir des dérivés substitués d'acide tétronique de formule (IV) 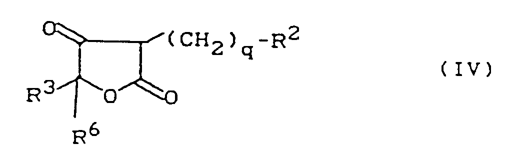 dans laquelle
R² R³, R⁶ et q ont les définitions indiquées ci-dessus,
avec des agents d'alkylation ou d'acylation de formule (V)
R¹ - E¹ (V)
dans laquelle
R¹ a la définition indiquée ci-dessus et
E¹ est un groupe partant attirant les électrons,
le cas échéant en présence d'un diluant et en la présence éventuelle d'un catalyseur ou d'une substance auxiliaire de réaction.

8. Compositions herbicides, caractérisées par une teneur en eau moins un dérivé de 5H-furanne-2-one de formule (I) suivant la revendication 1.

9. Procédé pour combattre des plantes non désirées, caractérisé en ce qu'on fait agir des dérivés de 5H-furanne-2-one de formule (I) suivant la revendication 1 sur les plantes et/ou sur leurs milieux.

10. Utilisation de dérivés de 5H-furanne-2-one de formule (I) suivant la revendication 1 pour combattre des plantes non désirées.

11. Procédé de préparation de compositions herbicides, caractérisé en ce qu'on mélange des dérivés de 5H-furanne-2-one de formule (I) suivant la revendication 1 avec des diluants et/ou des agents tensio-actifs.
